# EUROPEAN PATENT SPECIFICATION

(11) **EP 1 315 732 B1**
(45) Date of publication and mention of the grant of the patent: **07.06.2006**
(21) Application number: 01966236.0
(22) Date of filing: 24.08.2001
(51) Int. Cl.: C07D 493/04, C07D 491/04, C07D 313/00, C07D 225/06, A61P 43/00, A61K 31/395, C07D 303/00, C07D 225/00

(54) **RADICICOL AND MONOCILLIN AND THEIR ANALOGUES AND USES THEREOF**
RADICICOL UND MONOCILLIN UND IHRE ANALOGEN UND IHRE ANWENDUNGEN
RADICICOL ET MONOCILLIN ET LEURS ANALOGUES ET LEURS UTILISATIONS

(30) Priority: 25.08.2000 US 228277 P; 11.07.2001 US 304553 P; 24.08.2001 US 938754
(43) Date of publication of application: 04.06.2003
(73) Proprietor: Sloan-Kettering Institute For Cancer Research, New York, New York 10021 (US)
(72) Inventor: DANISHEFSKY, Samuel, J., Englewood, NJ 07631 (US); GARBACCIO, Robert, M., Landsdale, PA 19446 (US); BAESCHLIN, Daniel, K., CH-4144 Arlesheim (CH); STACHEL, Shawn, J., Perkasie, PA 18944 (US); SOLIT, David, New York, NY 01121 (US); ROSEN, Neil, Englewood, NJ 07631 (US)
(74) Representative: Crooks, Elizabeth Caroline
(86) International application number: PCT/US2001/026577
(87) International publication number: WO 2002/016369

(56) References cited:
- EP-A- 0 889 042
- WO-A-00/38674
- US-A- 4 166 764
- US-A- 5 650 430
- SHIOTSU, YUKIMASA ET AL: "Novel oxime derivatives of radicicol induce erythroid differentiation associated with preferential G1 phase accumulation against chronic myelogenous leukemia cells through destabilization of Bcr-Abl with Hsp90 complex" BLOOD (2000), 96(6), 2284-2291, XP002190760
- LAMIPLAS M., LETT, R.: "CONVERGENT STEREOSPECIFIC TOTAL SYNTHESIS OF MONOCHIRAL MONOCILLIN I RELATED MACROLIDES" TETRAHEDRON LETTERS, vol. 33, no. 6, 1992, pages 773-776, XP001061596 GB cited in the application
- KI, SE WON ET AL: "Radicicol binds and inhibits mammalian ATP citrate lyase" J. BIOL. CHEM., vol. 275, no. 50, 2000, pages 39231-39236, XP002190759
- GARBACCIO R. M., DAMISHEFSKY S.J.: "EFFICIENT ASYMMETRIC SYNTHESIS OF RADICICOL DIMETHYL ETHER: A NOVEL APPLICATION OF RING-FORMING OLEFIN METATHESIS" ORGANIC LETTERS, vol. 2, no. 20, 2000, pages 3127-3129, XP002190761 USA

## Description

### BACKGROUND OF THE INVENTION

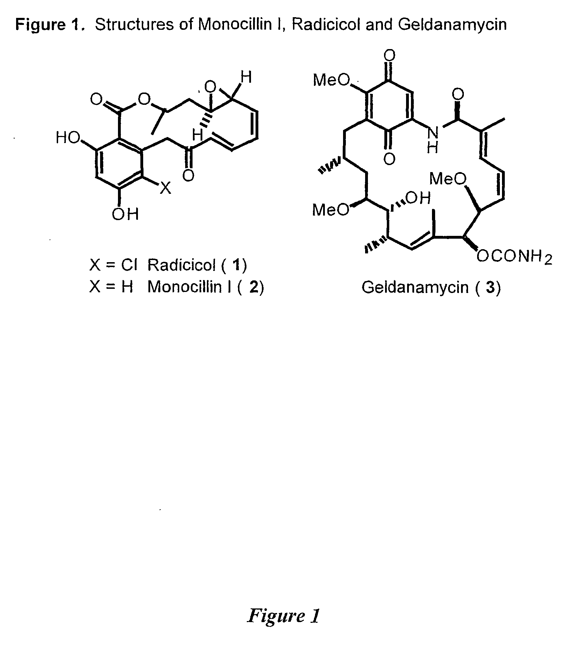

Radicicol (Delmotte *et al. Nature* **1953**, *171,* 344; Ayer *et al. Canad J Microbiol.* **1980**, 26, 766) (**1**) and monocillin I (Ayer *et al. Canad. J Microbiol.* **1980**, *26*, 766) (**2**) are resorcylic macrolides which can both be isolated from Monocillium nordinii (Ayer *et al. Canad. J. Microbiol.* **1980**, *26*, 766) (Figure 1). While the skeletal structure of radicicol was determined in 1964, (McCapra *et al. Tetrahedron Lett.* **1964**, 869; Mirrington *et al. Tetrahedron Lett.* **1964**, 365) its relative and absolute stereochemical configuration was not unambiguously established until 1987 (Cutler *et al. Agric. Biol. Chem.* **1987**, 51, 3331). The structure of monocillin I was confirmed by its direct conversion into radicicol. Affirmation of these structures was achieved by their only total synthesis through the efforts of Lett and Lampilas (Lampilas *et al. Tetrahedron Lett.* **1992**, *33*, 773 and 777).

Both radicicol (**1**) and monocillin I (**2**) (see Figure 1) exhibit a variety of antifungal and antibiotic properties not shared by other members of this class of natural products. Recently, the antitumor properties of radicicol have come into focus as its ability to suppress the transformed phenotype caused by various oncogenes such as *src*, *ras* and *raf* has been linked to its tight binding (20 nM) and inhibition of the Hsp90 molecular chaperone (Roe *et al*. *J*. *Med*. *Chem*. **1999**, *42*, 260-266). This 'anti-chaperone' activity may stimulate depletion of oncogenic proteins, and could therefore be of clinical interest. Specifically, occupancy of the ATP binding pocket of Hsp90 is believed to lead to the degradation in the proteasome of a subset of proteins involved in signal transduction that require Hsp90 for conformational maturation (see, Schneider *et al. Proc. Natl. Acad. Sci.* USA *93*: 14536-14541, **1996**; Mimnaugh *et al. J Biol. Chem. 271*: 22796-22801, **1996**; Whitesell *et al. Mol. Endocrinol. 10*: 705-712, **1996**). These proteins include the HER and insulin receptor families of tyrosine kinases, Raf- 1 serine kinase and steroid receptors to name a few. Downregulation of any of these would be expected to have positive antiproliferative effects, so that Hsp90 is an attractive target for the development of antitumor drugs.

The demonstrated ability of radicicol to bind to and inhibit the activity of Hsp90 has generated an interest in further exploring the biological and pharmacological activity of radicicol and analogues thereof. Significantly, to date, only one synthesis of radicicol itself has been recorded (Lampilas *et al. Tetrahedron Lett.* **1992***, 33,* 773 and 777). Other groups have accessed a variety of analogues from the natural product itself (see, US Patent 5,650,430; US Patent 5,731,343; US Patent 6,239,168; US Patent 5,977,165; and US Patent 5,597,846), but have been limited in the range of analogues that can be generated. Thus, there remains a need to develop a practical synthesis of radicicol to generate novel analogs and conjugates to explore novel biological and pharmacological activities, and to improve the stability and therapeutic efficacy of radicicol and/or monocillin in the treatment of cancer.

### DESCRIPTION OF THE DRAWING

Figure 1 depicts structures of Monocillin I **(2)**, Radicicol **(1)** and Geldanamycin **(3)**.
Figure 2 depicts two strategies for the synthesis of radicicol **(1)** and monocillin **(2)**.
Figure 3 depicts the synthetic strategy for the construction of the chiral allylic alcohol.
Figure 4 depicts the synthetic strategy for the construction of intermediate **(18)**.
Figure 5 depicts the synthetic strategy for the construction of intermediate **(7)** via a Mitsunobou esterification.
Figure 6 depicts a synthetic strategy for the synthesis of radicicol **(1)** and monocillin **(2).**
Figure 7 depicts the synthesis of a variety of chiral components **(28), (30)** and **(32).**
Figure 8 depicts the synthesis of dithiane fragment **(34).**
Figure 9 depicts the synthesis of a variety of benzoic acid components **(35), (36), (37)** and **(38)**.
Figure 10 depicts the generation of diversity at aromatic positions in the macrocycle.
Figure 11 depicts the synthesis of a variety of analogues **(40)**, **(42)**, **(44)** and **(46)**.
Figure 12 depicts the synthesis of a variety of analogues **(48)**, **(50)**, **(52)** and **(54)**.
Figure 13 depicts the synthesis of the chiral cyclopropyl moiety **(30)** and generation of intermediate **(39)**.
Figure 14 depicts the synthetic scheme for cyclopropyl-monocillin I **(2c)** and cyclopropyl-radicicol **(40)**.
Figure 15 depicts the synthesis of a variety of inventive conjugates.
Figure 16 depicts the synthesis of a variety of inventive conjugates.
Figure 17 depicts the results of MCF7 cells (HER2 overexpressed, Rb positive) treated with radicicol and analogues.
Figure 18 depicts the results of BT474 cells (HER2 overexpressed, Rb positive) treated with radicicol and analogues. The gels demonstrate reduction of HER2 levels over a range of concentrations.
Figure 19 depicts the growth of MCF7 cells (HER2 overexpressed, Rb positive) treated with radicicol and analogues.
Figure 20 depicts the ability of radicicol and cyclopropyl radicicol to inhibit breast cancer cells with wild type Rb and small cell lung cancer cells with defective Rb function.
Figure 21 depicts the growth curve for N417 cells (Rb negative cell line) for radicicol and analogues thereof.

### DESCRIPTION OF THE INVENTION

In recognition of the need to develop novel and effective cancer therapies, the present invention provides novel synthetic methodologies enabling access to macrocycles having a broad range of biological and pharmacological activity. In certain embodiments, the inventive compounds are useful in the treatment of cancer. In certain other embodiments of special interest, the compounds are useful for the treatment of cancers comprising Rb negative cancer cells.

### 1) General Description of Compounds of the Invention

The compounds of the invention include compounds of the general formula (I) as further defined below: 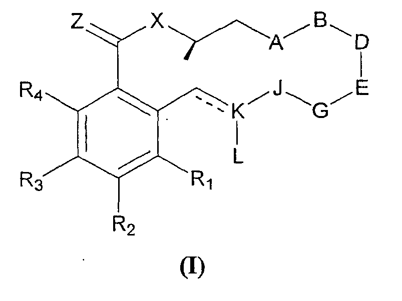 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{c})₂, wherein each occurrence of R_{c} is independently hydrogen, a protecting group, or an aliphatic, heteroaliphatic, aryl, heteroaryl, alkylaryl, or alkylheteroaryl moiety;
R₄ is hydrogen, halogen, cyano, OR_{D}, -N(R_{D})₂, -SR_{D}, -O(C=C)R_{D}, -N(R_{D}XC=O)(R_{D}), -C(O)R_{D}, -C(O)OR_{D}, -CON(R_{D})₂, -OCO₂R_{D}, or an aliphatic, heteroaliphade, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein R_{F} is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 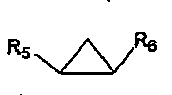 wherein R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -C(O)NR_{K}, -S(O)₂R_{K}, -O(C=O)R_{K}, -N(R_{K})(C=O)(R_{K}), -C(O)R_{K}, -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅₋CHR₆-, R₅ and R₆ taken together represent a substituted or unsubstituted 3-7 membered aliphatic, heteroaliphatic, aryl or heteroaryl ring,
D and E together represent -CHR₈-CHR₉-, -CR₈=CR₉-, wherein R₈ and R₉ are each independently hydrogen or lower alkyl;
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-)-, CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line - represents a bond, whereby a double bond is present, then K and L together represent C-N(RL₎₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl and alkylheteroaryl moiety may be substituted or unsubstituted; wherein one or any two of R₁, R_{A}, R₂, R_{B}, R₃, R_{C}, R₄, R_{D}, R₅, R₆, R_{J}, or R_{L}, are optionally a linker covalently bonded to a compound selected from the group consisting of radicicol, monocilin, analogues of radicicol and monocillin, geldanamycin, analogues of geldanamycin, and steroids; and
pharmaceutically acceptable derivatives thereof.

In certain embodiments for the compounds as described above, one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, and the linker is an aliphatic or heteroaliphatic moiety, whereby said aliphatic or heteroaliphatic moiety is substituted or unsubstituted, branched or unbranched, or cyclic or acyclic.

In certain other embodiments for the compounds as described above, one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, and the linker is a moiety having one of the structures -(CH₂)ₙ-CH=CH-(CH₂)ₘ-, -(CH₂)ₚ-C≡C-(CH₂)_{q}-, or CH₂(CH₂)₈CH₂-, wherein each occurrence of n, m, p, q and s is independently an integer from 0-10. In certain embodiments, one or more of the hydrogen atoms may be replaced with a substituent including, but not limited to alkyl, heteroalkyl, secondary or tertiary amine, hydroxyl, thiol, aryl, heteroaryl, alkylaryl or alkylheteroaryl.

### 2) Featured Classes of Compounds

It will be appreciated that for compounds as generally described above, certain classes of compounds are of special interest. For example, one class of compounds of special interest includes those compounds having the structure of formula (I) in which Z and X are each O, and the compound has the structure: 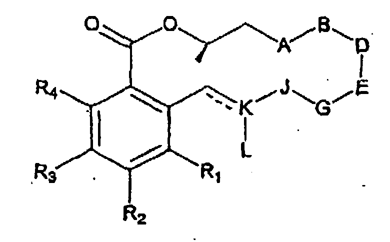 and R₁, R₂, R₃, R₄, A-B, D-E, G-J, and K-L are as defined above and in subclasses herein.

Another class of compounds of special interest consists of compounds having the structure of formula (I) in which Z is O and X is NR_{G}, and the compound has the structure: 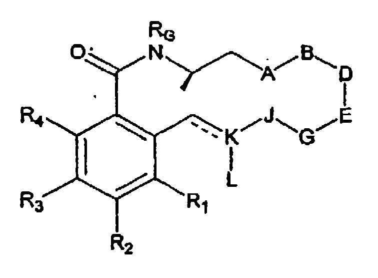 and R₁, R₂, R₃, R₄, R_{G}, A-B, D-E, G-J, and K-L are as defined above and in subclasses herein.

Another class of compounds of special interest consists of compounds having the structure of formula (I) in which G and J together represent -CH₂-CH₂- and the compound has the structure: 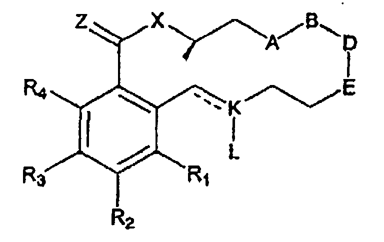 and R₁, R₂, R₃, R₄, Z, X, A-B, D-E, and K-L are as defined above and in subclasses herein.

Another class of compounds of special interest consists of compounds having the structure of formula (I) in which A-B is a cyclopropyl ring and the compound has the structure: 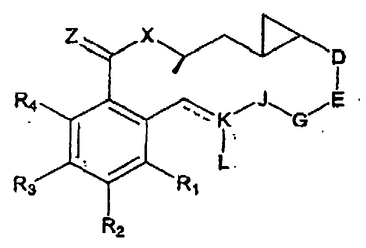 and R₁, R₂, R₃, R₄, Z, X, D-E, G-J, and K-L are as defined above and in subclasses herein.

Another class of compounds of special interest consists of compounds having the structure of formula (I) in which the dotted line - is absent whereby a single bond is present, K and L together represent -CH₂- and the compound has the structure: 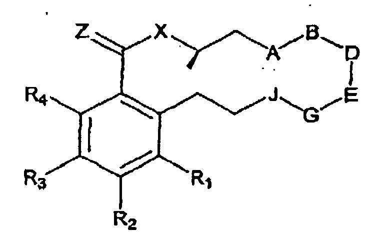 and R₁, R₂, R₃, R₄, Z, X, A-B, D-E, and G-J are as defined above and in subclasses herein.

Another class of compounds of special interest consists of compounds having the structure of formula (I) in which the dotted line - is absent whereby a single bond is present, K-L together represent C=O and the compound has the structure: 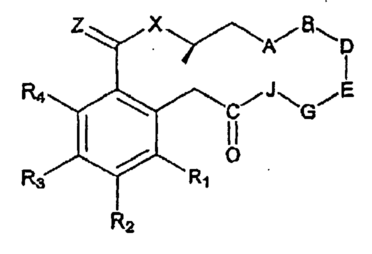 and R₁, R₂, R₃, R₄, Z, X, A-B, D-E, and G-J are as defined above and in subclasses herein.

Another class of compounds of special interest consists of compounds having the structure of formula (I) in which the dotted line - is absent whereby a single bond is present, K and L together represent C=N-O-R_{L} and the compound has the structure: 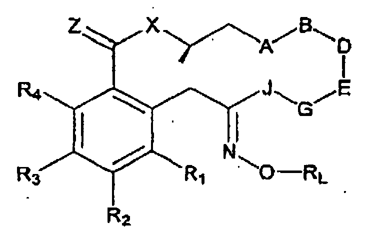 and R₁, R₂, R_{3,} R₄, Z, X, A-B, D-E, G-J, and R_{L} are as defined above and in subclasses herein.

Another class of compounds of special interests consists of compounds having the structure of formula (I) in which the dotted line - is absent whereby a single bond is present, A and B together represent a cyclopropyl group, K and L together represent C=N-O-R_{L} and the compound has the structure: 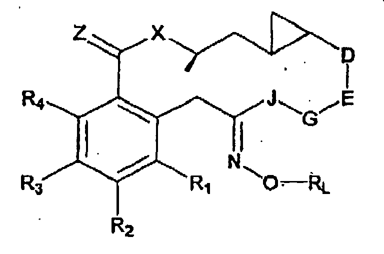 and R₁, R₂, R₃, R₄, Z, X*,* D-E, G-J and R_{L} are defined above and in subsclasses herein.

Another class of compounds of special interest consists of compounds having the structure of formula (I) in which the dotted line - is absent whereby a single bond is present, K, and L together represent C=CH₂ and the compound has the structure: 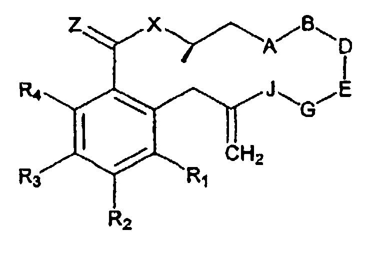 and R₁, R₂, R₃, R₄, Z, X, A-B, D-E, and G-J are as defined above and in subclasses herein.

Another class of compounds of special interest consists of compounds having the structure of formula (I) in which the dotted line - is absent whereby a single bond is present, K and L together represent a dithiane, -C(-S(CH₂)₃S-)-, and the compound has the structure: 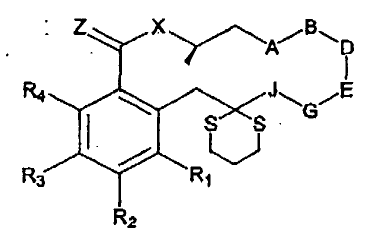 and R₁, R₂, R₃, R₄, Z, X, A-B, D-E and G-J are as defined above and in subclasses herein.

The following structure illustrates several exemplary types of compounds of these classes. Others will be readily apparent to the reader:

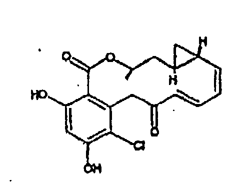

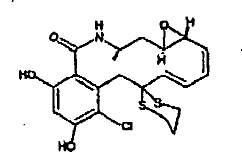

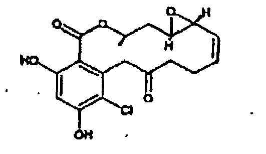

Other classes of compounds in which dimers and heterodimers of radicicol and analogues thereof are formed with radicicol, analogues thereof, monocillin and analogues thereof, geldanamycin and analogues thereof, and steroids are illustrated by the following sorts of compounds:

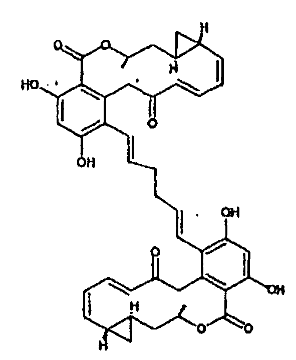

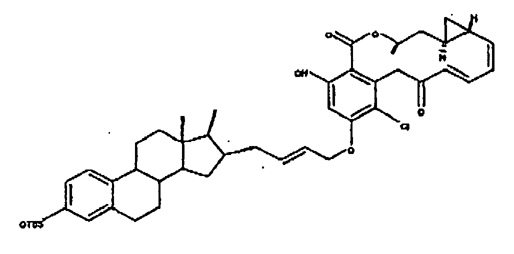

A number of important subclasses of each of the foregoing classes deserve separate mention; these subclasses include subclasses of the foregoing classes in which:
i) Z and X are each O;
ii) Z is O and X is NH;
iii) A and B together are cyclopropyl;
iv) R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), or -OCO₂R_{J}, -OSO₂R_{J}, and each occurrence of R_{J} is independently hydrogen, a protecting group or an aliphatic, heteroaliphatic, aryl, heteroaryl, alkylaryl, or alkylheteroaryl moiety,
v) R₅ and R₆ are each independently hydrogen, or lower alkyl;
vi) D and E together are -CHR₈-CHR₉-;
vii) D and E together are -CR₈=CR₉-;
viii) R₈ and R₉ are each hydrogen;
ix) G and J together are -CHR₁₀-CHR₁₁-;
x) G and J together are -CR₁₀=CR₁₁-;
xi) R₁₀ and R₁₁ are each hydrogen;
xii) K and L together are C=O;
xiii) K and L togethet are -CH₂-;
xiv) K and L together are C=CH₂;
xv) K and L together are a dithiane moiety;
xvi) K and L together are C=N-O-R_{L};
xvii) R₁ is hydrogen, halogen, lower alkyl, lower heteroalkyl, lower alkylaryl, lower alkylheteroaryl, or N(R_{A})₂, wherein each occurrence of R_{A} is independently hydrogen, a protecting group, or an aliphatic, heteroaliphatic, aryl, heteroaryl, alkylaryl, or alkylheteroaryl moiety and R₃ is hydrogen, halogen, lower alkyl, lower heteroalkyl, lower alkylaryl, lower alkylheteroaryl, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
xxii) R₁ and R₃ are each independently halogen, hydrogen, or lower alkyl;
xxiii) R₂ is hydrogen, halogen, -OR_{B}, -N(R_{B})₂, -SR_{B}, -O(C=O)R_{B}, -N(R_{B})(C=O)(R_{B}), -C(O)R_{B}, -C(O)OR_{D}, -CON(R_{B})₂, -OCO₂R_{B}, or lower alkyl, lower heteroalkyl, lower alkylaryl, lower alkylheteroaryl, wherein each occurrence of R_{B} is independently hydrogen, a protecting group or an aliphatic, heteroaliphatic, aryl, heteroaryl, alkylaryl, or alkylheteroaryl moiety, and R₄ is hydrogen, halogen. -OR_{D}, -N(R_{D})₂, -SR_{D}, -O(C=O)R_{D}, -N(R_{D})(C=OXR_{D}), -C(O)R_{D}, -C(O)OR_{D}, -CON(R_{D})₂, -OCO₂RD, or lower alkyl, lower heteroalkyl, lower alkylaryl, lower alkylheteroaryl, wherein each occurrence of R_{D} is independently hydrogen, a protecting group or an aliphatic, heteroaliphatic, aryl, heteroaryl, alkylaryl, or alkylheteroaryl moiety,
xxiv) R₂ is hydrogen or -OR_{B}, wherein each occurrence of R_{B} is independently hydrogen, a protecting group or an aliphatic, heteroaliphatic, aryl, heteroaryl, alkylaryl, or alkylheteroaryl moiety, and R₄ is hydrogen or -OR_{D}, wherein each occurrence of R_{D} is independently hydrogen, a protecting group or an aliphatic, heteroalipliatic, aryl, heteroaryl, alkylaryl, or alkylheteroaryl moiety;
xxv) one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids;
xxvi) one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, and the linker is an aliphatic or heteroaliphatic moiety, whereby said aliphatic or heteroaliphatic moiety is substituted or unsubstituted, branched or unbranched, or cyclic or acyclic;
xxvii) one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, and the linker is a moiety having one of the structures -(CH₂)ₙ-CH=CH-(CH₂)ₘ-, -(CH₂)_{P}-C≡C-(CH₂)_{q}-, or -CH₂(CH₂)ₛCH₂-, wherein each occurrence ofn, m, p, q and s is independently an integer from 0-10, and wherein one or more of the hydrogen atoms are optionally replaced with a substituent including, but not limited to alkyl, heteroalkyl, secondary or tertiary amine, hydroxyl, thiol, aryl, heteroaryl, alkylaryl, or alkylheteroaryl.

As the reader will appreciate, compounds of particular interest include, among others, those which share the attributes of one or more of the foregoing subclasses. Some of those subclasses are illustrated by the following sorts of compounds:

### I) Compounds of the formula:

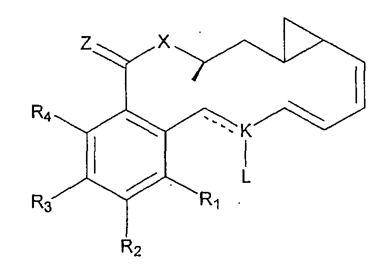

wherein Z is O and X is O or NH;
R₁ is hydrogen, halogen, lower alkyl, lower heteroalkyl, lower alkylaryl, lower alkylheteroaryl, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or lower alkyl, lower heteroalkyl, lower alkylaryl, lower alkylheteroaryl, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, lower alkyl, lower heteroalkyl, lower alkylaryl, lower alkylheteroaryl, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or lower alkyl, lower heteroalkyl, lower alkylaryl, lower alkylheteroaryl, wherein each occurrence of R_{D} is independently hydrogen, a protecting group or an aliphatic, heteroaliphatic, aryl, heteroaryl, alkylaryl, or alkylheteroaryl moiety;
K and L together represent C=O, C=S, CH-CH₃, CH-CH(R_{L})₂, C=C(R_{L})₂, -CH₂-, -C(-S(CH₂)₃S-)-, CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, or R_{L} are optionally a linker covalently bonded to a compound selected from the group consisting of radicicol, monocillin, analogues of radicicol and monocillin, geldanamycin, analogues of geldanamycin, and steroids.

In certain embodiments of the compounds described above, R₁ and R₃ are each independently halogen, hydrogen, or lower alkyl; R₂ is hydrogen or -OR_{B}, wherein each occurrence of R_{B} is independently hydrogen, a protecting group or an aliphatic, heteroaliphatic, aryl, heteroaryl, alkylaryl, or alkylheteroaryl moiety, and R₄ is hydrogen or -OR_{D}, wherein each occurrence of R_{D} is independently hydrogen, a protecting group or an aliphatic, heteroaliphatic, aryl, heteroaryl, alkylaryl, or alkylheteroaryl moiety.

### II) Compounds of the formula:

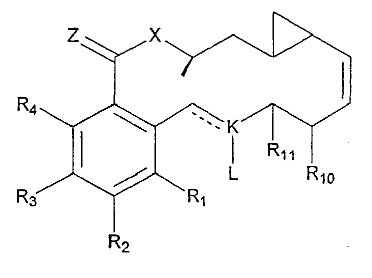

wherein Z is O and X is O or NH;
R₁ is hydrogen, halogen, lower alkyl, lower heteroalkyl, lower alkylaryl, lower alkylheteroaryl, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or lower alkyl, lower heteroalkyl, lower alkylaryl, lower alkylheteroaryl, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, lower alkyl, lower heteroalkyl, lower alkylaryl, lower alkylheteroaryl, or -N(R_{C})₂, wherein each occurrence of R_{C} is independently hydrogen, a protecting group, or an aliphatic, heteroaliphatic, aryl heteroaryl, alkylaryl, or alkylheteroaryl moiety;
R₄ is hydrogen, halogen, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or lower alkyl, lower heteroalkyl, lower alkylaryl, lower alkylheteroaryl, wherein each occurrence of R_{D} is independently hydrogen, a protecting group or an aliphatic, heteroaliphatic, aryl, heteroaryl, alkylaryl, or alkylheteroaryl moiety;
R₁₀ and R₁₁ are each independently hydrogen or lower alkyl;
K and L together-represent C=O, S=O, CH-CH₃, CH-CH(R_{L})₂, C=C(R_{L})₂, -CH₂-, -C(-S(CH₂)₃S-)-, CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line - represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl or alkylheteroaryl moiety, or two occurrences of R_{L} taken together represent a 3 to 7-metnbered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R₄, R₂, R_{B}, R₃, R_{C}, R₄, R_{D}, or R_{L} are optionally a linker covalently bonded to a compound selected from the group consisting of radicicol, monocillin, analogues of radicicol and monocillin, geldanamycin, analogues of geldanamycin, and steroids.

In certain embodiments of compounds described above, R₁ and R₃ are each independently halogen, hydrogen, or lower alkyl; R₂ is hydrogen or -OR_{B}, wherein each occurrence of R_{B} is independently hydrogen, a protecting group or an aliphatic, heteroaliphatic, aryl, heteroaryl, alkylaryl, or alkylheteroaryl moiety, and R₄ is hydrogen or -OR_{D}, wherein each occurrence of R_{D} is independently hydrogen, a protecting group or an aliphatic, heteroaliphatic, aryl, heteroaryl, alkylaryl, or alkymeteroaryl moiety.

Some of the foregoing compounds can exist in various isomeric forms. The invention encompasses the compounds as individual isomers substantially free of other isomers and alternatively, as mixtures of various isomers, e.g., racemic mixtures of stereoisomers. The invention also encompasses tautomers of specific compounds as described above. In addition to the above-mentioned compounds per se, this invention also encompasses pharmaceutically acceptable derivatives of these compounds and compositions comprising one or more compounds of the invention and one or more pharmaceutically acceptable excipients or additives.

Compounds of this invention which are of particular interest include those which:
- exhibit cytotoxic or growth inhibitory effect on cancer cell lines maintained in vitro or in animal studies using a scientifically acceptable cancer cell xenograft model;
- exhibit cytotoxic or growth inhibitory effect on cancer cell lines comprising Rb negative cells;
- exhibit inhibitory activity for the HER-family of protein kinases; and
- bind to and/or inhibit the Hsp90 family of chaperones;
- exhibit cytotoxic or growth inhibitory effect on cancer cell lines comprising Rb positive cells; and
- exhibit cytotoxic or growth inhibitory effect on cancer cell lines upregulated in the androgenic cell receptor (e.g., including, but not limited to prostate cancer cells).

This invention also provides a pharmaceutical preparation comprising at least one of the compounds as described above and herein, or a pharmaceutically acceptable derivative thereof, which compounds are capable of inhibiting the growth of or killing cancer cells, and, in certain embodiments of special interest are capable of inhibiting the growth of or killing cancer cells comprising Rb negative cancer cells.

The invention further provides a method for inhibiting tumor growth and/or tumor metastasis. In certain embodiments of special interest, the invention provides a method of treating cancers by inhibiting tumor growth and/or tumor metastasis for tumors comprising Rb negative cancer cells. The method involves the administration of a therapeutically effective amount of the compound or a pharmaceutically acceptable derivative thereof to a subject (including, but not limited to a human or animal) in need of it. In certain embodiments, specifically for treating cancers comprising Rb negative cancer cells, the therapeutically effective amount is an amount sufficient to kill or inhibit the growth of Rb negative cancer cell lines. In certain embodiments, the inventive compounds are useful for the treatment of solid tumors. In still other embodiments of interest, the inventive compounds are useful for the treatment of glioblastoma, retinoblastoma or small cell lung cancer.

### 3) Compounds and Definitions

As discussed above, this invention provides novel compounds with a range of biological properties. Compounds of this invention have biological activities relevant for the treatment of diseases or other disorders such as proliferative diseases, including, but not limited to cancer. More generally, the compounds are useful in the regulation of the cell cycle pathway. As described above, the ability of certain compounds to bind to Hsp90 ultimately causes arrest of cell growth and apoptosis.

Compounds of this invention include those specifically set forth above and described herein, and are illustrated in part by the various classes, subgenera and species disclosed elsewhere herein.

It will be appreciated by one of ordinary skill in the art that asymmetric centers may exist in the compounds of the present invention. Thus, inventive compounds and pharmaceutical compositions thereof may be in the form of an individual enantiomer, diastereomer or geometric isomer, or may be in the form of a mixture of stereoisomers. In certain embodiments, the compounds of the invention are enantiopure compounds. In certain other embodiments, a mixtures of stereoisomers or diastereomers are provided.

Additionally, the present invention provides pharmaceutically acceptable derivatives of the inventive compounds, and methods of treating a subject using these compounds, pharmaceutical compositions thereof, or either of these in combination with one or more additional therapeutic agents. The phrase, "pharmaceutically acceptable derivative", as used herein, denotes any pharmaceutically acceptable salt, ester, or salt of such ester, of such compound, or any other adduct or derivative which, upon administration to a patient, is capable of providing (directly or indirectly) a compound as otherwise described herein, or a metabolite or residue thereof. Pharmaceutically acceptable derivatives thus include among others pro-drugs. A pro-drug is a derivative of a compound, usually with significantly reduced pharmacological activity, which contains an additional moiety which is susceptible to removal *in vivo* yielding the parent molecule as the pharmacologically active species. An example of a pro-drug is an ester which is cleaved in vivo to yield a compound of interest. Pro-drugs of a variety of compounds, and materials and methods for derivatizing the parent compounds to create the pro-drugs, are known and may be adapted to the present invention. Certain exemplary pharmaceutical compositions and pharmaceutically acceptable derivatives will be discussed in more detail herein below.

Certain compounds of the present invention, and definitions of specific functional groups are also described in more detail below. For purposes of this invention, the chemical elements are identified in accordance with the Periodic Table of the Elements, CAS version, Handbook of Chemistry and Physics, 75^{th} Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito: 1999. Furthermore, it will be appreciated by one of ordinary skill in the art that the synthetic methods, as described herein, utilize a variety of protecting groups. By the term "protecting group", has used herein, it is meant that a particular functional moiety, e.g., O, S, or N, is temporarily blocked so that a reaction can be carried out selectively at another reactive site in a multifunctional compound. In preferred embodiments, a protecting group reacts selectively in good yield to give a protected substrate that is stable to the projected reactions; the protecting group must be selectively removed in good yield by readily available, preferably nontoxic reagents that do not attack the other funcational groups; the protecting group forms an easily separable derivative (more preferably without the generation of new stereogenic centers); and the protecting group has a minimum of additional functionality to avoid further sites of reaction. As detailed herein, oxygen, sulfur, nitrogen and carbon protecting groups may be utilized. Exemplary protecting groups are detailed herein, however, it will be appreciated that the present invention is not intended to be limited to these protecting groups; rather, a variety of additional equivalent protecting groups can be readily identified using the above criteria and utilized in the method of the present invention. Additionally, a variety of protecting groups are described in "Protective Groups in Organic Synthesis" Third Ed. Greene, T.W. and Wuts, P.G., Eds., John Wiley & Sons, New York: 1999.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example of proliferative disorders, including, but not limited to cancer. The term "stable", as used, herein, preferably refers to compounds which possess stability sufficient to allow manufacture and which maintain the integrity of the compound for a sufficient period of time to be detected and preferably for a sufficient period of time to be useful for the purposes detailed herein.

The term "aliphatic", as used herein, includes both saturated and unsaturated, straight chain (i.e., unbranched), branched, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, as used herein, the term "alkyl" includes straight, branched and cyclic alkyl groups. An analogous convention applies to other generic terms such as "alkenyl", "alkynyl" and the like. Furthermore, as used herein, the terms "alkyl", "alkenyl", "alkynyl" and the like encompass both substituted and unsubstituted groups. In certain embodiments, as used herein, "lower alkyl" is used to indicate those alkyl groups (cyclic, acyclic, substituted, unsubstituted, branched or unbranched) having 1-6 carbon atoms.

In certain embodiments, the alkyl, alkenyl and alkynyl groups employed in the invention contain 1-20 aliphatic carbon atoms. In certain other embodiments, the alkyl, alkenyl, and alkynyl groups employed in the invention contain 1-10 aliphatic carbon atoms. In yet other embodiments, the alkyl, alkenyl, and alkynyl groups employed in the invention contain 1-8 aliphatic carbon atoms. In still other embodiments, the alkyl, alkenyl, and alkynyl groups employed in the invention contain 1-6 aliphatic carbon atoms. In yet other embodiments, the alkyl, alkenyl, and alkynyl groups employed in the invention contain 1-4 carbon atoms. Illustrative aliphatic groups thus include, but are not limited to, for example, methyl, ethyl, n-propyl, isopropyl, cyclopropyl, -CH₂-cyclopropyl, allyl, n-butyl, sec-butyl, isobutyl, tert-butyl, cyclobutyl, -CH₂-cyclobutyl, n-pentyl, sec-pentyl, isopentyl, tert-pentyl, cyclopentyl, -CH₂₋cyclopentyl, n-hexyl, sec-hexyl, cyclohexyl, -CH₂-cyclohexyl moieties and the like, which again, may bear one or more substituents. Alkenyl groups include, but are not limited to, for example, ethenyl, propenyl, butenyl, 1-methyl-2-buten-1-yl, and the like. Representative alkynyl groups include, but are not limited to, ethynyl, 2-propynyl (propargyl), 1-propynyl and the like.

The term "alkoxy", or "thioalkyl" as used herein refers to an alkyl group, as previously defined, attached to the parent molecular moiety through an oxygen atom or through a sulfur atom. In certain embodiments, the alkyl group contains 1-20 alipahtic carbon atoms. In certain other embodiments, the alkyl group contains 1-10 aliphatic carbon atoms. In yet other embodiments, the alkyl, alkenyl, and alkynyl groups employed in the invention contain 1-8 aliphatic carbon atoms. In still other embodiments, the alkyl group contains 1-6 aliphatic carbon atoms. In yet other embodiments, the alkyl group contains 1-4 aliphatic carbon atoms. Examples of alkoxy, include but are not limited to, methoxy, ethoxy, propoxy, isopropoxy, n-butoxy, tert-butoxy, neopentoxy and n-hexoxy. Examples of thioalkyl include, but are not limited to, methylthio, ethylthio, propylthio, isopropylthio, n-butylthio, and the like.

The term "alkylamino" refers to a group having the structure -NHR' wherein R' is alkyl, as defined herein. In certain embodiments, the alkyl group contains 1-20 aliphatic carbon atoms. In certain other embodiments, the alkyl group contains 1-10 aliphatic carbon atoms. In yet other embodiments, the alkyl, alkenyl, and alkynyl groups employed in the invention contain 1-8 aliphatic carbon atoms. In still other embodiments, the alkyl group contains 1-6 aliphatic carbon atoms. In yet other embodiments, the alkyl group contains 1-4 aliphatic carbon atoms. Examples of alkylamino include, but are not limited to, methylamino, ethylamino, iso-propylamino and the like.

Some examples of substituents of the above-described aliphatic (and other) moieties of compounds of the invention include, but are not limited to aliphatic; heteroaliphatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)₂Rₓ; -NRₓ(CO)Rₓ wherein each occurrence of Rₓ independently includes, but is not limited to, aliphatic, heteroaliphatic, aryl, heteroaryl, alkylaryl, or alkylheteroaryl, wherein any of the aliphatic, heteroaliphatic, alkylaryl, or alkylheteroaryl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substituents are illustrated by the specific embodiments shown in the Examples that are described herein.

In general, the terms "aryl" and "heteroaryl", as used herein, refer to stable mono- or polycyclic, heterocyclic, polycyclic, and polyheterocyclic unsaturated moieties having preferably 3-14 carbon atoms, each of which may be substituted or unsubstituted. Substituents include, but are not limited to, any of the previously mentioned substitutents, i.e., the substituents recited for aliphatic moieties, or for other moieties as disclosed herein, resulting in the formation of a stable compound. In certain embodiments of the present invention, "aryl" refers to a mono- or bicyclic carbocyclic ring system having one or two aromatic rings including, but not limited to, phenyl, naphthyl, tetrahydronaphthyl, indanyl, indenyl and the like. In certain embodiments of the present invention, the term "heteroaryl", as used herein, refers to a cyclic aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl,oxadiazolyl, thiophenyl, furanyl, quinolinyl, isoquinolinyl, and the like.

It will be appreciated that aryl and heteroaryl groups (including bicyclic aryl groups) can be unsubstituted or substituted, wherein substitution includes replacement of one, two or three of the hydrogen atoms thereon independently with any one or more of the following moieties including, but not limited to: aliphatic; heteroaliphatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)₂Rₓ; -NRₓ(CO)Rₓ wherein each occurrence of Rₓ independently includes, but is not limited to, aliphatic, heteroaliphatic, aryl, heteroaryl, alkylaryl, or alkylheteroaryl, wherein any of the aliphatic, heteroaliphatic, alkylaryl, or alkylheteroaryl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substitutents are illustrated by the specific embodiments shown in the Examples that are described herein.

The term "cycloalkyl", as used herein, refers specifically to groups having three to seven, preferably three to ten carbon atoms. Suitable cycloalkyls include, but are not limited to cyclopropyl, cyclobutyl, cyclopentyl, cyclohexyl, cycloheptyl and the like, which, as in the case of other aliphatic, heteroaliphatic or hetercyclic moieties, may optionally be substituted with substituents including, but not limited to aliphatic; heteroaliphatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)₂Rₓ; -NRₓ(CO)Rₓ wherein each occurrence of Rₓ independently includes, but is not limited to, aliphatic, heteroaliphatic, aryl, heteroaryl, alkylaryl, or alkylheteroaryl, wherein any of the aliphatic, heteroaliphatic, alkylaryl, or alkylheteroaryl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substitutents are illustrated by the specific embodiments shown in the Examples that are described herein.

The term "heteroaliphatic", as used herein, refers to aliphatic moieties which contain one or more oxygen, sulfur, nitrogen, phosphorus or silicon atoms, e.g., in place of carbon atoms. Heteroaliphatic moieties may be branched, unbranched, cyclic or acyclic and include saturated and unsaturated heterocycles such as morpholino, pyrrolidinyl, etc. In certain embodiments, heteroaliphatic moieties are substituted by independent replacement of one or more of the hydrogen atoms thereon with one or more moieties including, but not limited to aliphatic; heteroaliphatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)₂Rₓ; -NRₓ(CO)Rₓ wherein each occurrence of Rₓ independently includes, but is not limited to, aliphatic, heteroaliphatic, aryl, heteroaryl, alkylaryl, or alkylheteroaryl, wherein any of the aliphatic, heteroaliphatic, alkylaryl, or alkylheteroaryl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substitutents are illustrated by the specific embodiments shown in the Examples that are described herein.

The terms "halo" and "halogen" as used herein refer to an atom selected from fluorine, chlorine, bromine and iodine.

The term "haloalkyl" denotes an alkyl group, as defmed above, having one, two, or three halogen atoms attached thereto and is exemplified by such groups as chloromethyl, bromoethyl, trifluoromethyl, and the like.

The term "heterocycloalk-yl" or "heterocycle", as used herein, refers to a non-aromatic 5-, 6- or 7- membered ring or a polycyclic group, including, but not limited to a bi- or tri-cyclic group comprising fused six-membered rings having between one and three heteroatoms independently selected from oxygen, sulfur and nitrogen, wherein (i) each 5-membered ring has 0 to 1 double bonds and each 6-membered ring has 0 to 2 double bonds, (ii) the nitrogen and sulfur heteroatoms may be optionally be oxidized, (iii) the nitrogen heteroatom may optionally be quaternized, and (iv) any of the above heterocyclic rings may be fused to a benzene ring. Representative heterocycles include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl. In certain embodiments, a "substituted heterocycloalkyl or heterocycle" group is utilized and as used herein, refers to a heterocycloalkyl or heterocycle group, as defined above, substituted by the independent replacement of one, two or three of the hydrogen atoms thereon with but are not limited to aliphatic; heteroaliphatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)₂Rₓ; -NRₓ(CO)Rₓ wherein each occurrence of Rₓ independently includes, but is not limited to, aliphatic, heteroaliphatic, aryl, heteroaryl, alkylaryl, or alkylheteroaryl, wherein any of the aliphatic, heteroaliphatic, alkylaryl, or alkylheteroaryl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substitutents are illustrated by the specific embodiments shown in the Examples which are described herein.

### 4) Synthetic Methodology

As described above, access to analogues of radicicol and monocillin was previously limited to compounds accessed via the natural products (see, for example, US Patent 5,650,430; US Patent 5,731,343; US Patent 6,239,168; US Patent 5,977,165; and US Patent 5,597,846). In recognition of the need for an efficient and practical route to this class of therapeutic agents, the present invention provides novel synthetic methodologies for the synthesis of radicicol, monocillin, and analogues, and conjugates thereof. Although the synthesis of radicicol and monocillin is described specifically herein directly below (and in the Examples), it will be appreciated that this methodology is generally applicable to the generation of analogues and conjugates as discussed in more detail after the discussion of the synthesis of radicicol and monocillin.

### a) Synthesis of Radicicol and Monocillin:

As discussed above, prior to the present invention, only one synthesis of radicicol had been achieved (see Lampilas et al. Tetrahedron Lett. ***1992,** 33*, *773 and 777*). This synthesis, while novel, is lengthy and not suitable to the rapid generation of analogues. As described in more detail below, a novel synthetic route to radicicol and monocillin has been developed, which methodology allows for the efficient generation of a variety of analogues of radicicol, monocillin, homodimers, heterodimers, and conjugates thereof.

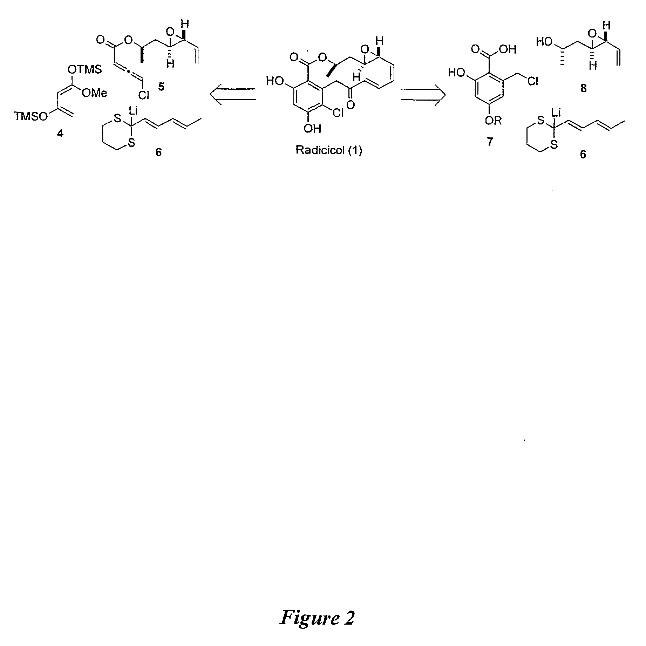

As shown in Figure 2, two strategies were developed for the synthesis of radicicol and monocillin, each of which relied on a highly convergent three stage coupling for macrolide formation. The first approach relied on a Diels Alder reaction of bis-1,3-trimethylsilyoxy-1-methoxy-butadiene (**4**) with 1,3 unsymmetrically di-substituted allene (**5**). Significantly, this allene cycloaddition chemistry constitutes a concise and powerful entry to substituted benzo structures of some considerable complexity. The product of this Diels Alder cycloaddition was then ligated to the requisite diene through chemo- and regioselective addition of an allylic dithiane (**6**) to the resulting benzylchloride of **4** + **5**. Lastly, stereospecific ring-closing metathesis of an olefin with a vinyl epoxide in such a context gave the desired 14-membered novel lactone cyclization. The second strategy relies on a direct Mitsunobu esterification of an appropriately substituted benzoic acid derivative (**7**) with the vinyl epoxide fragment (**8**). This product intersects with the first strategy, and is completed in the same fashion. This second strategy has the distinct advantage of introducing the valuable vinyl epoxide fragment at a later stage, thus being more amenable to analog generation.

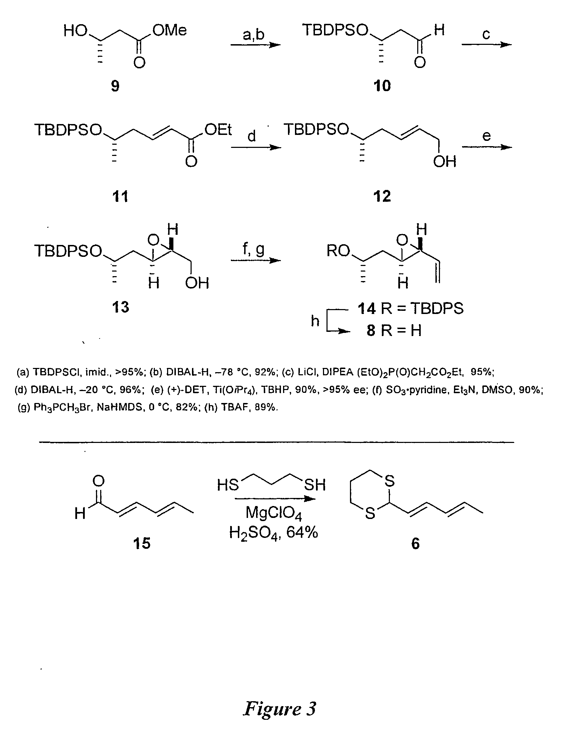

As shown in Figure 3, the synthesis commenced with construction of the chiral allylic alcohol (**8**) via a procedure which is similar to that described by Waldmann and co-workers. Thus methyl-(*S*)-3-hydroxybutyric acid (**9**) was silylated (TBDPSCl, imidazole, 95%) and the product reduced at low temperature (DIBAL-H, -78 °C, 92%) to provide directly the desired aldehyde (**10**). Wadsworth-Horner-Emmons homologation of **10** under Roush-Masamune conditions (LiCl, DIPEA, 95%) followed by a second reduction (DIBAL-H, 96%) yielded the desired *trans-*allylic alcohol (**12**). Sharpless asymmetric epoxidation ((-)-DET, Ti(O*i*Pr)₄, TBHP, 90%) gave the desired epoxyalcohol (**13**) with excellent (> 20:1) selectivity. The resulting epoxyalcohol was then oxidized (SO₃•pyridine, Et₃N, DMSO, 90%) and converted to the vinyl epoxide (**14**) via Wittig olefmation (PPh₃CH₃Br, NaHMDS, 82%). Fluoride-catalyzed removal of the TBDPS group proceeded smoothly (*n*Bu₄NF, 89%) to yield the desired secondary alcohol (**8**). The allylic dithiane (**6**), was secured in one step from commercially available 2,4-hexadienal (**15**) (MgClO₄, H₂SO₄, H₂S(CH₂)₃SH₂, 64%).

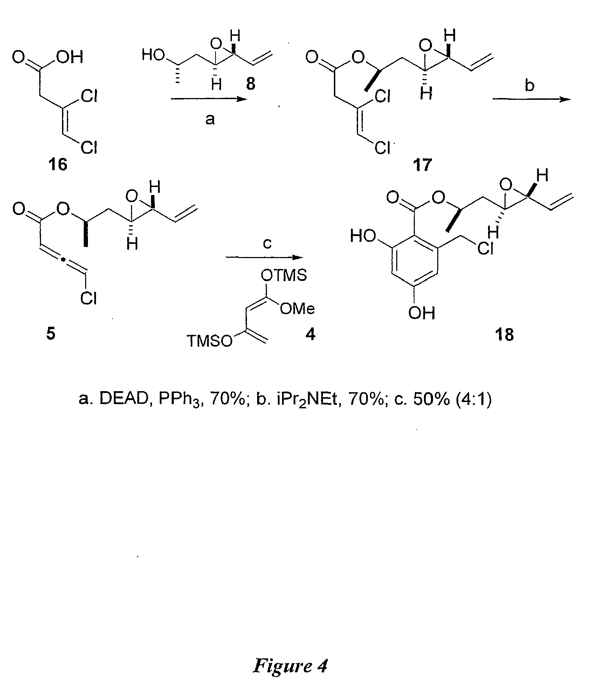

The first strategy then required the construction of the unsymmetrically substituted allene. These syntheses required the vinyl epoxide fragment containing all three stereocenters to be appended to the acid **16** via a Mistunobu esterification (70%, Figure 4). This was followed by an elimination to reach the desired allene **5** (70%). In this case, Diels Alder cycloaddition between **5** and the diene **4** afforded the desired resorcinylic chloride **18** as the major product (50%, 4:1 regioselectivity). This intermediate was carried to the end of the synthesis of **1**, and following mono-protection of the para-phenol, intersects with the below strategy.

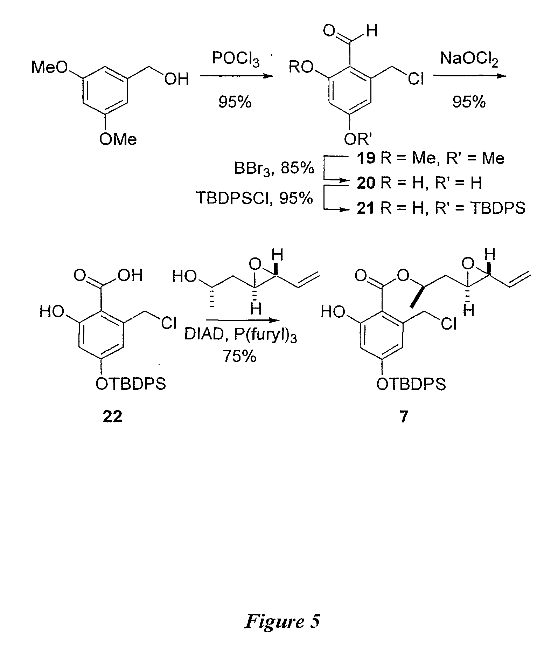

The alternative strategy (Figure 5) prompted the synthesis of an appropriately substituted benzoic acid, followed by Mitsunobu esterification of the vinyl epoxide fragment (**8**). Commercially available 3,5-dimethoxy benzyl alcohol was simultaneously formylated and chlorinated (POCl₃, DMF, 93%) to give the desired aldehyde (**19**). The methyl ethers were removed by BBr₃ (85%) and the para-phenol was selectively protected with TBDPSCl (95%) to furnish **21**. Then careful oxidation of this aldehyde (NaClO₂, sulfamic acid, 95%) yielded the desired benzoic acid (**22**) with no observed cyclization and no ring chlorination.

It was also found that employment of the free ortho phenol **22** in a Mitsunobu reaction resulted in a superior coupling that occurred without competing intramolecular cyclization when using trifurylphosphine in benzene as a solvent (Figure 5). The resulting ester intersects the above route to **1** and **2** and is directly used in the subsequent dithiane alkylation reaction. This second solution has a distinct advantage in the synthesis of analogues as it introduces the fragment with all three stereogenic centers at the latest possible stage. Both provide routes to an otherwise difficult assembly.

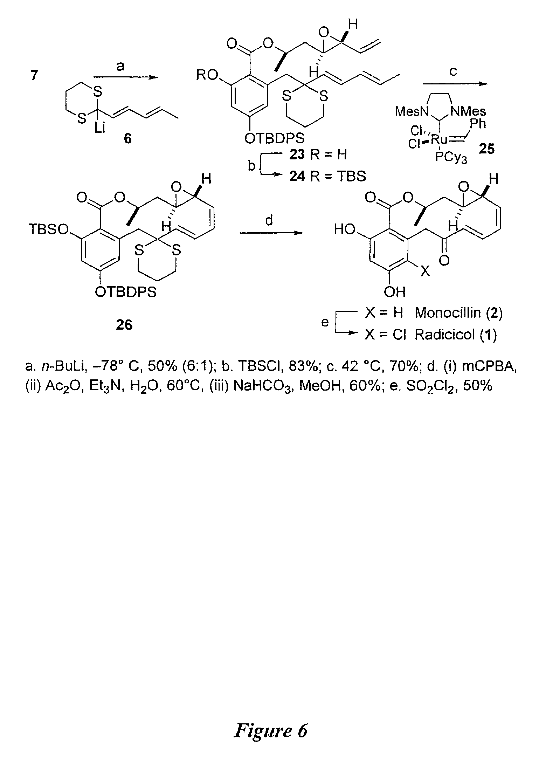

It also was found that the free ortho phenol **7** was a remarkably efficient substrate for the subsequent dithiane alkylation (Figure 6). Addition of **6** to the lithium salt of the benzyl chloride (**7**) proceeded with good (6:1) α:γ regioselectivity (50%) and subsequent protection was uneventful (TBSCl, 83%). For achieving macrolide formation a ring closing metathesis of a diene and a vinyl epoxide as are present in **24** was envisioned. Other resorcylic acid type macrolides have been elegantly reached by Fürstner and coworkers utilizing olefin metathesis, although not utilizing vinyl epoxide precursors (see, Furstner *et al. Tetrahdron* **1999***, 55*, 8215). In a recent communication, Grubbs and coworkers reported the successful intermolecular cross-coupling of a vinyl epoxide utilizing a new generation, and highly active ruthenium-based olefin metathesis catalyst (**25**)**.** Ring-closing metathesis of the vinyl epoxide and the diene with the new generation Grubbs catalyst (Grubbs *et al.*, *J. Am. Chem. Soc.* **2000,** *122,* 3783-3784) closed the 14-membered ring **26** stereospecifically in 70% yield. Notably, this transformation was also performed in the presence of two sulfur atoms. The unusual Pummerer-like dithiane removal, followed by hydrolysis of the resulting phenolic acetates, served as a global deprotection and gave monocillin I (**2**) in 58% yield (see, for example, hishi *et al. J. Am. Chem. Soc.* **1973**, *95,* 6490; Fukuyama *et al. Tetrahedron* **1981**, *37*, 2045; Smith *et al. J. Am. Chem. Soc.* **1986**, *108,* 3110). Finally, a regioselective, high-yielding chlorination was discovered using SO₂Cl₂ in Et₂O (55%) to directly convert monocillin I into radicicol (**1**).

### a)General Synthetic Methodology:

Clearly, the synthetic methodology as described above provides for the rapid synthesis of a variety of analogues of radicicol, monocillin, dimers, and conjugates thereof. It will be appreciated that the ability to rapidly generate a range of analogues is important because it is believed that in vivo activity is lost due to certain structural characteristics of radicicol and monocillin. For example, it is postulated that in vivo activity is lost due to the nucleophilic action of cellular thiols (i.e. glutathione) on either the epoxide or the α,β unsaturated ketone of radicicol. This nucleophilic addition changes the overall conformation of radicicol, and results in an inability to bind to Hsp90. A second likely pathway of deactivation involves the conjugation of the aromatic ring, or perhaps cytochrome P-450 oxidation.

Without wishing to be bound by any particular theory, one strategy to restore in vivo activity would be to reduce the affinity of radicicol to nucleophiles such as thiols, specifically to deactivate electrophilic sites in radicicol. Here care must be taken not to dramatically change the overall conformation of the natural product. Thus these analogues have been designed to attenuate electrophilicity with simple alterations to the structure that should not affect the overall conformation. It should be emphasized that the analogues as described using the methodology herein cannot be made from the natural product. The three component nature of this process described above and described more generally below emphasizes the ability to generate numerous analogues by the modification of one component and its incorporation into a short and efficient process.

In general, the method involves the synthesis of analogues from three easily obtainable and diversifiable components. Depicted below is a general retrosynthetic strategy for the synthesis of analogues:

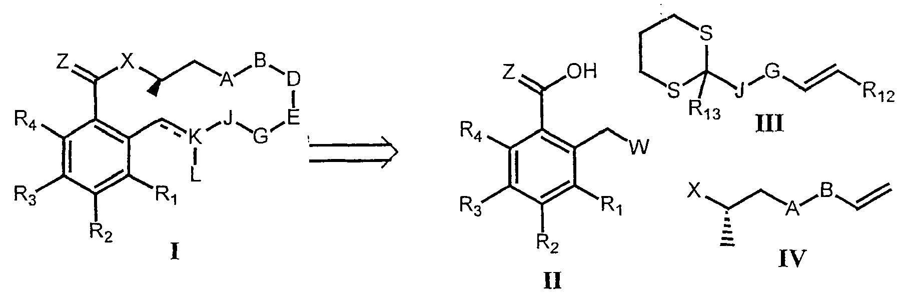

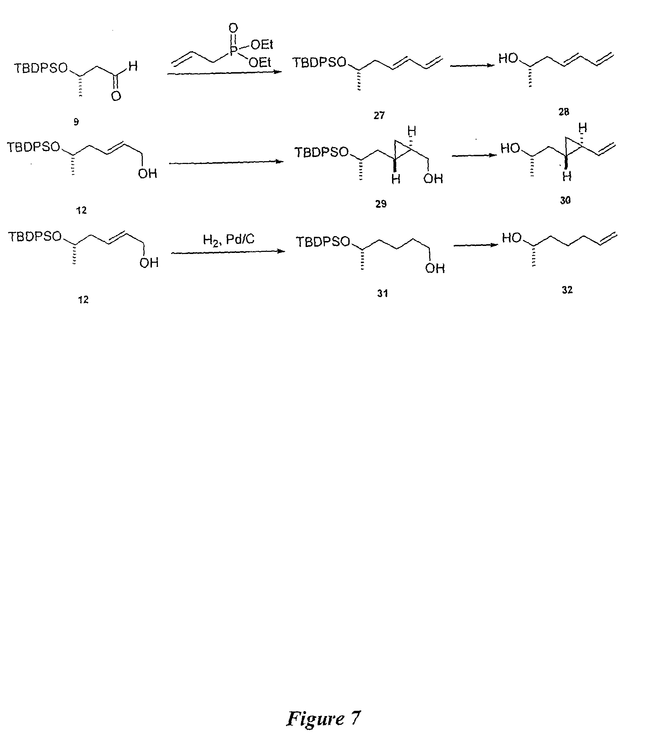

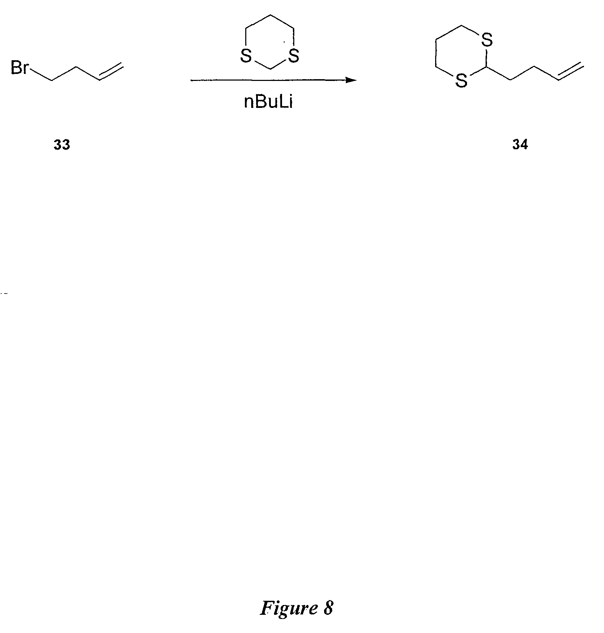

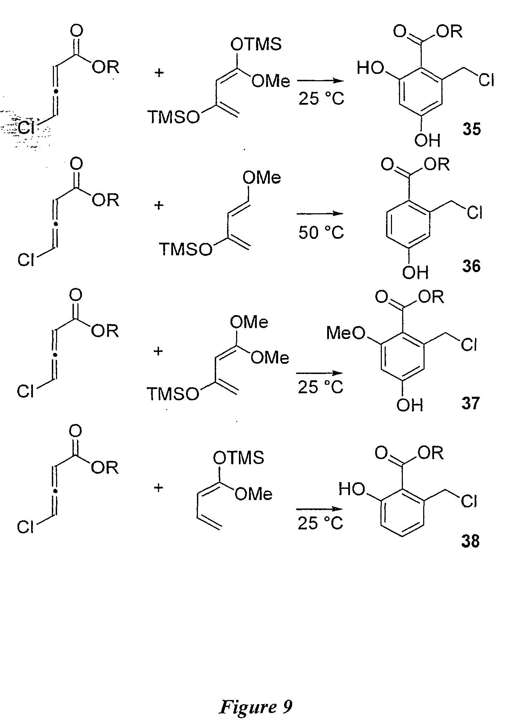

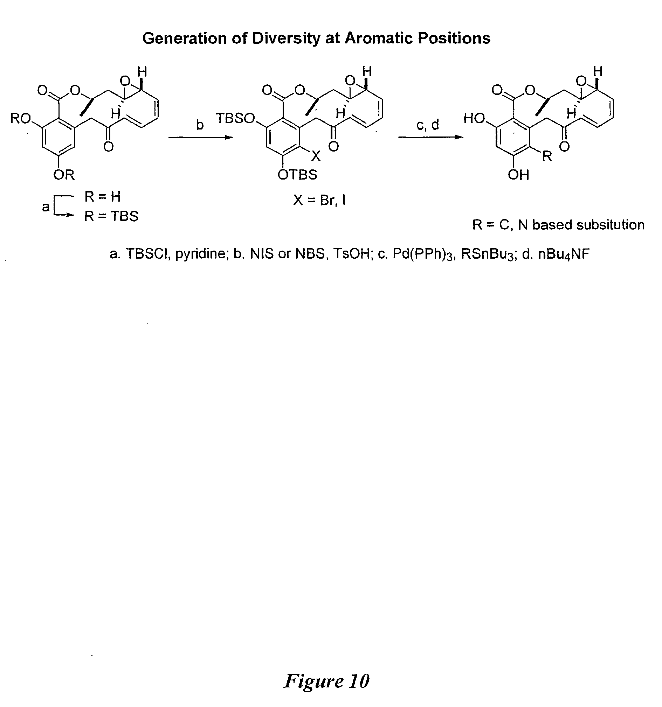

For simplicity, the components varied can be called: 1) the chiral component **IV** (exemplary embodiments of which are shown in Figure 7), 2) The dithiane component **III** (an exemplary embodiment of which is shown in Figure 8), and 3) the benzoic acid component **II** (exemplary embodiments of which are shown in Figures 9 and 10). It should be noted that these analogs can be generated either with a single modification, or they may be combined in a single entity to maximize their benefit if they are found to be synergistic. It will also be appreciated, as described in more detail herein, that each of the components can be diversified prior to formation of the macrocycle, or alternatively or additionally, can be diversified after formation of the macrocycle.

As depicted generally below, the synthesis of analogues can be carried out in a similar fashion to the synthesis of radicicol and monocillin as described above: 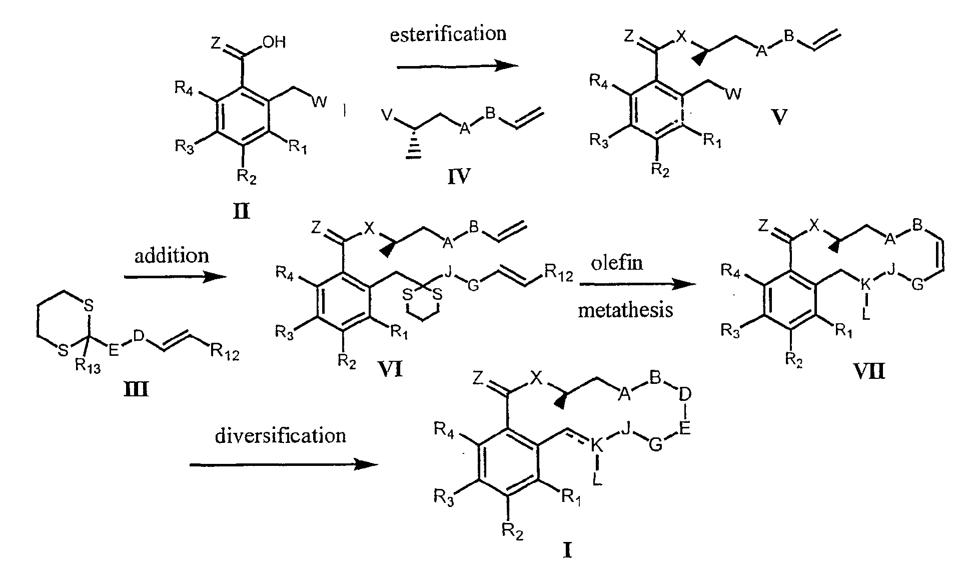 wherein each of R₁-R₄, Z, X, A-B, D-E, G-J, K-L is as defined above generically and as defined in classes and subclasses described herein, and R₁₂ is hydrogen or lower alkyl, R₁₃ is hydrogen or an alkali metal, W is a halogen and V is OH, SH or NHR_{G}, wherein R_{G} is as defined above and herein.

Reaction of the benzoic acid component **(II)** (which can be diversified prior to the esterification reaction, as exemplified herein), with an esterification reagent and a chiral component **(IV)** yields intermediate **(V)**. Subsequent reaction with a dithiane component (which can also be diversified prior to reaction as exemplified herein) under suitable conditions to effect addition yields intermediate **(IV)**. This intermediate can then be treated with an olefin metathesis catalyst under suitable conditions to effect olefin metathesis to generate intermediate **(VII)**. This intermediate can then be further reacted to diversify certain potentially reactive sites in the molecule (e.g., double bond generated after the olefin metathesis, aromatic positions, if A-B is an epoxide, the epoxide can be opened to generate further sites of diversity that can be reacted with still other reagents (e.g., linker-conjugate moieties), to name a few), or can be further reacted to deprotect any sites (e.g., free alcohols, amines) that may have been protected during the synthesis of the analogues.

Thus, in addition to providing inventive compounds as described above and herein, the present invention additionally provides a method for the synthesis of compounds having the general structure **(I)**: 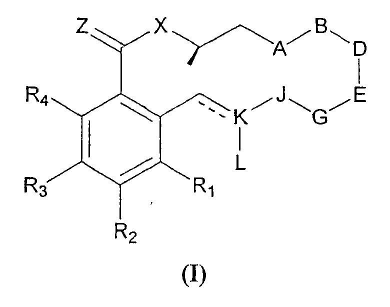 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein R_{F} is hydrogen, a protecting group, an aliphatic, heteroaliphatic, aryl, heteroaryl; alkylaryl, or alkylheteroaryl moiety;
X is O, S or NR_{G}, wherein R_{G} is hydrogen or lower alkyl;
A and B together represent 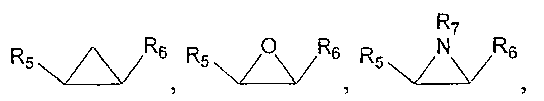 -CHR₅-CHR₆-, -CR₅=CR₆-, wherein R₅ and R₆ are each independently hydrogen, halogen, cyano, -OR_{J}, -N(Rⱼ)₂, -SRⱼ, -O(C=O)Rⱼ, -O(S=O)Rj ,-N(R_{J})(C=O)(R_{J}), -C(=O)Rⱼ, -C(=O)OR_{J}, -CON(Rⱼ)₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -C(O)NR_{K}, -S(O)₂R_{K}, -O(C=O)R_{K}, -N(R_{K})(C=O)(R_{K}), -C(O)R_{K}, -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅₋CHR₆-, R₅ and R₆ taken together represent a substituted or unsubstituted 3-7 membered aliphatic, heteroaliphatic, aryl or heteroaryl ring,
D and E together represent -CHR₈-CHR₉-, -CR₈=CR₉-, wherein R₈ and R₉ are each independently hydrogen or lower alkyl;
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-)-, CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geldanamycin, analogues of geldanamycin, and steroids,
said method comprising:
(1) reacting a benzoic acid component having the structure: 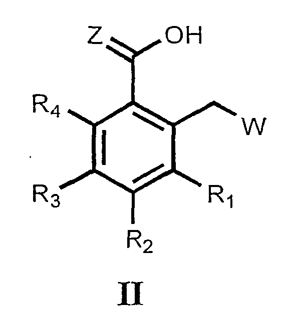 wherein R₁-R₄ and Z are as defined above, and wherein W is halogen, with a chiral component (**IV**) having the structure: 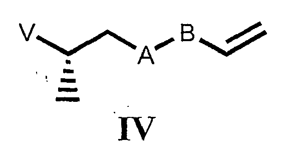 wherein A and B are as defined above, and wherein V is NHR_{G}, wherein R_{G} is hydrogen or lower alkyl, SH, or OH in the presence of an esterification reagent to generate an intermediate **(V)** having the structure: 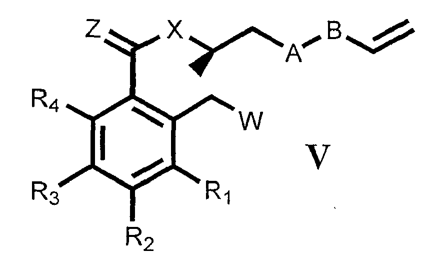
(2) reacting the intermediate (V) with a dithiane having the structure (**III**)**:** 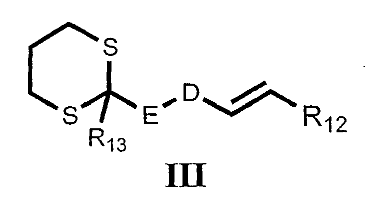 wherein R₁₃ is hydrogen or an alkali metal salt and wherein R₁₂ is hydrogen or lower alkyl,
   under conditions to add the dithiane to generate an intermediate (VI) having the structure: 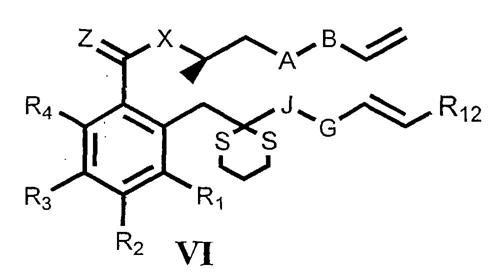
(3) if any one or more of R₁-R₄ is an unprotected thio, amino or hydroxyl group, optionally protecting said unprotected group;
(4) cyclizing the intermediate (**VI**) in the presence of an olefin metathesis catalyst to generate the compound (**VII**): 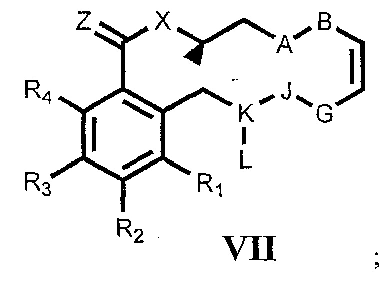 and
(5) optionally further reacting the macrocycle (**VII**) with one or more reagents to diversify and optionally deprotecting the macrocycle to generate a compound having the formula (**I**).

In addition to the general method described and depicted above, the present invention provides additional synthetic methods, and compounds, as described herein, in which each of the intermediate steps and intermediate compounds **(II)**, **(III)**, **(IV)**, **(V)**, **(VI)** and **(VII)** are provided, as described below and generically herein. It will be appreciated that the classes, and subclasses, as described above for the inventive compounds are also intended to encompass the inventive methods and intermediate compounds as described above and herein. Thus, certain classes and subclasses of interest in which the moieties R₁-R₄, Z, X, A-B, D-E, G-J and K-L are specifically defined (and moieties defined within those definitions) also apply to the inventive methods and intermediate compounds. It will be appreciated that certain exemplary species of the compounds of formula (**I**) and intermediates **(II)**, **(III)**, **(IV)**, **(V)**, **(VI)** and **(VII)** are described herein, but are not limited to those species.

More generally, the present invention additionally provides a method for the synthesis of a macrocycle having the structure: 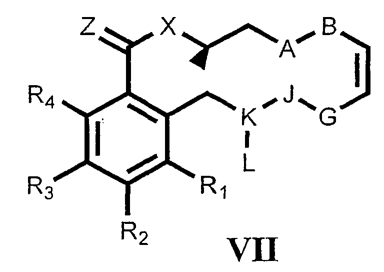 as defined above and herein, wherein said method comprises cyclizing the intermediate (**VI**): 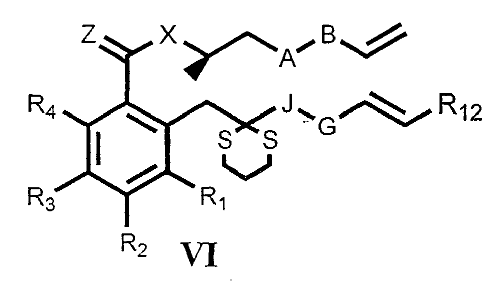 in the presence of an olefin metathesis catalyst to generate the compound (**VII**).

In certain embodiments, if any one or more of R₁-R₄ is an unprotected thio, amino or hydroxyl group, the method further comprises optionally protecting said unprotected group.

In still other embodiments, the method optionally further comprises reacting (**VII**) with one or more reagents to diversify the macrocycle and generate a compound having the structure (**I**). In yet other embodiments, the method further comprises optionally deprotecting the compound having the structure (**I**), to generate a deprotected compound having the structure **(I)**.

It will be appreciated that in certain embodiments of the methods as described above and herein W is Cl, V is OH or NHR_{G}, R₁₃ is Li or hydrogen, and R₁₂ is hydrogen or methyl. It will also be appreciated that each of the steps as described above can be carried out using reagents and conditions as described for the synthesis of radicicol and monocillin, or they may be modified using other available reagents. For example, a variety of esterification conditions, dithiane addition and olefin metathesis conditions are well-known in the art and can be utilized in the method of the invention. See, generally, March, Advanced Organic Chemistry, John Wiley & Sons, 1992.

As mentioned above, it will also be appreciated that each of the components used in the synthesis of analogues can be diversified either before synthesis or alternatively after the construction of the macrocycle. As used herein, the term "diversifying" or "diversify" means reacting an inventive compound **(VII)** or **(I)**, as defined herein, at one or more reactive sites to modify a functional moiety or to add a functional moiety. For example, the aromatic ring can be diversified (prior to or after reaction) to either add functionality (e.g., where hydrogen is present, a halogen, e.g., Cl, can be added) or to modify functionality (e.g., where a hydroxyl group is present on the aromatic ring, the aromatic ring can be diversified by reacting with a reagent to protect the hydroxyl group, or in another example, by reacting with a reagent to add a linker moiety that has a conjugate (e.g., geldanamycin, etc.) attached thereto). Described generally below are a variety of schemes to assist the reader in the synthesis of a variety of analogues, either by diversification of the intermediate components or by diversification of the macrocyclic structures **(VII)** and **(I).**

For example, the chiral component can be easily modified as depicted in Figure 7. The aldehyde **9** can be homologated directly to the diene **27**, and then deprotected to give the alcohol **28**. Alternatively the epoxide can be replaced with a cyclopropane, a far less reactive electrophile, by using a diastereoselective cyclopropanation reaction developed by Charette et al (**30**). The cyclopropyl alcohol would be converted to the final fragment using the known route. In addition, the epoxide can be simply removed by hydrogenation of the allyl alcohol **12** and then straightforward oxidation, olefination and deprotection to give **32**.

The dithiane fragment is also easily modified, and can effect significant advantages to the analogs accessed. Figure 8 depicts one exemplary embodiment in which commercially available 4-bromobutene **33** can be alkylated by lithiated 1,3-dithiane to provide a dithiane fragment alternative **34**. It will be appreciated that other modifications can be effected to generate additional diversity prior to addition of this component.

Additionally, the aromatic ring can incorporate numerous changes, just a few examples are presented in Figure 9. Here novel Diels Alder methodolgy is utilized to present different substitution patterns around the benzene ring **35-38**. It will be appreciated that traditional aromatic synthesis can also be utilized to access permutations on the aromatic core. For example, as shown in Figure 10, diversity can be generated at aromatic positions, in one embodiment, after generation of the core structure. Specifically, R₁ and R₃ include substitution patterns arising from a cross-coupling strategy as depicted to enable introduction of amino, aliphatic, heteroaliphatic, aryl, heteroaryl, and alkylheteroaryl moieties.

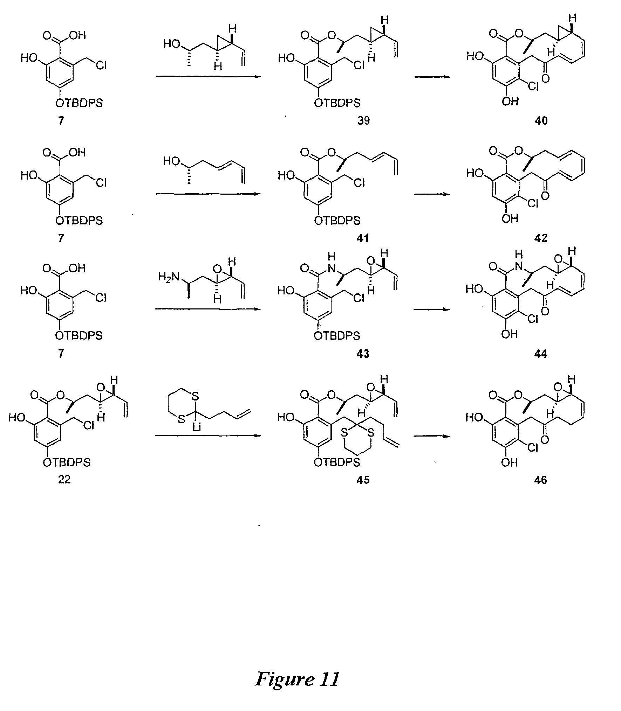

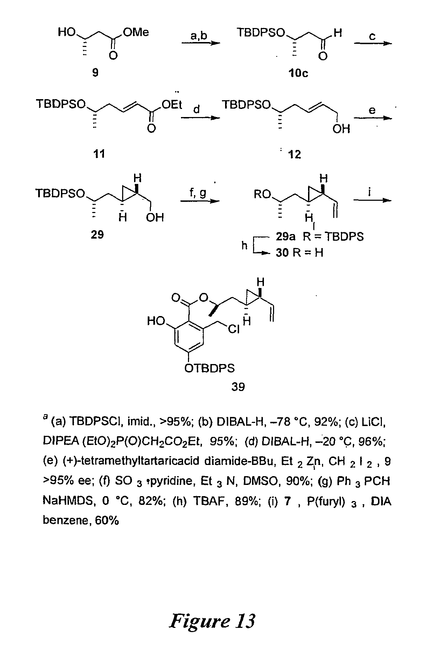

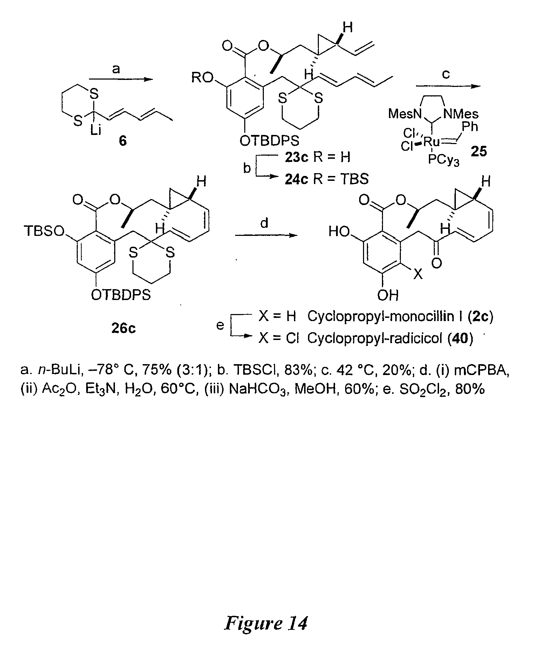

Figure 11 depicts a variety of analogues that can be synthesized using the methodology as described herein. For example, the first two analogs (**40** and **42**) recognize the electrophilicity of the vinyl epoxide, and replace it with significantly less reactive functionality. Specifically, the cyclopropane and the olefin are far less reactive to many electrophiles, and will serve to closely maintain the overall conformation of the molecule. Exclusion of the epoxide according to Scheme **5** gives the desepoxy compound, or alternatively, the related analog with no double bond at that position. One exemplary embodiment of the synthesis of two cyclopropyl analogues **(2c)** and **(40)** is described in detail in Figures 13 and 14.

The third analog (**44**) serves to replace the labile ester functionality with a stronger amide component. Esterase activity is responsible for the premature metabolism of many potential therapeutic agents, and therefore this analogs aims to prevent this activity. As depicted in Figure 7, simple modification of the chiral component and incorporation into this process provides a facile route to this analog.

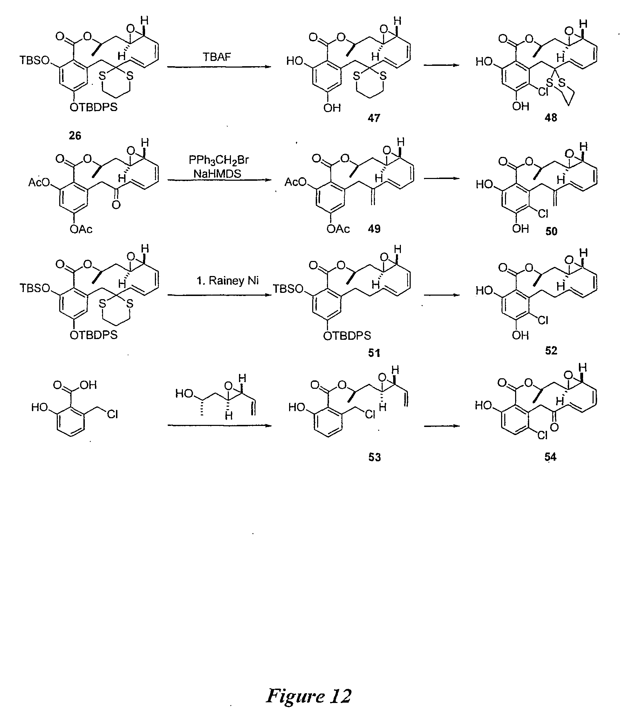

The second set of analogs (**46, 48, 50, 52**) (see Figures 11 and 12) reduce the electrophilicity of the αβ-unsaturated ketone moiety. These represent replacement of the ketone with functionality that are not electron withdrawing, and therefore deactivate the system to nucleophilic addition (Michael addition), and to minimize conformational impact. Analog **46** removes the double bond thereby ruling out the possibilty of a thiol related Michael component to radicicol's deactivation. Analogs **48, 50, 52** remove the carbonyl influence with a dithiane, olefin and an aliphatic domain respectively, and represent examples of diversification of the macrocycle core structure after ring closure via olefin metathesis.

Lastly the aromatic domain can be modified (an exemplary embodiment of modification prior to esterification, dithiane addition and macrocycle formation) and analog **54** is presented as an exemplary analogue.

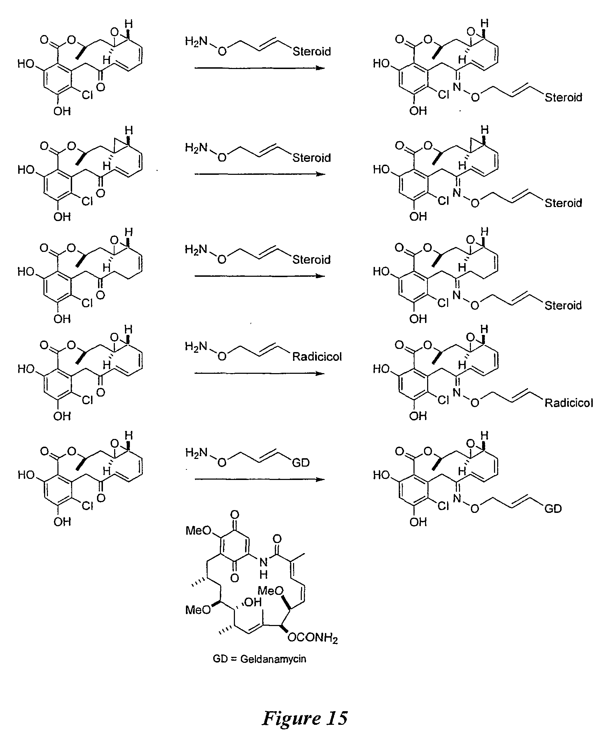

As detailed above, a variety of reactions can be utilized to diversify the radicicol and/or monocillin core structures either during assembly or after assembly of the macrocycle. An alternative strategy endeavours not only to restore in vivo activity and achieve diversity, but also to enhance it. In a fashion similar to that produced with geldanamycin, a number of known steroid hormones can linked to radicicol and analogs via an oxime technology that has already been shown to restore in vivo activity (see US Patent 6,239,168; US Patent 5,977,165 and Soga *et al. Cancer Res.* **1999**, *59,* 2931-2938). The steroid hormones bind specifically to receptors found in important Hsp90 complexes. In addition, dimers of radicicol or heterodimers of radicicol and geldanamycin will be used to probe the activity of bifunctional binding agents on Hsp90 activity (see Figure 15).

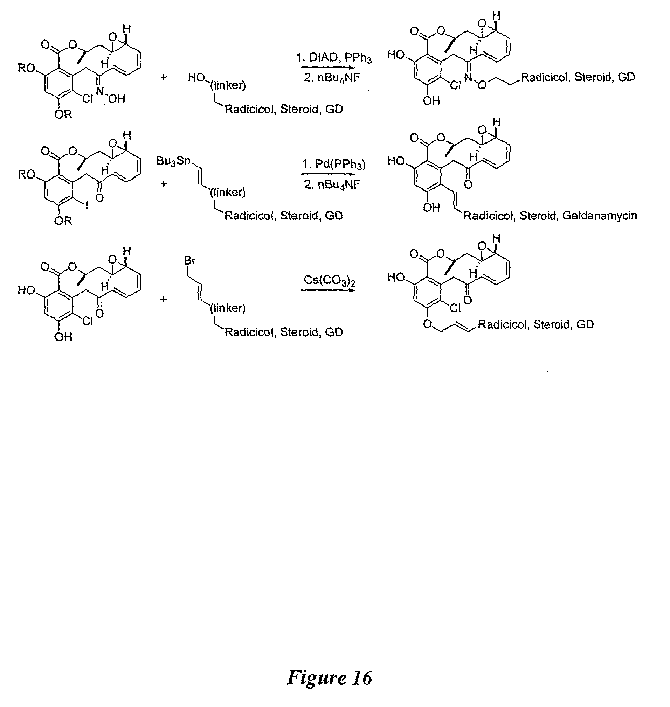

In addition to conjugation via oxime technology as described above and depicted in Figure 15, it will be appreciated that conjugation can be effected through available functionality on the aromatic ring or through the A-B moiety, as described generally herein. Figure 16 depicts a variety of methods that can be utilized to effect conjugation.

It will be appreciated that a variety of linkers can be utilized to effect conjugation of geldanamycin, radicicol, monocillin and analogues thereof and steroids to the inventive compounds. As described above, any one or any two of R₁, R_{A}, R₂, R_{B}, R₃, R_{C}, R₄, R_{D}, R₅, R₆, R_{J}, or R_{L} may potentially be a site for conjugation and conjugation can be effected through a linker covalently bonded to a compound to be conjugated. The term "linker" as used herein, is intended to encompass a chemical moiety that is capable of effecting a stable (e.g., sufficiently unhindered that the conjugation can be performed) covalent linkage between an inventive compound as described herein, and another conjugate (geldanamycin, radicicol, monocillin, analogues thereof as described herein and elsewhere) and steroids. It will be appreciated that a variety of linkers can be utilized, including, but not limited to heteroatom linkages (e.g., O-S(=O)O, -O-(C=O)-O-, heteroalkyl, etc.), and aliphatic or heteroaliphatic linkages, in which the aliphatic and heteroaliphatic linkages may be substituted or unsubstituted, branched or unbranched, or cyclic or acyclic. In certain embodiments for the compounds as described above, the linker is an aliphatic or heteroaliphatic moiety, whereby said aliphatic or heteroaliphatic moiety is substituted or unsubstituted, branched or unbranched, or cyclic or acyclic. For example, it will be appreciated that this linker may be of varying length, and that altering the length of the linker may, in certain circumstances, confer a therapeutic benefit. In general, the linker may be 1-12 carbon atoms in length, and may also be 1-10, 1-6, or 1-4 carbon atoms in length, in other embodiments of special interest. As described above, the linker may be a linear chain or a substituted chain, for example incorporating double or triple bonds, an aryl group or a secondary or tertiary amine. In certain other embodiments for the compounds as described above, the linker is a moiety having one of the structures -(CH₂)ₙ-CH=CH-(CH₂)ₘ-, -(CH₂)ₚ₋C≡C-(CH₂)_{q}-, or -CH₂(CH₂)ₛCH₂-, wherein each occurrence of n, m, p, q and s is independently an integer from 0-10. As described generally above, it will be appreciated that one or more of the hydrogen atoms may be replaced with a substituent including, but not limited to alkyl, heteroalkyl, secondary or tertiary amine, hydroxyl, thiol, aryl, heteroaryl, alkylaryl, or alkylheteroaryl. Similar dimers, trimers, and conjugates and linkers used for the conjugation thereof are described in more detail in Kuduh *et al*., *Bioorg*. *Med*. *Chem*. *Lett.* **2000**, *10*, 1303-1306; *Kuduk et al*. *Bioorg*. *Med*. *Chem*. *Lett*., **1999**, *9*, 1233-1238; Zheng *et al*. *Cancer Res*. **2000**, 60, 2090-2094; and WO00/61578). Additional guidance for the preparation of conjugates can be found in US Patent 5,977,165 (in which, for example, two radiciciol derivatives are linked via -O-S(=O)-O-).

In certain embodiments, the compound to be conjugated is selected from the group consisting of radicicol, monocillin, analogues of radicicol and monocillin, geldanamycin, analogues of geldanamycin, and steroids. In particular, any of the compounds of the present invention may be conjugated with one or two compounds of the same structure or with one or two compounds of different structures, such as geldanamycin, analogues thereof and steroids. The term "analogues", as used herein, is intended to encompass radiciciol analogues as described herein and elsewhere (see e.g., 5,977,165, 5,731,343, and 5,597,846) and geldanamycin analogues generally described in the art (see, e.g., WO00/61578). It will be appreciated that a variety of steroids can be utilized in the method of the present invention. In certain embodiments, steroids are utilized to develop selective cytotoxic agents directed towards cancer cells that express steroid receptors (e.g., estrogen receptor). Suitable steroids for use in the present invention include, but are not limited to, estradiol, estradiol valerate, estradiol cypionate, ethinyl estradiol, mestranol, quinestrol, estrone, estrone sulfate, equilin, testosterone, androstenedione, dehydroepiandrosterone, estriol 16α-hydroxydehydro-epiandrosterone, and 16α-hydroxyandrostenedione, to name a few.

### 5) Uses, Formulation and Administration

### Pharmaceutical Compositions

As discussed above this invention provides novel compounds which have biological properties which make them of interest for the treatment of cancer, in particular those cancers characterized in that they comprise Rb negative cancer cells. Accordingly, in another aspect of the present invention, pharmaceutical compositions are provided, which comprise any one of the compounds described herein (or a prodrug, pharmaceutically acceptable salt or other pharmaceutically acceptable derivative thereof), and optionally comprise a pharmaceutically acceptable carrier. In certain embodiments, these compositions optionally further comprise one or more additional therapeutic agents. Alternatively, a compound of this invention may be administered to a patient in need thereof in combination with the administration of one or more other therapeutic agents. For example, additional therapeutic agents for conjoint administration or inclusion in a pharmaceutical composition with a compound of this invention may be a cytotoxic agent or anticancer agent approved for the treatment of cancer, as discussed in more detail herein, or it may be any one of a number of agents undergoing approval in the Food and Drug Administration that ultimately obtain approval for the treatment of cancer (e.g., epothilones, geldanamycin, to name a few). It will also be appreciated that certain of the compounds of present invention can exist in free form for treatment, or where appropriate, as a pharmaceutically acceptable derivative thereof. According to the present invention, a pharmaceutically acceptable derivative includes, but is not limited to, pharmaceutically acceptable salts, esters, salts of such esters, or a prodrug or other adduct or derivative of a compound of this invention which upon administration to a patient in need is capable of providing, directly or indirectly, a compound as otherwise described herein, or a metabolite or residue thereof.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts of amines, carboxylic acids, and other types of compounds, are well known in the art. For example, S. M. Berge, *et al.* describe pharmaceutically acceptable salts in detail in *J*. *Pharmaceutical Sciences, 66:* 1-19 (**1977**), incorporated herein by reference. The salts can be prepared in situ during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Additionally, as used herein, the term "pharmaceutically acceptable ester" refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyrates, acrylates and ethylsuccinates.

Furthermore, the term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

As described above, the pharmaceutical compositions of the present invention additionally comprise a pharmaceutically acceptable carrier, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Fifteenth Edition, E. W. Martin (Mack Publishing Co., Easton, Pa., 1975) discloses various carriers used in formulating pharmaceutical compositions and known techniques for the preparation thereof. Except insofar as any conventional carrier medium is incompatible with the anti-viral compounds of the invention, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solution, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

### Uses of Compounds of the Invention

As described in more detail herein, in general, the present invention provides compounds useful for their ability to inhibit the growth of or kill cancer cells and thus are useful in the treatment of cancer. The compounds of the invention are also useful as inhibitors of Hsp90 and thus are useful, more generally as inhibitors of proteins such as transmembrane receptors (e.g., HER2, androgen receptor, erbB, EGFR, etc.), tyrosine kinases, serine and/or threonine kinases, transcriptional regulators, or of proteins that regulate them. In certain embodiments, the inventive compounds are also useful for the inhibition of the growth of or for the killing of Rb negative cancer cells and thus are useful in the treatment of cancers comprising Rb negative cancer cells. In certain other embodiments, the inventive compounds are also useful for' the destruction of cells expressing a HER-family tyrosine kinase. In still other embodiments, the inventive compounds are useful as inhibitors of the androgen receptor.

In general, the unregulated growth characteristic of cancer cells typically results from disruption of a mitogenic signal transduction pathway. Such pathways can be disrupted at any of a number of points, through activation or inhibition of proteins such as transmembrane receptors (e.g., HER2, which is often overexpressed in breast cancers; steroid receptors such as the androgen receptor, which is often overexpressed in prostate cancers; erbB; EGFR; etc.), tyrosine kinases (including those that are domains of transmembrane receptors), serine and/or threonine kinases (e.g., Akt; Raf; Src; etc.), transcriptional regulators (e.g., Rb; STATs; etc.), or of proteins that regulate them. For instance, the molecular chaperone Hsp90 is required for proper folding of a variety of signal transduction proteins, including, for example, steroid receptors, HER2, met, Akt, Raf, etc. When Hsp90 activity is blocked, these proteins are degraded, and mitogenic signal transduction is attenuated.

It has been previously demonstrated that geldanamycin acts as an Hsp90 inhibitor; administration of this compound to tumor cells results in degradation of Hsp90-regulated proteins and arrest in the G1 phase of the cell cycle. Tumors that express high levels of HER2 are particularly sensitive to such agents. For example, treatment of tumors with geldamycin leads to a dose dependent reduction in HER2 levels as well as visible cellular differentiation. Unfortunately, the cell cycle arrest observed after treatment with these compounds is dependent upon the retinoblastoma (Rb) protein. Geldanamycin is currently in Phase II clinical trials, however, geldanamycin has been shown to be ineffective for the treatment of tumor cells with defective Rb function.

Unexpectedly, the present invention demonstrates that radicicol and radicicol analogs function as Hsp90 inhibitors independent of Rb function. Tumor cells treated with these compounds arrest in G1 in the presence of Rb, and arrest in the prometaphase stage of mitosis in the absence of Rb. Unlike geldanamycin, such compounds are therefore useful for the treatment of Rb-positive and Rb-negative cancers. Importantly, there are cancers which currently lack sufficient treatment, and are comprised of Rb negative cells. These include, but are not limited to, small-cell lung carcinoma, glioblastoma (brain) and retinoblastoma (eye). Small-cell lung carcinoma does not have an effective treatment, results in a high mortality rate, and represents 25% of lung cancers. In some embodiments, it may be desirable to combine administration of the inventive compounds described herein with proapoptotic chemotherapeutic agents and/or with radiation therapy in order to encourage arrested cells to enter apoptosis.

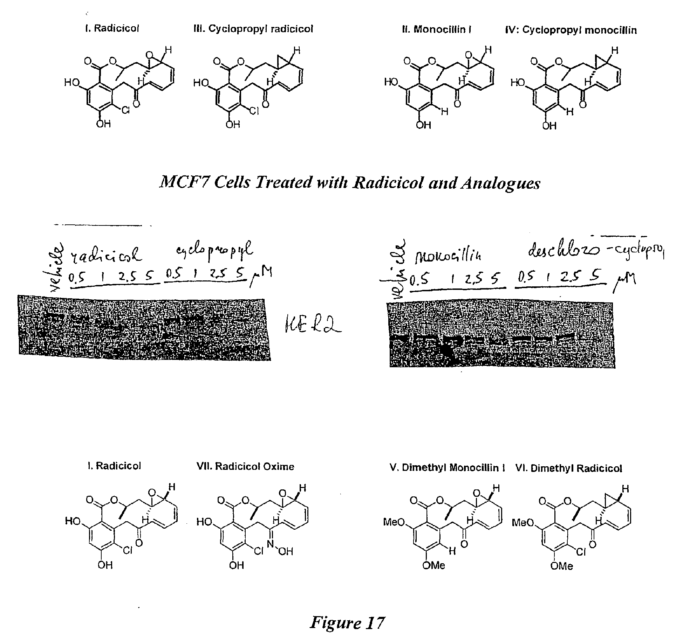

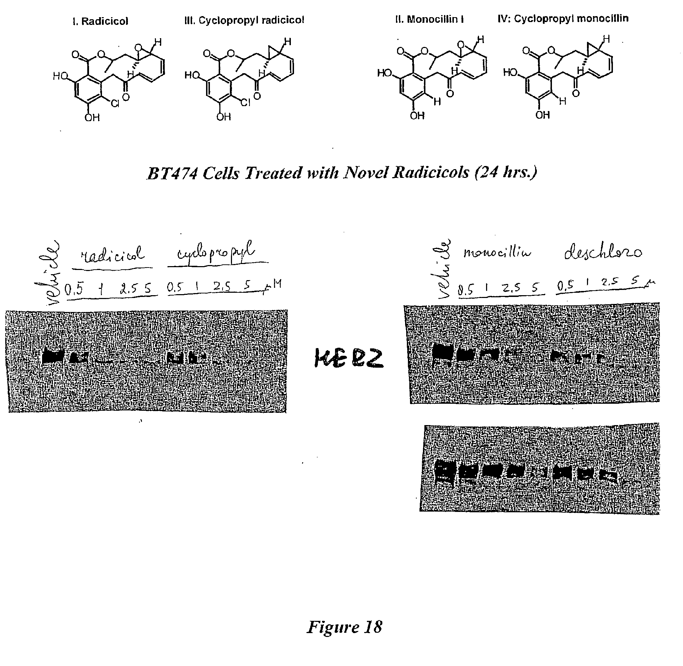

As demonstrated herein (see Figures 17-21), radicicol and certain of its analogues have been tested for their cytotoxicity in a panel of cancer cell lines, and importantly, for their ability to lead to the reduction of HER2. As depicted in Figure 17 and 18, four analogues have been tested for their ability to reduce HER2 in MCF7 and BT474 cells. Radicicol (**I**), monocillin I (**II**), cyclopropyl radicicol (**III**) and cyclopropyl monocillin I (**IV**) were each tested at similar concentrations (0.5 µM through 5 µM). These results demonstrate two previously unknown properties with respect to structure-activity relationships: the aromatic chloride contributes to efficacy, and the oxygen of the epoxide does not, despite its binding implication in the known crystal structure. Radicicol is approximately 10x superior in effecting destruction of Her-2 than monocillin I, which only lacks the aromatic chloride. In addition, the cyclopropane analog (**III**) is at least as effective, if not better than radicicol merely by replacing the oxygen of the epoxide with a CH₂ group. Significantly, currently, the cyclopropane analog can only be made by the route as detailed herein. Furthermore, while the vinyl epoxide, a reactive moiety, may be responsible for undesired cytotoxicity, the cyclopropane is far more stabile to non-productive cellular nucleophiles.

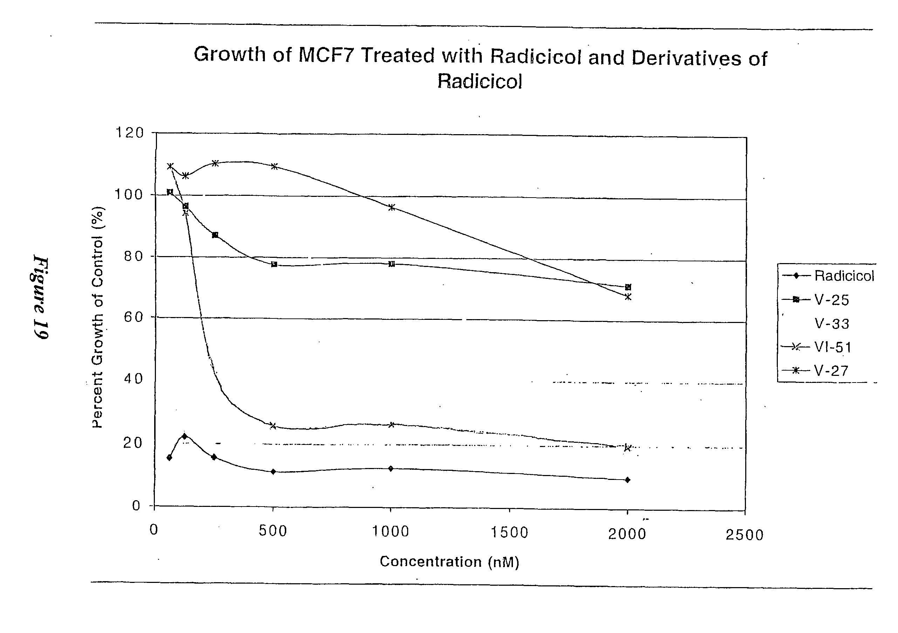

Additionally, the dimethyl ethers of both radicicol and monocillin (**V** and **VI** respectively) have been tested, as well as the oxime disclosed by Kyowa Hakko (**VII**) in a side by side experiment with radicicol. Radicicol again demonstrated efficacy, as did the oxime analog **VII** which has been reported to also be active *in vivo.* As shown in Figure 19, MCF7 cells (HER2 overexpressed, Rb positive) were treated with radicicol (V-27), radicicol oxime (VI-51), dimethyl monocillin (V-25) and dimethyl radicicol (V-33) and the growth of the MCF7 cells was monitored.

As discussed above, the action of geldanamycin and radicicol on Hsp90 in Rb (retinoblastoma) positive cells results in a clear G₁ (growth) phase block of the cell cycle, degradation of HER2, and eventual reversion, apoptosis or necrosis. Interestingly, and without a clear explanation, when radicicol is applied to Rb negative cells, the cells are blocked in the M (mitosis) phase. While geldanamycin and its derivatives are demonstrating success in Rb positive cell lines, they are much less effective in their ability to halt growth in the corresponding Rb negative cells. Radicicol and derivatives appear to have an advantage in these Rb negative cell lines as they demonstrate superior efficacy blocking these cells in mitosis.

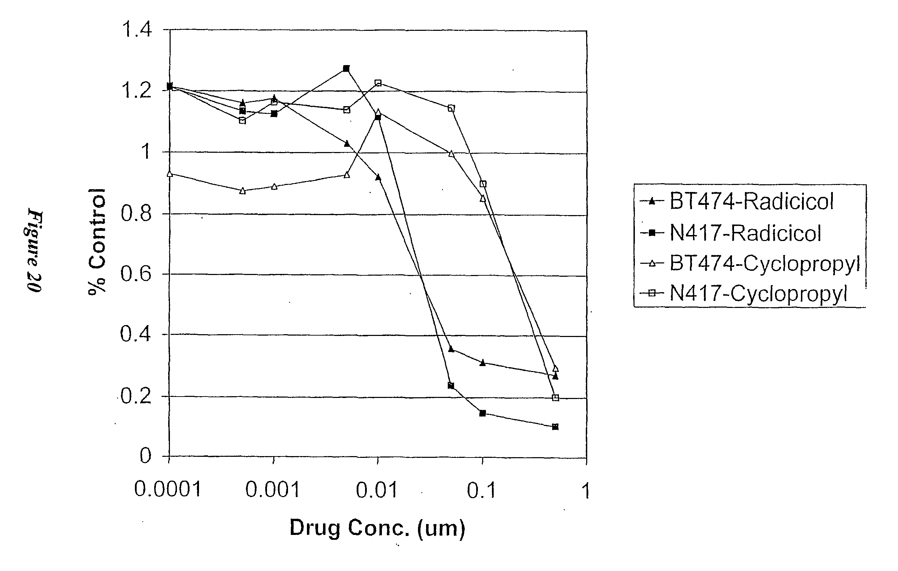

In addition, it has also been shown that 17-allylaminogeldanamycin (17-AGG), an ansamycin derivative, is much more potent against cells with wild type Rb than in cells with defective Rb function. It has also been shown that radicicol and cyclopropyl radicicol are potent inhibitors of both a breast cancer cell with wild type Rb and a small cell lung cancer cell line with defective Rb function (See Figure 20). Significantly, they have been found to be much more potent than 17-AGG in the latter cell type. These data suggest that radicicol derivatives are useful in the treatment of the 15-20% of human tumors with mutated Rb gene and especially in small lung cell cancer, in which the gene is almost always mutated.

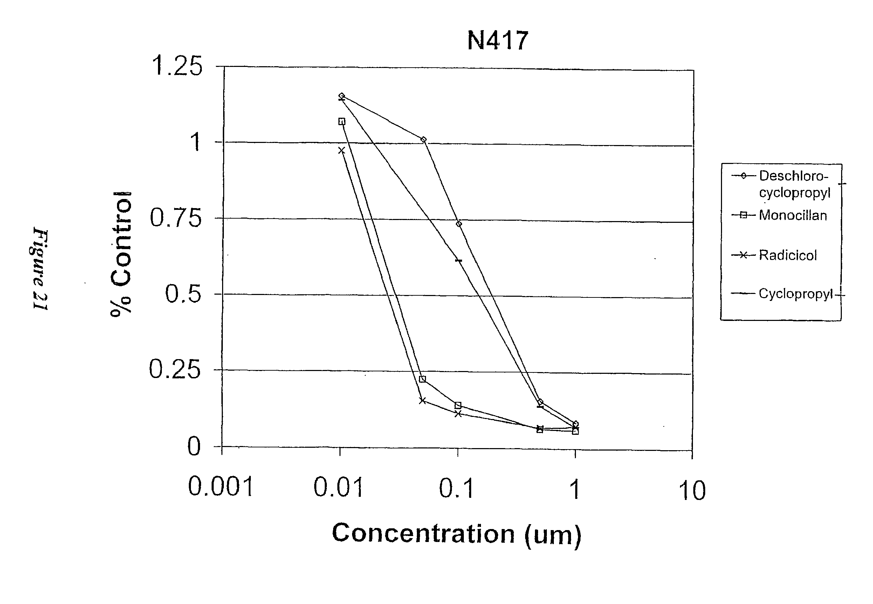

As discussed herein, the compounds of the present invention have been shown to reduce HER2 in MCF-7 and BT474 cells, and are cytotoxic against a panel of cancer cell lines (see exemplification herein and Figures 19, 20 and 21), in particular against Rb negative cancer cell lines (Figures 20 and 21). The inventive compounds are thus useful for the inhibition of the growth of or for killing cancer cells and are useful in the treatment of cancer (or more generally useful in the treatment of proliferative disorders). In addition, compounds as described herein have been found to act as potent inhibitors of cancer cell lines comprising Rb negative cells, and thus are useful in the treatment of cancers comprising Rb negative cells. Currently, there is no effective treatment for many of these cancers comprising Rb negative cells. The method of the invention comprises administering to a subject in need thereof a therapeutically effective amount of a compound of the invention.

In certain embodiments of the present invention a "therapeutically effective amount" of the inventive compound or pharmaceutical composition is that amount effective for detectable killing or inhibiting the growth of cancer cells, and in certain embodiments of special interest an amount for detectable killing or inhibiting the growth of cancer cells comprising Rb negative cancer cells.

The compounds and compositions, according to the method of the present invention, may be administered using any amount and any route of administration effective for killing or inhibiting the growth of tumor cells. Thus, the expression "effective amount" as used herein, refers to a sufficient amount of agent to kill or inhibit the growth of tumor cells. The exact amount required will vary from subject to subject, depending on the species, age, and general condition of the subject, the severity of the infection, the particular anticancer agent, its mode of administration, and the like. The anticancer compounds of the invention are preferably formulated in dosage unit form for ease of administration and uniformity of dosage. The expression "dosage unit form" as used herein refers to a physically discrete unit of anticancer agent appropriate for the patient to be treated. It will be understood, however, that the total daily usage of the compounds and compositions of the present invention will be decided by the attending physician within the scope of sound medical judgment. The specific therapeutically effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

Furthermore, after formulation with an appropriate pharmaceutically acceptable carrier in a desired dosage, the pharmaceutical compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds of the invention may be administered at dosage levels of about 0.001 mg/kg to about 50 mg/kg , from about 0.01 mg/kg to about 25 mg/kg, or from about 0.1 mg/kg to about 10 mg/kg of subject body weight per day, one or more times a day, to obtain the desired therapeutic effect. I will also be appreciated that dosages smaller than 0.001 mg/kg or greater than 50 mg/kg (for example 50-100 mg/kg) can be administered to a subject. In certain embodiments, compounds are administered orally or parenterally.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compomd in the proper medium. Absorption enhancers can also be used to increase the flux of the compound across the skin. The rate can be controlled by either providing a rate controlling membrane or by dispersing the compound in a polymer matrix or gel.

As discussed above, in one aspect, the compounds of the present invention are useful as anticancer agents, and thus may be useful in the treatment of cancer, by effecting tumor cell death or inhibiting the growth of tumor cells. In general, the inventive anticancer agents are useful in the treatment of cancers and other proliferative disorders, including, but not limited to glioblastoma, retinoblastoms, breast cancer, cervical cancer, colon and rectal cancer, leukemia, lung cancer (including, but not limited to small cell lung cancer), melanoma, multiple myeloma, non-Hodgkin's lymphoma, ovarian cancer, pancreatic cancer, prostate cancer, and gastric cancer, to name a few. In certain embodiments, the inventive anticancer agents are active against cancers comprising Rb negative cells, including, but not limited to small cell lung cancer, retinoblastoma and glioblastoma. In certain other embodiments, the inventive anticancer agents are active against breast cancer cells, leukemia cells and melanoma cells, and thus are useful for the treatment of breast cancer, leukemias (e.g., myeloid, lymphocytic, myelocytic and lymphoblastic leukemias) and malignant melanomas. In still other embodiments, the inventive anticancer agents are active against solid tumors and also kill and/or inhibit the growth of multidrug resistant cells (MDR cells).

It will also be appreciated that the compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another anticancer agent), or they may achieve different effects (e.g., control of any adverse effects).

For example, other therapies or anticancer agents that may be used in combination with the inventive anticancer agents of the present invention include surgery, radiotherapy (in but a few examples, γ-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), to name a few. Additionally, the present invention also encompasses the use of certain cytotoxic or anticancer agents currently in clinical trials and which may ultimately be approved by the FDA (including, but not limited to, epothilones and analogues thereof and geldanamycins and analogues thereof). For a more comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999, the entire contents of which are hereby incorporated by reference.

### TREATMENT KITS

In other embodiments, the present invention relates to a kit for conveniently and effectively carrying out the methods in accordance with the present invention. In general, the pharmaceutical pack or kit comprises one or more containers filled with one or more of the ingredients of the pharmaceutical compositions of the invention. Such kits are especially suited for the delivery of solid oral forms such as tablets or capsules. Such a kit preferably includes a number of unit dosages, and may also include a card having the dosages oriented in the order of their intended use. If desired, a memory aid can be provided, for example in the form of numbers, letters, or other markings or with a calendar insert, designating the days in the treatment schedule in which the dosages can be administered. Alternatively, placebo dosages, or calcium dietary supplements, either in a form similar to or distinct from the substituted purine dosages, can be included to provide a kit in which a dosage is taken every day. Optionally associated with such container(s) can be a notice in the form prescribed by a governmental agency regulating the manufacture, use or sale of pharmaceutical products, which notice reflects approval by the agency of manufacture, use or sale for human administration.

### EQUIVALENTS

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### EXEMPLIFICATION

**Methyl (3S)-3-(*tert*-butyldiphenylsilyloxy)-butyrate (9a).** A solution of methyl (*R*)-3-hydroxy-butyrate (**9**, 1.9 g, 16.0 mmol) in 20 mL of anhydrous CH₂Cl₂ at 0 °C was treated with imidazole (2.2 g, 32.0 mmol) in one portion. After 10 min, *tert*-butyldiphenyl-chlorosilane was added dropwise and the resulting exothermic reaction was allowed to warm to 25 °C and stirred 4 h. The reaction mixture was diluted with Et₂O (200 mL), and washed successively with 5% aqueous NH₄Cl (100 mL) and saturated aqueous NaCl (100 mL). The organic layer was dried (MgSO₄), concentrated under reduced pressure, and purified by flash chromatography (SiO₂, 6 × 12 cm, 0-5% EtOAc/hexanes gradient) to provide **9a** (5.6 g, 97%) as a colorless oil: ¹H NMR (CDCl₃, 400 MHz) δ 7.69 (m, 4H), 7.41 (m, 6H), 4.31 (m, 1H), 3.60 (s, 3H), 2.57 (dd, *J* = 14.6, 7.1 Hz, 1H), 2.40 (dd, *J* = 14.6, 5.7 Hz, 1H), 1.12 (d, *J* = 6.1 Hz, 3H), 1.03 (s, 9H); ¹³C NMR (CDCl₃, 100 MHz) δ 171.8, 135.8, 135.8, 134.2, 133.8, 129.6, 129.5, 127.5, 127.5, 66.8, 51.4, 44.4, 26.8, 23.6, 19.1; IR (film) *v*ₘₐₓ 2953, 2858, 1742, 1428, 1378, 1303 1194, 1111 cm⁻¹; ESIMS *m*/*z* 379 ([M + Na⁺], C₂₁H₂₈NaO₃Si requires 379). (-)-(3*R*)-**9a:** [α]²⁵_{D} -5.1 (*c* 0.67, CH₂Cl₂).

**(3*S*)-3-(*tert*-Butyldiphenylsilyloxy)-butan-1-al (10).** A solution of **9a** (5.6 g, 15.7 mmol) in 100 mL of anhydrous toluene at -78 °C was treated dropwise with DIBAL-H (17.3 mL, 17.3 mmol). After 10 min, the reaction was successively treated with MeOH (1.0 mL) and saturated aqueous NH₄Cl (10.0 mL). The resulting mixture was stirred 1 h at 25 °C followed by addition of Et₂O (200 mL) and after 1 h further stirring, addition of MgSO₄ (3 g). The resulting suspension was filtered through celite and concentrated under reduced pressure to provide **10** (5.0 g, 92%) which was carried on crude as a colorless oil: ¹H NMR (CDCl₃, 400 MHz) δ 9.77 (t, *J*= 2.5 Hz, 1H), 7.69 (m, 4H), 7.44 (m, 6H), 4.34 (m, 1H), 2.50 (m, 2H), 1.18 (d, *J*= 6.2 Hz, 3H), 1.04 (s, 9H); ¹³C NMR (CDCl₃, 100 MHz) δ 202.0, 135.8, 135.8, 134.0, 133.5, 129.8, 129.7, 127.7, 127.5, 65.6, 52.7, 26.8, 23.8, 19.1; IR (film) *v*ₘₐₓ 2962, 2858, 1728, 1427, 1111, 1024 cm⁻¹; ESIMS *m*/*z* 349 ([M + Na⁺], C₂₀H₂₆NaO₂Si requires 349).

**Ethyl (5*S*)-5-(*tert*-butyldiphenylsilyloxy)-hex-2-enoate (11).** To a stirred suspension of LiCl (0.78 g, 18.4 mmol), triethylphosphonoacetate (3.65 mL, 18.4 mmol) and diisopropylethylamine (2.67 mL, 15.3 mmol) in anhydrous CH₃CN (80 mL) at 25 °C was added a solution of **10** (5.0 g, 15.3 mmol) in anhydrous CH₃CN. After 12 h, the reaction mixture was diluted with Et₂O (200 mL), and washed successively with H₂O (200 mL) and saturated aqueous NaCl (200 mL). The organic layer was dried (MgSO₄), and concentrated under reduced pressure. Flash chromatography (SiO₂, 6 × 12 cm, 4% EtOAc/hexanes) provided **11** (5.8 g, 95%) as a colorless oil: δ 7.70 (m, 4H), 7.43 (m, 6H), 6.94 (dt, *J* = 15.3, 7.5 Hz, 1H), 5.78 (d, *J* = 15.3 Hz, 1H), 4.19 (q, *J* = 7.0 Hz, 2H), 3.98 (m, 1H), 2.33 (m, 2H), 1.30 (t, *J* = 7.0 Hz, 3H), 1.11 (d, *J* = 7.5 Hz, 3H), 1.06 (s, 9H); ¹³C NMR (CDCl₃, 100 MHz) δ 166.4, 145.5, 135.8, 135.8, 134.3, 133.9, 129.6, 129.6, 127.6, 127.5, 123.4, 68.5, 60.1, 42.1, 26.9, 23.2, 19.2, 14.2; IR (film) *v*ₘₐₓ 2964, 1722, 1654, 1427, 1265, 1176, 1111 cm⁻¹; ESIMS *m*/*z* 419 ([M + Na⁺], C₂₄H₃₂NaO₃Si requires 419). (+)-(5*R*)-**11**: [α]²⁵_{D} +31 (*c* 0.71, CH₂Cl₂).

**(5*S*)-5-(*tert*-Butyldiphenylsilyloxy)-hex-2-en-1-ol (12)**. A solution of **11** (5.6 g, 14.1 mmol) in 100 mL of anhydrous CH₂Cl₂ at -20 °C was treated dropwise with DIBAL-H (30.0 mL, 30.0 mmol). After 2 h, the reaction was warmed to 0 °C for 1 h and then to 25 °C for 2 h. The reaction was then treated with sat. aqueous NH₄Cl (10.0 mL) and stirred 1 h at 25 °C. The resulting mixture was diluted with Et₂O (200 mL) and stirred 1 h at 25 °C followed by addition of MgSO₄ (3 g). The suspension was filtered through celite and concentrated under reduced pressure. Flash chromatography (SiO₂, 6 × 12 cm, 0-30% EtOAc/hexanes) provided 12 (4.8 g, 96%) as a colorless oil: ¹H,NMR (CDCl₃, 500 MHz) δ 7.69 (m, 4H), 7.42 (m, 6H), 5.57 (m, 2H), 4.01 (d, *J* = 4.1 Hz, 2H), 3.90 (m, 1H), 2.18 (m, 2H), 1.09 (d, *J* = 6.0 Hz, 3H), 1.06 (s, 9H); ¹³C NMR (CDCl₃, 125 MHz) δ135.8, 135.8, 134.7, 134.6, 131.3, 129.4, 129.3, 127.5, 127.4, 69.2, 63.6, 42.2, 26.9, 23.2, 19.2; IR (film) *v* 3333, 2857, 1428, 1111, 997 cm⁻¹; ESIMS *m*/*z* 377 ([M +Nₐ⁺], C₂₂H₃₀NaO₂Si requires 377). (+)-(5*R*)-**12**: [α]²⁵_{D} +20 (*c* 0.71, CH₂Cl₂).

***(2R, 3R,* 5*S*)-5-(*tert*-Butyldiphenylsilyl)-2,3-(oxiranyl)-hexan-1-ol (13).** To a suspension of flame dried 5Å molecular sieves in 20 mL of anhydrous CH₂Cl₂ at -30 °C was added in sequential fashion: (D)-(-)-diethyl tartrate (0.120 µL, 0.67 mmol), Ti(O*i*Pr)₄ (0.170 µL, 0.56 mmol) and *tert*-butylhydroperoxide (2.5 mL, 3.4 M solution in toluene, 8.5 mmol). The reaction mixture was stirred at -30 °C for 30 min before the addition of 12 (2.0 g, 5.6 mmol) in anhydrous CH₂Cl₂ (3.0 mL). The reaction was then stored in a -30 °C freezer for 12 h without stirring. The reaction was then warmed to -20 °C and quenched by the addition of 10% NaOH/saturated aqueous NaCl (2.0 mL). Upon further warming to -10 °C, the reaction was diluted with Et₂O (50 mL), treated with MgSO₄ (2.0 g) and celite (500 mg) and stirred an addition 15 min. The reaction was allowed to settle for 1 h before filtration through celite using Et₂O. Concentration under reduced pressure, followed by flash chromatography (SiO₂, 6 × 8 cm, 30% EtOAc/hexanes) afforded **13** (1.9 g, 90%) as a colorless oil: ¹H NMR (CDCl₃, 400 MHz) δ 7.69 (m, 4H), 7.40 (m, 6H), 4.09 (m, 1H), 3.84 (ddd, *J* = 12.6, 5.7, 2.6 Hz, 1H), 3.55 (ddd, *J* = 12.6, 7.2, 4.5 Hz, 1H), 3.03 (dt, *J* =5.9, 2.3 Hz, 1H), 2.82 (m, 1H), 1.75 (m, 2H), 1.63 (dt, *J* = 13.9, 5.8 Hz, 1H), 1.14 (d, *J* = 6.2 Hz, 3H), 1.06 (s, 9H); ¹³C NMR (CDCl₃, 100 MHz) δ 135.8, 135.8, 134.4, 133.9, 129.6, 129.5, 127.6, 127.5, 67.7, 61.5, 58.6, 53.2, 41.6, 26.9, 23.8, 19.2; IR (film) 3426, 2930, 2856, 1472, 1427, 1378, 1361, 1111 cm⁻¹; ESIMS *m*/*z* 393 ([M + Na⁺], C₂₂H₃₀NaO₃Si requires 393). (+)-(2*R*, 3*R*, 5*S*)-**13**: [α]²⁵_{D} +14 (*c* 1.1, CH₂Cl₂).

**(2*R*, 3*R*, 5*S*)-5-(*tert*-Butyldiphenylsilyl)-2,3-(oxiranyl)-hexan-1-al (13a).** A solution of 13 (1.8 g, 4.9 mmol) and Et₃N (3.4 mL, 24 mmol) in of 4:1 CH₂Cl₂/DMSO (50 mL) at 0 °C was treated with SO₃**•**pyridine (2.9 g, 17 mmol) and stirred 30 min at 25 °C. The reaction was diluted with EtOAc (200 mL), washed sequentially with H₂O (3 x 50 mL), saturated aqueous NaHCO₃ (50 mL), and saturated aqueous NaCl (50 mL) and dried (MgSO₄). Evaporation of the solvents under reduced pressure directly provided pure **13a** (1.6 g, 90%) as a colorless oil: ¹H NMR (CDCl₃, 500 MHz) δ 8.93 (d, *J* = 6.3 Hz, 1H), 7.72 (m, 4H), 7.45 (m, 6H), 4.13 (m, 1H), 3.32 (dt, *J* = 6.6, 1.9 Hz, 1H), 3.06 (dd, *J* = 6.4, 1.9 Hz, 1H), 1 .86 (m, 1H), 1.68 (m, 1H), 1.17 (d; *J* = 6.1 Hz, 3H), 1.11 (s, 9H); ¹³C NMR (CDCl₃, 100 MHz) δ198.1, 135.7, 135.7, 134.0, 133.7, 129.7, 129.6, 127.6, 127.5, 67.3, 59.2, 54.0, 41.0, 26.9, 23.7, 19.1; IR (film) 3070, 2892, 2857, 1729, 1427, 1111 cm⁻¹; ESIMS *m*/*z* 391 ([M + Na⁺], C₂₂H₂₈NaO₃Si requires 391). (-)-(2*R*, 3*R*, 5*S*)-**13a**: [α]²⁵_{D} -42 (*c* 0.93, CH₂Cl₂).

**(3*R*, 4*R*, 6*S*)-6-(*tert*-Butyldiphenylsilyl)-3,4-(oxiranyl)-hept-1-ene (14).** Methyltriphenylphosphonium bromide (1.93 g, 5.42 mmol) and a stir bar were added to a flask and thoroughly flame dried. Anhydrous THF (30 mL) was added via cannula under Ar and the resulting suspension was cooled to 0 °C prior to the addition of NaHMDS (5.13 mL, 5.13 mmol, 1.0 M in THF) in dropwise fashion. The resulting gold suspension was warmed to 25 °C for 30 min and then recooled to -10 °C prior to the addition of **13a** (1.05 g, 2.85 mmol) in anhydrous THF (5.0 mL). The reaction was complete within 10 min, and was quenched by the addition of saturated aqueous NH₄Cl (50 mL). The mixture was then extracted with Et₂O (100 mL), washed sequentially with H₂O (50 mL) and saturated aqueous NaCl (50 mL) and dried (MgSO₄). Removal of the solvents under reduced pressure followed by flash chromatography (SiO₂, 6 × 6 cm, 5% EtOAc/hexanes) provided **14** (0.85 g, 82%) as a colorless oil: ¹H NMR (CDCl₃, 400 MHz) δ 7.68 (m, 4H), 7.42 (m, 6H), 5.53 (ddd, *J*= 17.3, 10.1, 7.5 Hz, 1H), 5.41 (dd, *J*= 17.3, 1.5 Hz, 1H), 5.25 (dd, *J* = 10.1, 1.5 Hz, 1H), 4.07 (m, 1H), 2.99 (dd, *J* = 7.4, 2.0 Hz, 1H), 2.91 (dt, *J* = 5.6, 2.0 Hz, 1H), 1.70 (m, 1H), 1.10 (d, *J*= 6.1 Hz, 3H), 1.06 (s, 9H); ¹³C NMR (CDCl₃, 100 MHz) δ135.8, 135.8, 134.4, 134.0, 129.6, 129.5, 127.6, 127.5, 119.1, 67.6, 59.0, 57.7, 42.0, 26.9, 23.8, 19.2; IR (film) 2963, 2856, 1472, 1377, 1111 cm⁻¹ ; ESIMS *m*/*z* 389 ([M + Na⁺], C₂₃H₃₀ NaO₂Si requires 389). (+)-(3*R*, 4*R*, 6*S*)-**14**: [α]²⁵_{D} -4.3 (*c* 1.0, CH₂Cl₂).

**(3*R*, 4*R*, 6*S*)-6-(Hydroxy)-3,4-(oxiranyl)-hept-1-ene (8).** To a solution of 14 (0.78 g, 2.13 mmol) in 20 mL of anhydrous THF at 25 °C was treated with *n*Bu₄NF (2.55 mL, 1.0 M solution in THF, 2.55 mmol) and stirred 4 h. The reaction was concentrated under reduced pressure. Flash chromatography (SiO₂, 3 × 8 cm, 0-50% EtOAc/hexanes gradient) provided **8** (0.243 g, 89%) as a colorless and volatile oil: ¹H NMR (CDCl₃, 400 MHz) δ 5.57 (m, 1H), 5.47 (dd, *J* = 17.1, 1.3 Hz, 1H), 5.28 (dd, *J* = 10.0, 1.3 Hz), 4.01 (m, 1H), 3.22 (dd, *J* = 7.3, 2.1 Hz, 1H), 3.04 (m, 1H), 2.10 (br s, 1H), 1.87 (m, 1H), 1.63 (m, 1H), 1.23 (d, *J* = 6.3 Hz, 3H); ¹³C NMR (CDCl₃, 100 MHz) δ135.3, 119.5, 65.4, 58.3, 58.0, 40.1, 23.6; IR (film) 3422, 2968, 2931, 1456, 1407, 1375, 1319 cm⁻¹; ESIMS *m*/*z* 151 ([M + Na⁺], C₇H₁₂NaO₂ requires 151). (+)-(3*R*, 4*R*, 6*S*)-**8**: [α]²⁵_{D} +55 (*c* 1.2, CH₂Cl₂).

**(*E*,*E*)-2-(1,3-Pentadien-1-yl)-1,3-dithiane (6).** To a stirred solution of 1,3 propanedithiol (5.0 mL, 50.0 mmol), MgClO₄ (0.6 g, 2.5 mmol) and H₂SO₄ (20 µL) in anhydrous CHCl₃ (80 mL) at -10 °C was added hexadienal (**15,** 5.5 mL, 50.0 mmol) in anhydrous CHCl₃ (20 mL) in dropwise fashion via cannula. The reaction stirred at 25 °C for 2 h before being poured into cold 10% KOH (100 mL) followed by stirring for 15 min. The organic layer was separated, washed sequentially with 10% KOH (50 mL), H₂O (50 mL), dried (MgSO₄) and filtered through celite. Concentration under reduced pressure was followed by flash chromatography (SiO₂, 6 × 12 cm, 4% EtOAc/hexanes) to provide 6 (6.4 g, 67%) as a slightly yellow oil (9:1 *E*:*Z*): ¹H NMR (CDCl₃, 400 MHz) δ6.33 (dd, *J*= 15.1, 10.4 Hz, 1H), 6.01 (ddd, *J*= 15.1, 10.4, 1.3 Hz, 1H) 5.74 (dq, *J* = 15.1, 6.6 Hz, 1H), 5.59 (dd, *J* = 15.1, 7.8 Hz, 1H), 4.64 (d, *J* = 7.8 Hz, 1H), 2.85 (m, 4H), 2.07 (m, 1H), 1.83 (m, 1H), 1.74 (d, *J* = 6.6 Hz, 3H), 1.06 (s, 9H); ¹³C NMR (CDCl₃, 100 MHz) δ 134.2, 131.9, 130.7, 126.9, 47.9, 30.7, 25.6, 18.6; IR (film) *v*ₘₐₓ 3018, 2899, 1421, 1274, 986 cm⁻¹; ESIMS *m*/*z* 209 ([M + Na⁺], C₉H₁₄NaS₂ requires 209). Z isomer: ¹H NMR δ 6.67 (dd, *J*= 15.1, 11.0 Hz, 1H), 4.70 (d, 7.8 Hz, 1H).

**2-(Chloromethyl)-4,6-dimethoxy-benzaldehyde (19).** POCl₃ (11.1 mL, 119.0 mmol) was dropped slowly via cannula into anhydrous DMF (17.0 mL) at 0 °C, and the resulting solution was stirred at 25 °C for 20 min. A solution of 3,5-dimethoxy-benzylalcohol (5.0 g, 29.0 mmol) in anhydrous DMF (3.0 mL) was added slowly and the reaction was warmed to 75 °C for 2 h. The reaction was then allowed to cool to 25 °C and poured into ice water (250 mL). The mixture was neutralized with 2N NaOH to pH = 7 and stirred 1.5 h at 25°C. The resulting precipitate was filtered, washed thoroughly with H₂O (5 x 50 mL) and dried under vacuum to give **19** (6.0 g, 93%): ¹H NMR (CDCl₃, 400 MHz) δ10.46 (s, 1H), 6.75 (d, *J*= 2.1 Hz, 1H), 6.44 (d, *J* = 2.1 Hz, 1H), 5.10, (s, 2H), 3.91 (s, 3H), 3.91 (s, 3H); ¹³C NMR (CDCl₃, 100 MHz) δ 189.9, 165.2, 165.0, 142.3, 115.9, 107.5, 97.6, 56.0, 55.6, 44.8; IR (film) νₘₐₓ 2980, 2884, 1670, 1597, 1327, 1204, 1150 cm⁻¹; ESIMS *m*/*z* 237 ([M + Na⁺], C₁₀H₁₁NaO₃Cl requires 237).

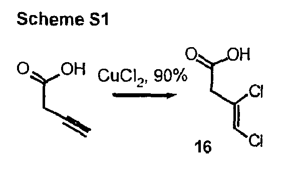

**3,4-Dichlorobut-3-enoic acid (16).** (Uemura, S. *et al. J. Chem. Soc. Perkin I* **1977**, 676). 3-Butynoic acid (6.00 g, 71.0 mmol), CuCl₂ (144 g, 1.07 mol), and LiCl (45.0 g, 1.07 mol) were refluxed in CH₃CN (520 mL) for 12 h. The solvent was removed, and the resulting oil was filtered through celite with EtOAc (200 mL). The solution was then washed with H₂O (100 mL), sat. aqueous NaCl (100 mL) and dried (MgSO₄). Flash column chromatography (SiO₂, 6 × 10 cm, 10-20% Et₂O/hexanes) provided acid **16** (10.6 g, 96%) as a light green oil: ¹H NMR (CDCl₃, 400 MHz) δ 11.70 (br s, 1H), 6.41 (s, 1H), 3.76 (s, 2H); ¹³C NMR (CDCl₃, 100 MHz) δ 174.7, 127.2, 118.7, 39.3; IR (film) *v*ₘₐₓ 3090, 1716, 1652, 1558, 1540 cm⁻¹; ESIMS *m*/*z* 177 ([M + Na⁺], C₄H₃NaO₂Cl₂ requires 177).
Epimer at the secondary methyl center was also made and covered within:

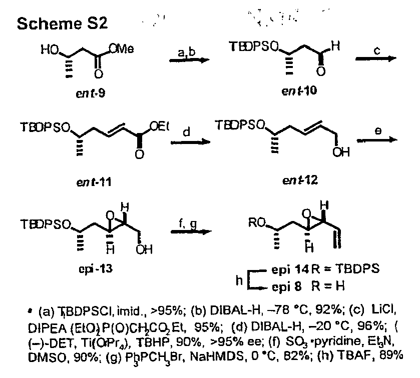

***(2R, 3R,* 5*R*)-5-(*tert*-Butyldiphenylsilyl)-2,3-(oxiranyl)-hexan-1-ol (epi-13).** Same as for **13:** ¹H NMR (CDCl₃, 400 MHz) δ 7.71 (m, 4H), 7.43 (m, 6H), 4.07 (m, 1H), 3.85 (ddd, *J* = 12.6, 5.7, 2.6 Hz, 1H), 3.55 (ddd, *J*= 12.6, 7.2, 4.5 Hz, 1H), 3.08 (dt, *J*=5.9, 2.3 Hz, 1H), 2.84 (m, 1H), 1.81 (m, 2H), 1.74 (dt, *J* = 13.9, 5.8 Hz, 1H), 1.17 (d, *J*= 6.2 Hz, 3H), 1.07 (s, 9H); ¹³C NMR (CDCl₃, 100 MHz) δ135.8, 135.8, 134.3, 134.0, 129.6, 129.6, 127.6, 127.5, 67.4, 61.6, 58.1, 52.9, 41.1, 26.9, 23.2, 19.1; IR (film) 3446, 2931, 2857, 1472, 1427, 1111 cm⁻¹; ESIMS *m*/*z* 393 ([M + Na⁺], C₂₂H₃₀NaO₃Si requires 393). (+)-(2*R*, 3*R*, 5*R*)-**epi-13**: [α]²⁵_{D} +23 (*c* 0.8, CH₂Cl₂).

***(2R,* 3*R*, 5*R*)-5-(*tert*-Butyldiphenylsilyl)-2,3-(oxiranyl)-hexan-1-al (epi-13a).** Same as for **13a:** ¹H NMR (CDCl₃, 500 MHz) δ 8.96 (d, *J* = 6.3 Hz, 1H), 7.68 (m, 4H), 7.45 (m, 6H), 4.09 (m, 1H), 3.39 (dt, *J*= 6.6, 1.9 Hz, 1H), 3.06 (dd, *J*= 6.3, 1.9 Hz, 1H), 1.75 (m, 2H), 1.19 (d, *J*= 6.2 Hz, 3H), 1.08 (s, 9H); ¹³C NMR (CDCl₃, 500 MHz) δ198.2, 135.8, 135.7, 134.0, 133.7, 129.8, 129.7, 127.7, 127.6, 67.2, 58.6, 53.9, 40.6, 26.9, 23.2, 19.1; IR (film) 2931, 1733, 1472, 1111 cm⁻¹; ESIMS *mlz* 391 ([M + Na⁺], C₂₂H₂₈NaO₃Si requires 391). (-)-(2*R*, 3*R*, 5*R*)-**epi-13a:** [α]²⁵D -15 (*c* 0.54, CH₂Cl₂).

**(3*R*, 4*R*, 6*R*)-6-(*tert*-Butyldiphenylsilyl)-3,4-(oxiranyl)-hept-1-ene (epi-14).** Same as for **14:** ¹H NMR (CDCl₃, 400 MHz) δ 7.69 (m, 4H), 7.45 (m, 6H), 5.58 (ddd, *J* = 17.3, 9.9, 7.4 Hz, 1H), 5.45 (dd, *J*= 17.3, 1.6 Hz, 1H), 5.28 (dd, *J*= 9.9, 1.6 Hz, 1H), 4.09 (m, 1H), 3.06 (dd, *J* = 7.4, 2.1 Hz, 1H), 3.00 (dt, *J* = 5.8, 2.1 Hz, 1H), 1.82 (dt, *J* = 13.9, 5.8 Hz, 1H), 1.66 (dt, 3.9, 5.8 Hz, 1H), 1.18 (d, *J*= 6.2 Hz, 3H), 1.08 (s, 9H); ¹³C NMR (CDCl₃, 100 MHz) δ135.8, 135.8, 134.4, 134.0, 129.6, 129.5, 127.6, 127.5, 119.1, 67.5, 58.5, 57.3, 41.5, 26.9, 23.2, 19.2; IR (film) 2963, 2857, 1472, 1379, 1111 cm⁻¹ ; ESIMS *m*/*z* 389 ([M + Na⁺], C₂₃H₃₀NaO₂Si requires 389). (+)-(3*R*, 4*R*, 6*R*)-**epi-14**: [α]²⁵D +15 (*c* 0.67, CH₂Cl₂).

***(3R, 4R,* 6*R*)-6-(Hydroxy)-3,4-(oxiranyl)-hept-1-ene (epi-8).** Same as for 8: ¹H NMR (CDCl₃, 400 MHz) δ 5.36 (ddd, *J* = 17.0, 7.2, 3.0 Hz, 1H), 5.26 (dd, *J*= 17.0, 1.8 Hz, 1H), 5.07 (dd, *J* = 9.8, 1.8 Hz, 1H), 3.87 (m, 1H), 2.92 (dd, *J* = 7.2, 2.2 Hz, 1H), 2.77 (m, 1H), 2.0 (br s, 1H), 1.64 (dt, *J* = 14.2, 4.4 Hz, 1H), 1.39 (dt, *J* = 14.2, 7.6 Hz, 1H), 1.08 (d, *J* = 6.2 Hz, 3H); ¹³C NMR (CDCl₃, 100 MHz) δ135.2, 119.6, 66.4, 58.3, 58.2, 40.8, 23.4; IR (film) 3403, 2966, 1428, 1113 cm⁻¹; ESIMS *m*/*z* 151 (M + Na⁺, C₇H₁₂NaO₂ requires 151). (+)-(3*R*, 4*R*, 6*R*)-**epi-8**: [a]²⁵_{D} +18 (*c* 0.38, CH₂Cl₂).

**Ester (17):** A solution of alcohol **8** (100 mg, 0.78 mmol) and triphenylphosphine (204 mg, 0.78 mmol) in anhydrous THF (4.0 mL) was cooled to 0 °C, treated with DIAD (diisopropylazodicarboxylate) (157 µL, 0.78 mmol) and stirred at 0 °C for 10 min. Acid 16 (0.121 g, 0.78 mmol) was then added in anhydrous THF (1.0 mL) and stirred 1 h at 25 °C. The reaction mixture was concentrated *in vacuo* and purified by chromatography on silica gel (SiO₂, 2 × 8 cm, 5% EtOAc/hexanes) to afford **17** as a colorless oil (0.152 mg, 73%):¹H NMR (CDCl₃, 400 MHz) δ 6.36 (s, 1H), 5.56 (ddd, *J*= 17.2, 10.0, 7.4 Hz, 1H), 5.47 (dd, *J*= 17.2, 1.8 Hz, 1H), 5.29 (dd, *J* = 10.0, 1.8 Hz, 1H), 5.17 (m, 1H), 3.57 (s, 2H), 3.10 (dd, *J* = 7.1, 2.0 Hz, 1H), 2.92 (dt, *J*= 5.7, 2.0 Hz, 1H), 1.85 (m, 2H), 1.34 (d, *J*= 6.3 Hz, 3H); ¹³C NMR (CDCl₃, 125 MHz) δ 167.2, 135.5, 128.3, 119.8, 118.0, 70.3, 58.3, 57.0, 40.0, 38.2, 20.0; IR (film) *v*ₘₐₓ 3087, 2984, 1739, 1331, 1257, 1180 cm⁻¹; ESIMS *m*/*z* 287 ([M+Na⁺], C₁₁H₁₄NaO₃Cl₂ requires 287). (+)-(*R*, *R*, *R*)-**17**: [α]²⁵_{D} +14 (*c* 1.7, CH₂Cl₂).

**Allene (5):** A solution of **17** (150 mg, 0.56 mmol) in anhydrous CH₃CN (5.6 mL) at 0 °C was treated with distilled *i*Pr₂NEt (1.0 mL, 5.6 mmol) and stirred at 25 °C for 1 h. The reaction was diluted with Et₂O (20 mL), washed sequentially with 0.1 N HCl (10 mL), H₂O (10 mL), and saturated aqueous NaCl (10 mL), and dried (Mg₂SO₄). Evaporation of the solvents under reduced pressure followed by flash chromatography (SiO₂, 2 × 6 cm, 0-7% EtOAc/hexanes gradient) afforded the allene 5 (77 mg, 60%) as a light yellow oil: ¹H NMR (CDCl₃, 400 MHz) δ 6.47 (dd, *J*= 5.8, 2.4 Hz, 1H), 5.95 (d, *J*= 5.8 Hz, 1H), 5.57 (ddd, *J*= 17.2, 10.0, 7.3 Hz, 1H), 5.50 (d, *J*= 17.2 Hz, 1H), 5.39 (d, *J* = 10.0 Hz, 1H), 5.17 (m, 1H), 3.11 (dt, *J* = 3.8, 1.9 Hz, 1H), 2.92 (m, 1H), 1.88 (m, 2H), 1.37 (d, *J* = 6.4 Hz, 3H); ¹³C NMR (CDCl₃, 125 MHz) δ 211.7, 163.0, 135.5, 119.8, 96.2, 93.1, 70.4, 58.3, 57.0, 38.3, 20.1; IR (film) *v*ₘₐₓ 3053, 2983, 1970, 1716, 1382, 1264, 1175 cm⁻¹; ESIMS *m*/*z* 287 ([M + Na⁺], C₁₁H₁₃NaO₃Cl requires 287). (-)-(*R*, *R*, *R*)-**5**: [α]²⁵_{D} -24 (*c* 0.20, CH₂Cl₂).

**Diels Alder Product (18):** Allene 5 (72 mg, 0.32 mmol) at 0 °C was treated neat with precooled (0 °C) 1,3-bistrimethylsilyloxy-1-methoxy-butadiene (**4,** 178 mg, 0.64 mmol) and stirred 1 h at 25 °C. The reaction was diluted with EtOH (1 mL), cooled to 0 °C, and treated with BF•Et₃N (750 µL, 2M solution in EtOH). The resulting solution was stirred 1 h at 25°C before dilution with CH₂Cl₂, washing with 5% aqueous NH₄Cl, and drying with Na₂SO₄. Evaporation of the solvents under reduced pressure followed by flash chromatography (SiO₂, 2 × 8 cm, 0-40% EtOAc/hexanes gradient) afforded the major regioisomer (**18,** 4:1) as a clear oil (49 mg, 50%): ¹H NMR (CDCl₃, 400 MHz) δ 11.78 (s, 1H), 6.43 (d, *J*= 2.5 Hz, 1H), 6.36 (d, *J*= 2.5 Hz, 1H), 6.11 (br s, 1H), 5.56 (ddd, *J* = 17.1, 9.7, 7.2 Hz, 1H), 5.46 (m, 2H), 5.31 (dd, *J* = 9.7, 2.0 Hz, 1H), 4.95 (d, *J* = 11.4 Hz, 1H), 4.72 (d, *J* = 11.4 Hz, 1H), 3.19 (dd, *J* = 7.1, 2.1 Hz, 1H), 3.06 (m, 1H), 2.15 (dt, *J* = 14.7,4.4 Hz, 1H), 1.96 (dt, *J*= 14.7, 7.4 Hz, 1H), 1.51 (d, *J*= 6.3 Hz, 3H); ¹³C NMR (CDCl₃, 100 MHz) δ 170.1, 166.0, 160.9, 141.6, 134.9, 120.5, 111.9, 104.6, 104.1, 71.0, 58.5, 57.7, 46.6, 38.5, 20.2; IR (film) *v*ₘₐₓ, 3352, 2985, 1651, 1621, 1454, 1358, 1259, 1166, 1107 cm⁻¹; ESIMS *m*/*z* 335 ([M + Na⁺], C₁₅H₁₇NaO₅Cl requires 335). (-)-(*R*, *R*, *R*)-**18**: [α]²⁵_{D} -38 (*c* 0.14, CH₂Cl₂).

**2-Chloromethyl-4,6-dihydroxybenzaldehyde (20):** A solution of 2-chloromethyl-4,6-dimethoxy benzaldehyde **18** (1.0 g, 4.65 mmol) in 50 mL of anhydrous CH₂Cl₂ at -78 °C was treated with boron tribromide (1.0 M in CH₂Cl₂, 23.3 mmol) dropwise via syringe. The solution was stirred at -78 °C for 10 min, warmed to 25 °C and stirred for 20 h. The reaction was quenched by the addition of 1N HCl (100 mL) and extracted with Et₂O (3 x 100 mL). The combined organic layers were dried over MgSO₄ filtered, and concentrated *in vacuo.* Chromatography on silica gel (SiO₂, 4 × 6 cm, 20% EtOAc/hexanes) afforded 20 (0.83 g, 96%) as a white solid: Mp 164 - 166 °C (Et₂O); ¹H NMR (DMSO_{*d6*}, 500 MHz) δ 11.71 (s, 1H), 10.90 (s, 1H), 10.18 (s, 1H), 6.50 (s, 1H), 6.31 (s, 1H), 5.01 (s, 2H); ¹³C NMR (DMSO_{*d6*}, 125 MHz) δ191.6, 165.3, 164.7, 142.5, 111.3, 110.9, 102.6, 42.9; IR (film) νₘₐₓ 3090, 1624, 1575, 1488, 1268, 1234, 1172, 729 cm⁻¹; ESIMS m/z 221 ([M + Cl]⁻, C₈H₇Cl₂O₃ requires 221).

**4-*tert*-Butyldiphenylsiloxy-2-chloromethyl-6-hydroxy benzaldehyde (21):** A solution of **20** (0.60 g, 3.22 mmol) in 55 mL 1:1 CH₂Cl₂ : THF at 0 °C was treated with imidazole (0.24 g, 3.55 mmol) followed by tert-butyldiphenylchlorosilane (0.89 g, 3.22 mmol). The reaction was warmed to 25 °C and stirred for 12 h. The reaction mixture was quenched with 1N HCl (100 mL) and extracted with Et₂O (3 x 100 mL). The combined organic layers were dried (MgSO₄), filtered, and concentrated *in vacuo.* Flash chromatography on silica gel (SiO₂, 4 × 6 cm, 10% EtOAc/hexanes) afforded **21** (1.33 g, 97%) as a white solid: Mp 87-89 °C (CH₂Cl₂); ¹H NMR (CDCl₃, 500 MHz) δ12.18 (s, 1H), 10.17 (s, 1H), 7.71 (d, *J* = 6.8 Hz, 4H), 7.48 (t, *J* = 7.3 Hz, 2H), 7.42 (t, *J* = 7.3 Hz, 4H), 6.38 (d, *J* = 2.1 Hz, 1H), 6.25 (d, *J* = 1.9 Hz, 1H), 4.61 (s, 2H), 1.13 (s, 9H); ¹³C NMR (CDCl₃, 125 MHz) δ192.8, 166.6, 163.5, 142.1, 135.8, 131.8, 130.9, 128.5, 115.5, 112.5, 109.2, 42.2, 26.8, 19.9; IR (film) *v*ₘₐₓ 3072, 2932, 2853, 1643, 1623, 1569, 1377, 1297, 1199, 1173, 1115, 784 cm⁻¹; ESIMS m/z 459 ([M + Cl]⁻, C₂₄H₂₅Cl₂O₃Si requires 459).

**4-*tert*-Butyldiphenylsiloxy-2-chloromethyl-6-hydroxybenzoic acid (22):** A solution of **21** (350 mg, 0.825 mmol) in THF : H₂O : DMSO (8.0 mL : 14 mL : 0.80 mL) was cooled to 0 °C. Sulfamic acid (224 mg, 2.31 mmol) was added followed by sodium chlorite (193 mg, 2.15 mmol) and the solution was stirred at 0 °C for 30 min. The reaction mixture was diluted with saturated aqueous NH₄Cl (50 mL) and extracted with Et₂O (3 x 50 mL). The combined organic layers were dried (MgSO₄), filtered, and concentrated *in vacuo* to afford **21** (360 mg, 100%) as a yellow foam. This material was used without further purification: ¹H NMR (CDCl₃, 500 MHz) δ11.33 (s, 1H), 7.70 (d, *J*= 6.8 Hz, 4H), 7.45 (t, *J*= 7.3 Hz, 2H), 7.39 (t, *J*= 7.3 Hz, 4H), 4.79 (s, 2H), 1.11 (s, 9H); ¹³C NMR (CDCl₃, 125 MHz) δ174.1, 166.2, 161.7, 142.0, 135.6, 131.9, 130.5, 128.2, 116.4, 1,08.6, 103.7, 46.1, 26.6, 19.7; IR (film) *v*ₘₐₓ 3050, 2958, 2858, 2564, 1641, 1612, 1428, 1365, 1253, 1175, 1115, 829, 700 cm⁻¹; ESIMS *m*/*z* 463 ([M + Na⁺], C₂₄H₂₅ClNaO₄Si requires 463).

**Ester (7):** A solution of DIAD (136 mg, 0.68 mmol) in benzene (7.5 mL) was treated with tri-2-furyl phosphine (160 mg, 0.68 mmol) and stirred at 25 °C for 10 min. Alcohol **8** (86 mg, 0.68 mmol) was then added in benzene (5 mL) and stirred 15 min afterwhich benzoic acid **22** (270 mg, 0.62 mmol) was added in benzene (5 mL) and stirring continued for 12 h. The reaction mixture was concentrated *in vacuo* and purified by chromatography on silica gel (SiO₂, 2 × 6 cm, 5% EtOAc/hexanes) to afford 7 as a colorless oil (220 mg, 65%): ¹H NMR (CDCl₃, 500 MHz) δ11.56 (s, 1H), 7.71 (d, *J*= 7.9 Hz, 4H), 7.46 (t, *J* = 7.3 Hz, 2H), 7.40 (t, *J*= 7.5 Hz, 4H), 6.46 (d, *J*= 2.4 Hz, 1H), 6.29, (d, *J* = 2.4 Hz, 1H), 5.58-5.39 (m, 3H), 5.28 (d, *J*= 10.0 Hz, 1H), 4.83 (d, *J* = 11.2 Hz, 1H), 4.65 (d, *J* = 11.2 Hz, 1H), 3.13 (dd, *J* = 7.4, 1.9 Hz, 1H), 2.99 (m, 1H), 2.10-1.95 (m, 2H), 1.48 (d, *J* = 6.4 Hz, 3H), 1.11 (s, 9H); ¹³C NMR (CDCl₃, 125 MHz) δ170.0, 165.5, 160.8, 140.8, 135.6, 135.0, 132.0, 130.5, 128.2, 119.9, 116.3, 108.8, 105.2, 71.0, 58.2, 57.2, 46.5, 38.3, 26.6, 20.0, 19.7; IR (film) *v*ₘₐₓ 3436, 3071, 2930, 2857, 1653, 1610, 1574, 1472, 1428, 1259, 1174, 822, 700 cm⁻¹; ESIMS m/z 573 ([M + Na⁺], C₃₁H₃₅ClNaO₅Sirequires 573); (-)-(*R, R, R*)-**7**: [α]²⁵_{D} -22 (*c* 1.8, CH₂Cl₂).

**Dithiane Addition Product (23).** The dithiane 6 (103 mg, 0.56 mmol) was charged to a flame dried flask equipped with a stir bar under Ar pressure. Anhydrous THF (2.0 mL) was added via cannula and the resulting solution was cooled to -20 °C. *n*BuLi (223 µL, 2.5 M solution in hexanes, 0.57 mmol) was added in a steady stream and the resulting dark purple reaction was stirred at -20 °C for 30 min, and then cooled to -78 °C. Concurrently, 7 (180 mg, 0.32 mmol) was charged to a flame dried flask equipped with a stir bar under Ar pressure. Anhydrous THF (2.0 mL) was added via cannula and the resulting solution was cooled to -78 °C, treated dropwise with *n*BuLi (131 µL, 2.5 M solution in hexanes, 0.32 mmol) and stirred 5 min. The dithiane anion solution was cannulated *into* the lithium salt solution of 7 at -78 °C. The resulting purple reaction was stirred 2 h at -78 °C and was then quenched by the addition of sat. NH₄Cl (10 mL). The mixture was extracted with Et₂O (15 mL), washed with sat. aqueous NaCl, and dried (Na₂SO₄). Evaporation of the solvents under reduced pressure followed by flash chromatography (SiO₂, 2 × 6 cm, 0-5% EtOAc/hexanes gradient) afforded an inseparable 6:1 mixture of α (**23**):γ (**23a**) alkylation products as a colorless oil (114 mg, 50%). This mixture was carried on and spectral data is reported for the major, desired adduct **23**: ¹H NMR (C₆D₆, 400 MHz) δ 11.64 (s, 1H), 7.78 (m, 4H), 7.14 (m, 6H, obscured by solvent peak), 6.73 (d, *J*= 2.5 Hz, 1H), 6.66 (d, *J* = 2.5 Hz, 1H), 6.30 (dd, *J* = 15.0, 10.5 Hz, 1H), 5.96 (ddd, *J* = 14.6, 10.5, 1.2 Hz, 1H), 5.51 (dd, *J*= 15.0, 6.8 Hz, 1H), 5.46 (d, *J*= 15.3 Hz, 1H), 5.41 (ddd, *J*= 13.0, 10.3, 7.5 Hz, 1H), 5.21 (dd, *J* = 17.0, 1.3 Hz, 1H), 5.20 (m, 1H), 4.98 (dd, *J* = 1.0.3, 1.3 Hz, 1H), 3.78 (d, *J* = 13.3 Hz, 1H), 3.63 (d, *J* = 13.3 Hz, 1H), 2.83 (dd, *J* = 7.5, 1.9 Hz, 1H), 2.74 (ddd, *J* = 6.7, 4.9, 1.9 Hz, 1H), 2.47 (m, 2H), 2.09 (m, 2H), 1.78 (ddd, *J* = 14.2, 6.5, 5.3 Hz, 1H), 1.68 (ddd, *J* = 14.2, 6. 5, 4.9 Hz, 1H), 1. 57 (m, 1H), 1.55 5 (d, *J* = 6.6 Hz, 3H), 1.3 7 (m, 1H), 1.27 (d, ,7 = 6.4 Hz, 3H), 1.14 (s, 9H); ¹³C NMR (C₆D₆, 100 MHz) δ 171.5, 165.1, 160.1, 139.1, 136.7, 136.2, 136.2, 135.0, 133.8, 133.0, 131.4, 130.7, 130.2, 128.2, 118.9, 109.3, 108.3, 71.3, 58.5, 57.0, 56.4, 47.1, 38.5, 27.7, 27.6, 27.0, 27.0, 25.8, 20.0, 20.0, 18.5; IR (film) *v*ₘₐₓ 2931, 1651, 1574, 1427, 13.11, 1256, 1171 cm⁻¹; ESIMS *m*/*z* 723 ([M + Na⁺], C₄₀H₄₉NaO₅S₂Si requires 723).

**TBS Protection of Phenol (24).** A solution of 23 (90 mg, 0.13 mmol) in 2.0 mL of anhydrous DMF at 0 °C was treated with imidazole (61 mg, 0.89 mmol) in one portion. *tert-*Butyldimethylchlorosilane (77 mg, 0.51 mmol) was added in one portion and the resulting reaction was warmed to 25 °C for 3 h. The reaction mixture was diluted with Et₂O (10 mL), and washed successively with 5% aqueous NH₄Cl (5 mL), H₂O (3 x 3 mL) and saturated aqueous NaCl (5 mL). The organic layer was dried (MgSO₄), concentrated under reduced pressure, and purified by flash chromatography (SiO₂, 2 × 6 cm, 0-4% EtOAc/hexanes gradient) to provide 24 (85 mg, 83%) as a colorless oil: ¹H NMR (CDCl₃, 400 MHz) δ 7.71 (m, 4H), 7.42 (m, 6H), 6.62 (d, *J* = 2.1 Hz, 1H), 6.23 (dd, *J* = 15.1, 10.4 Hz, 1H), 6.07 (m, 1H), 6.01 (d, *J* = 2.1 Hz, 1H), 5.66 (dq, *J* = 13.6, 13.6, 6.6 Hz, 1H), 5.56 (ddd, *J*= 17.4, 10.0, 7.4 Hz), 5.44 (m, 2H), 5.22 (m, 2H), 3.16 (d, *J* = 14.0 Hz, 1H), 3.10 (d, *J* = 14.0 Hz, 1H), 3.10 (dd, *J* = 7.5, 1.9 Hz, 1H), 3.00 (dt, *J* = 5.3, 1.9 Hz, 1H), 2.81 (m, 2H), 2.61 (m, 2H), 2.10-1.82 (m, 4H), 1.74 (d, *J* = 6.6 Hz, 3H), 1.43 (d, *J*= 6.4 Hz, 3H), 1.07 (s, 9H), 0.77 (s, 9H), -0.19 (s, 3H), -0.20 (s, 3H); ¹³C NMR (C₆D₆, 100 MHz) δ 166.9, 156.8, 154.6, 136.9, 136.3, 136.2, 136.2, 134.9, 134.0, 133.4, 132.0, 130.6, 129.8, 128.2, 122.8, 118.7, 117.4, 110.5, 69.8, 58.5, 57.1, 55.9, 46.0, 38.7, 27.8, 26.9, 26.2, 25.7, 20.3, 20.0, 18.7, 18.6, -4.1; IR (film) *v*ₘₐₓ 2931, 2857, 1733, 1597, 1558, 1472, 1428, 1340, 1256,' 1,170 cm⁻¹; ESIMS *m*/*z* 837 ([M + Na⁺], C₄₆H₆₂NaO₅S₂Si₂ requires 837). (-)-(*R*, *R*, *R*)-**24**: [α]²⁵_{D} -5.3 (*c* 1.2, CH₂Cl₂).

**RCM Product (26).** A solution of **24** (70 mg, 86 µmol) in 43 mL anhydrous CH₂Cl₂ under Ar was treated with Ru-catalyst **25** (7.0 mg, 8.6 µmol) and heated to 42 °C for 2 h. DMSO (40 µL) was added and the reaction was stirred at 25 °C overnight (Ahn *et al. Org. Lett.* **2001**, *3*, 1411). The reaction was concentrated and directly subjected to flash chromatography (SiO₂, 2 × 5 cm, 0-10% EtOAc/hexanes gradient) which provided pure macrocycle **26** (46 mg, 70%) as a colorless oil: ¹H NMR (CDCl₃, 400 MHz) δ 7.67 (m, 4H), 7.45-7.30 (m, 7H), 6.73 (dd, *J*= 15.6, 10.3 Hz, 1H), 6.11 (d, *J* = 2.2 Hz, 1H), 5.99 (dd, *J* = 10.7, 10.3 Hz, 1H), 5.71 (d, *J* = 15.6 Hz, 1H), 5.48 (dd, *J* = 10.7, 3.2 Hz, 1H), 5.23 (m, 1H), 3.67 (d, *J* = 16.1 Hz, 1H), 3.30 (m, 1H), 3.17 (d, *J* = 16.1 Hz, 1H), 3.00 (m, 2H), 2.75 (m, 2H), 2.61 (m, 1H), 2.33 (dt, *J* = 14.3, 2.9 Hz, 1H), 2.00 (m, 2H), 1.85 (m, 1H), 1.55 (d, *J*= 6.5 Hz, 3H), 1.51 (m, 1H), 1.07 (s, 9H), 0.83 (s, 9H), -0.04 (s, 3H), -0.05 (s, 3H); ¹³C NMR (CDCl₃, 100 MHz) δ 167.7, 156.5, 152.6, 136.2, 135.5, 135.5, 135.4, 132.6, 132.4, 131.1, 129.8, 128.4, 128.0, 127.7, 127.7, 121.5, 113.5, 108.7, 69.7, 60.3, 56.2, 55.7, 52.5, 38.8, 37.6,28.0, 26.4, 26.3, 25.4,24.0, 21.0, 19.4, 18.4, 17.9, 14.2, -4.5, -4.7; IR (film) *v*ₘₐₓ 2931, 2858, 1732, 1598, 1574, 1471, 1428, 1342, 1261, 1169 cm⁻¹; ESIMS *m*/*z* 773 ([M + H⁺], C₄₃H₅₇O₅Si₂S₂ requires 773). (-)-(*R*, *R*, *R*)-**26**: [α]²⁵_{D} -4.2 (*c* 0.58, CH₂Cl₂).

**Monocillin (2).** A solution of **26** (45 mg, 58 µmol) in 10 mL of anhydrous CH₂Cl₂ at -10 °C was treated with *m*CPBA (14.3 mg, 58 µmol, 75%) in one portion and stirred 5 min. The reaction was quenched with 10% Na₂S₂O₃ (10 mL) and 5% aqueous NaHCO₃ (5 mL), and extracted with CH₂Cl₂ (3 x 5 mL). The solvents were removed under reduced pressure and the crude monosulfoxide was dissolved in 9 mL of THF:H₂O:Ac₂O:Et₃N (10:1:3:4) and heated to 60 °C for 12 h. The reaction was concentrated under reduced pressure. The oil was dissovled in CH₂Cl₂ (10 mL), and washed with 5% aqueous NH₄Cl (10 mL), and the solvents were again removed. The crude was then dissolved in 10 mL 1:1 MeOH:5% aqueous NaHCO₃ and stirred an additional 12 h to remove the phenolic acetates. The resulting solution was extracted with EtOAc (3 x 5 mL), washed with sat. aqueous NaCl (10 mL), dried (Na₂SO₄) and the solvents were removed under reduced pressure. Flash chromatography (SiO₂, 2 × 5 cm, 50% EtOAc/hexanes) provided monocillin I (**2**) (11.4 mg, 60%) as a pale yellow solid: ¹H NMR (CDCl₃, 500 MHz) δ 11.38 (s, 1H), 7.85 (dd, *J* = 15.9, 11.4 Hz, 1H), 6.36 (d, *J* = 2.3 Hz, 1H), 6.33 (d, *J* = 2.3Hz, 1H), 6.27 (dd, *J* = 11.3, 10.4 Hz, 1H), 6:01 (br s, 1H), 5.99 (d, *J* = 15.9 Hz, 1H), 5.94 (dd, *J* = 10.3, 2.7 Hz, 1H), 5.58 (m, 1H), 5.21 (d, *J* = 13.9 Hz, 1H), 3.62 (d, *J* = 13.9 Hz, 1H), 3.30 (m, 1H), 3.10 (m, 1H), 2.42 (dt, *J* = 15.1, 3.0 Hz, 1H), 1.99 (m, 1H), 1.62 (d, *J* = 6.8 Hz, 3H); ¹³C NMR (CDCl₃, 100 MHz) δ 201.8, 170.0, 166.4, 162.3, 143.0, 139.1, 137.5, 130.7, 130.1, 110.2, 103.9, 103.4, 71.3, 55.8, 55.6, 43.3, 36.4, 19.1; IR (film) *v*ₘₐₓ 3310, 2916, 1652, 1616, 1456, 1259, 1203 cm⁻¹; ESIMS *m*/*z* 353.0 ([M + Na⁺], C₁₈H₁₈NaO₆ requires 353.0). (+)-(*R*, *R*, *R*)-**2**: [α]²⁵D +48 (*c* 0.38, CH₂Cl₂).

**Radicicol (1).** Monocillin I (**2**) (2.0 mg, 6.0 µmol) was dissolved in anhydrous Et₂O (0.5 mL) at 0 °C and treated with 210 µL of SO₂Cl₂ solution (0.06 M in CH₂Cl₂). The reaction was monitored closely by TLC to prevent over chlorination. Upon completion, the reaction was diluted with EtOAc (5 mL), washed with 5% aqueous NH₄Cl (5 mL), saturated aqueous NaCl (5 mL) and dried (Na₂SO₄). The solvents were removed under reduced pressure and flash chromatography (SiO₂, 2 × 5 cm, 60% EtOAc/hexanes) provided radicicol (1) (1.2 mg, 58%) as a white solid: ¹H NMR (CD₃OD, 400 MHz) δ 7.57 (dd, *J*= 16.1, 9.8, Hz, 1H), 6.46 (s, 1H), 6.21 (dd, *J* = 10.7, 9.9 Hz, 1H), 6.06 (d, *J*= 16.1 Hz, 1H), 5.75 (dd, *J*= 10.8, 4.0 Hz, 1H), 5.35 (m, 1H), 4.14 (d, *J* = 16.3 Hz, 1H), 3 .89 (d, *J* = 16.3 Hz, 1H), 3.29 (m, 1H, obscured by CD₃OD), 3.04 (dt, *J*= 8.6, 2.7 Hz, 1H), 2.39 (dt, *J*= 14.7, 3.4 Hz, 1H), 1.70 (m, 1H), 1.49 (d, *J*= 6.6 Hz, 3H); ¹³C NMR (CD₃OD, 100 MHz) δ 199.7, 169.2, 159.0, 158.1, 140.9, 137.2, 135.2, 131.7, 131.0, 115.2, 103.9, 103.9, 72.2,157.0, 56.6, 46.6, 37.8, 18.8; IR (film) *v*ₘₐₓ 3332, 2991, 1651, 1602, 1307, 1245, 1107 cm⁻¹; UVₘₐₓ (MeOH) 262.1 and 269.0 nm; ESIMS *m*/*z* 387.0 ([M + Na⁺], C₁₈H₁₇NaO₆Cl requires 387.0). (+)-(*R*, *R*, *R*)-**1**: [α]²⁵_{D} +89 (*c* 0.50, CH₂Cl₂).

**(2S, 3S, 5S)-5-(*tert*-Butyldiphenylsilyl)-2,3-(cyclopropyl)-hexan-1-ol (29).** To a solution of Et₂Zn (11.0 mL, 1.0 M in hexanes, 11.2 mmol) in 18 mL of anhydrous CH₂Cl₂ and 1.1 mL of anhydrous DME at -10 °C was added dropwise in sequential fashion: CH₂I₂ (1.82 mL, 22.0 mmol), (+)-TMAD-BBu (1.80 g, 6.7 mmol in 4.0 mL anhydrous CH₂Cl₂) and ent-**13** (2.0 g, 5.6 mmol in 4.0 mL anhydrous CH₂Cl₂). The reaction was then stirred at 25 °C for 12h. The reaction was then cooled to 0 °C and quenched by the addition of NH₄Cl (20 mL). The reaction was diluted with EtOAc (100 mL), and the organic layer was separated and dried with MgSO₄. Concentration under reduced pressure, followed by flash chromatography (SiO₂, 6 × 8 cm, 15-20% EtOAc/hexanes gradient) afforded **29** (1.74 g, 84%) as a colorless oil: ¹H NMR (CDCl₃, 400 MHz) δ 7.68 (m, 4H), 7.41 (m, 6H), 3.90 (m, 1H), 3.36 (m, 2H), 1.42 (ddd, *J*= 13.5, 6.7, 5.4 Hz, 1H), 1.27 (dt, *J* = 13.9, 6.9 Hz, 1H), 1.14 (d, *J* = 6.1 Hz, 3H), 1.06 (s, 9H), 0.78 (m, 1H), 0.58 (m, 1H), 0.29 (dt, *J*= 8.5, 4.7 Hz, 1H), 0.13 (dt, *J*= 8.1, 4.7 Hz, 1H); ¹³C NMR (CDCl₃, 100 MHz) δ 136.0, 136.0, 134.9, 134.7, 129.7, 129.7, 127.7, 127.7, 70.2, 67.3, 43.5, 27.2, 23.4, 21.5, 19.4, 13.6, 9.7; ESIMS *m*/*z* 391 ([M+Na⁺], C₂₃H₃₂NaO₂Si requires 391).

***(2S, 3S,* 5*S*)-5-(*tert*-Butyldiphenylsilyl)-2,3-(cyclopropyl)-hexan-1-al (29a).** A solution of **29** (1.73 g, 4.7 mmol) and Et₃N (3.3 mL, 24 mmol) in of 4:1 CH₂Cl₂/DMSO (40 mL) at 0 °C was treated with SO₃•pyridine (2.9 g, 17 mmol) and stirred 30 min at 25 °C. The reaction was diluted with EtOAc (200 mL), washed sequentially with H₂O (3 x 50 mL), saturated aqueous NaHCO₃ (50 mL), and saturated aqueous NaCl (50 mL) and dried (MgSO₄). Evaporation of the solvents under reduced pressure directly provided pure **29a** (1.6 g, 90%) as a colorless oil: ¹H NMR (CDCl₃, 500 MHz) δ 8.93 (d, *J* = 6.3 Hz, 1H), 7.68 (m, 4H), 7.43 (m, 6H), 3.94 (m, 1H), 1.50 (m, 2H), 1.22 (m, 2H), 1.19 (d, *J* = 6.3 Hz, 3H), 1.07 (s, 9H), 0.90 (m, 1H), 0.75 (m, 1H); ¹³C NMR (CDCl₃, 125 MHz) δ 201.0, 136.0, 136.0, 134.7, 134.3, 129.9, 129.8, 127.8, 127.7, 69.5, 42.5, 30.5, 27.2, 23.4, 19.4, 19.2, 15.0; ESIMS *m*/*z* 389 ([M + Na⁺], C₂₃H₃₀NaO₂Si requires 389).

**(3*S,* 4*S,* 6*S*)-6-(*tert*-Butyldiphenylsilyl)-3,4-(cyclopropyl)-hept-1-ene (14c).** Methyltriphenylphosphonium bromide (3.3 g, 9.4 mmol) and a stir bar were added to a flask and thoroughly flame dried. Anhydrous THF (50 mL) was added via cannula under Ar and the resulting suspension was cooled to 0 °C prior to the addition of NaHMDS (9.4 mL, 9.4 mmol, 1.0 M in THF) in dropwise fashion. The resulting gold suspension was warmed to 25 °C for 30 min and then recooled to -10 °C prior to the addition of aldehyde (1.05 g, 2.85 mmol) in anhydrous THF (5.0 mL). The reaction was complete within 10 min, and was quenched by the addition of saturated aqueous NH₄Cl (50 mL). The mixture was then extracted with Et₂O (100 mL), washed sequentially with H₂O (50 mL) and saturated aqueous NaCl (50 mL) and dried (MgSO₄). Removal of the solvents under reduced pressure followed by flash chromatography (SiO₂, 6 × 6 cm, 2% EtOAc/hexanes) provided **14c** (1.23 g, 71%) as a colorless oil: ¹H NMR (CDCl₃, 500 MHz) δ 7.69 (m, 4H), 7.40 (m, 6H), 5.33 (m, 1H), 4.99 (dd, *J*= 17.0, 1.6 Hz, 1H), 4.81 (dd, *J* = 10.2, 1. 6 Hz, 1H), 3.92 (m, 1H), 1.46 (m, 1H), 1.3 6 (m, 1H), 1.09 (d, *J* = 6.2 Hz, 3H), 1.06 (s, 9H), 0.90 (m, 1H), 0.73 (m, 1H), 0.50 (dt, *J*= 8.8, 4.5 Hz, 1H), 0.34 (dt, *J*= 8.1, 4.5 Hz, 1H); ¹³C NMR (CDCl₃, 125 MHz) δ 142.1, 136.1, 135.1, 134.7, 129.7, 129.6, 127.7, 127.6, 111.4, 70.1, 43.8, 27.2, 23.4, 22.8, 19.4, 17.7, 13.8; ESIMS *m*/*z* 387 ([M + Na⁺], C₂₃H₃₀NaO₂Si requires 387).

**(3S, 4S, 6S)-3,4-(Cyclopropyl)-6-(hydroxy)-hept-1-ene (30).** To a solution of 29a (1.23 g, 3.37 mmol) in 20 mL of anhydrous THF at 25 °C was treated with *n*Bu₄NF (3.7 mL, 1.0 M solution in THF, 3.7 mmol) and stirred 4 h. The reaction was concentrated under reduced pressure. Flash chromatography (SiO₂, 3 × 8 cm, 0-40% EtOAc/hexanes gradient) provided 30 (0.378 g, 89%) as a colorless and volatile oil: ¹H NMR (CDCl₃, 500 MHz) δ 5.39 (ddd, *J* = 17.2, 10.2, 9.1 Hz, 1H), 5.04 (dd, *J* = 17.1, 1.5 Hz, 1H), 4.85 (dd, *J* = 10.2, 1.5 Hz, 1H), 3.90 (m, 1H), 2.39 (m, 1H), 1.43 (m, 2H), 1.22 (d, *J* = 6.1 Hz, 3H), 1.19 (m, 1H), 0.80 (m, 1H), 0.64 (dt, *J* = 9.1, 4.7 Hz, 1H), 0.59 (dt, *J* = 10.0, 4.7 Hz, 1H); ¹³C NMR (CDCl₃, 125 MHz) δ 141.7, 111.9, 68.6, 43.3, 23.3, 22.1, 17.7, 13.9; ESIMS *m*/*z* 149 (M + Na⁺, C₈H₁₄NaO requires 149).

**Ester (39):** A solution of DIAD (0.73 mL, 3.6 mmol) in benzene (40 mL) was treated with tri-2-furyl phosphine (850 mg, 3.6 mmol) and stirred at 25 °C for 10 min. Alcohol 30 (420 mg, 3.3 mmol) was then added in benzene (5 mL) and stirred 15 min after which benzoic acid 22 (1.6 g, 3.6 mmol) was added in benzene (5 mL) and stirring continued for 12 h. The reaction mixture was concentrated *in vacuo* and purified by chromatography On silica gel (SiO₂, 2 × 6 cm, 4% EtOAc/hexanes) to afford 39 as a colorless oil (1.40 g, 77%): ¹H NMR (CDCl₃, 400 MHz) δ 11.71 (s, 1H), 7.73 (m, 4H), 7.45 (m, 6H), 6.46 (d, *J* = 2.5 Hz, 1H), 6.29, (d, *J* = 2.5 Hz, 1H), 5.33 (m, 2H), 5.01 (dd, *J* = 17.1, 1.4 Hz, 1H), 4.84 (dd, *J* = 10.3, 1.5 Hz, 1H), 4.77 (d, *J* = 11.2 Hz, 1H), 4.67 (d, *J* = 11.2 Hz, 1H), 1.78 (dt, *J* = 13.7, 6.3 Hz, 1H), 1.64 (dt, *J* = 13.7, 7.1 Hz, 1H), 1.43 (d, *J*= 6.0 Hz, 3H), 1.16 (m, 1H), 1.12 (s, 9H), 0.81 (m, 1H), 0.61 (m, 2H); ¹³C NMR (CDCl₃, 100 MHz) δ 170.1, 165.4, 160.6, 141.3, 140.7, 135.6, 132.0, 130.5, 128.2, 128.1, 116.1, 112.2, 108.7, 105.3, 73.4, 46.5, 39.7, 26.6, 22.8, 19.7, 19.7, 17.1, 13.6; IR (film) νₘₐₓ 3072, 2959.' 1653, 1609, 1574, 1355, 1259 cm⁻¹; ESIMS m/z 571 ([M + Na⁺], C₃₂H₃₇ClNaO₄Si requires 571).

**Dithiane Addition Product (23c).** The dithiane 6 (477 mg, 2.5 mmol) was charged to a flame dried flask equipped with a stir bar under Ar pressure. Anhydrous THF (5.0 mL) was added via cannula and the resulting solution was cooled to -20 °C. *n*BuLi (1.05 mL, 2.5 M solution in hexanes, 2.6 mmol) was added in a steady stream and the resulting dark purple reaction was stirred at -20 °C for 30 min, and then cooled to -78 °C. Concurrently, **39** (780 mg, 1.4 mmol) was charged to a flame dried flask equipped with a stir bar under Ar pressure. Anhydrous THF (5.0 mL) was added via cannula and the resulting solution was cooled to -78 °C, treated dropwise with *n*BuLi (570 µL, 2.5 M solution in hexanes, 1.4 mmol) and stirred 5 min. The dithiane anion solution was cannulated *into* the lithium salt solution of **39** at -78 °C. The resulting blue reaction was stirred 2 h at -78 °C and was then quenched by the addition of sat. NH₄Cl (50 mL). The mixture was extracted with Et₂O (100 mL), washed with sat. aqueous NaCl, and dried (Na₂SO₄). Evaporation of the solvents under reduced pressure followed by flash chromatography (SiO₂, 2 × 6 cm, 0-2% EtOAc/hexanes gradient) afforded an inseparable 3:1 mixture of α (**23c**):γ alkylation products as a yellow oil (696 mg, 70%) data is for major product **23c**: ¹H NMR (C₆D₆, 400 MHz) δ 11.08 (s, 1H), 7.71 (m, 4H), 7.41 (m, 6H), 6.35 (d, *J*= 2.2 Hz, 1H), 6.26 (d, *J* = 2.2 Hz, 1H), 6.12 (dd, *J* = 10.6, 3.9 Hz, 1H), 6.00 (m, 1H), 5.69 (dd, *J*= 14.7, 6.8 Hz, 1H)), 5.35 (m, 2H), 5.17 (dd, *J* = 12.5, 6.2 Hz, 1H), 4.96 (dd, *J* = 17.0, 1.6 Hz, 1H), 4.78 (dd, *J* = 6.8, 1.6 Hz, 1H), 3.68 (d, *J* = 13 .9 Hz, 1H), 3.43 (d, *J* = 13.9 Hz, 1H), 2.76 (m, 2H), 2.52 (m, 2H), 2.00 (m, 1H), 1.75 (d, *J*= 7.4 Hz, 3H), 1.62 (m, 1H), 1.43 (d, *J*= 6.3 Hz, 3H), 1.31 (m, 1H), 1.25 (m, 1H), 1.09 (s, 9H), 0.83 (m, 1H), 0.63 (m, 1H), 0.55 (m, 1H); ¹³C NMR (C₆D₆, 100 MHz) δ 171.2, 163.7, 159.4, 141.6, 138.3, 135.9, 134.7, 132.8, 132.6, 130.8, 130.4, 128.2, 118.5, 112.2, 108.8, 107.6, 73.5, 56.0, 46.6, 39.9, 32.0, 27.7, 27.7, 26.8, 25.7, 23.0, 22.8, 20.1, 19.8, 18.4, 17.4, 14.6, 14.1; ESIMS *m*/*z* 721 ([M + Na⁺], C₄₁H₅₀NaO₄S₂Si requires 721).

**TBS Protection of Phenol (24c).** A solution of **23c** (686 mg, 0.98 mmol) in 4.0 mL of anhydrous DMF at 0 °C was treated with imidazole (270 mg, 3.9 mmol) in one portion. *tert-*Butyldimethylchlorosilane (300 mg, 1.9 mmol) was added in one portion and the resulting reaction was warmed to 25 °C for 3 h. The reaction mixture was diluted with Et₂O (30 mL), and washed successively with 5% aqueous NHaCl (10 mL), H₂O (3 x 5 mL) and saturated aqueous NaCl (10 mL). The organic layer was dried (MgSO₄), concentrated under reduced pressure, and purified by flash chromatography (SiO₂, 2 × 6 cm, 0-3% EtOAc/hexanes gradient) to provide a mixture of isomers of **24c** (85 mg, 83%) as a yellow oil. This mixture was separated using HPLC (Dynamax 60 Å, SiO₂, 25 × 100 mm, 15 mL/min, 2% EtOAc/hexanes, 50 mg injections) to affore pure **24c** as a colorless oil: ¹H NMR (CDCl₃, 400 MHz) δ 7.70 (m, 4H), 7.42 (m, 6H), 6.64 (d, *J* = 1.9 Hz, 1H), 6.26 (dd, *J* = 15.0, 10.5 Hz, 1H), 6.08 (m, 1H), 6.01 (d, *J* = 1.9 Hz, 1H), 5.66 (dq, *J* = 13.4, 6.6 Hz, 1H), 5.45 (d, *J* = 15.1 Hz, 1H), 5.37 (m, 1H), 5.10 (m, 1H), 5.02 (d, *J* = 15.1 Hz, 1H), 4.83 (d, *J* = 10.2 Hz, 1H), 3.17 (m, 2H), 2.81 (m, 2H), 2.62 (m, 2H), 1.95 (m, 1H), 1.80 (m, 1H), 1.75 (d, *J*= 6.4 Hz, 3H), 1.48 (m, 1H), 1.38 (d, *J*= 6.2 Hz, 3H), 1.21 (m, 1H), 1.07 (s, 9H), 0.85 (m,1H), 0.79 (s, 9H), 0.63 (m, 1H), 0.56 (m, 1H), -0.18 (s, 3H), -0.19 (s, 3H); ¹³C NMR (C₆D₆, 100 MHz) δ 167.1, 155.9, 153.5, 141.6, 135.6, 134.8, 134.3, 132.9, 132.7, 131.0, 130.0, 129.8, 128.5, 128.0, 12.1.7, 116.5, 111.8, 109.6, 72.1, 55.2, 53.6, 45.0, 39.9, 27.5, 26.5, 25.7, 25.3, 22.6, 19.8, 19.5, 18.4, 18.2, 17.3, 14.1, -4.6; ESIMS *m*/*z* 835 ([M + Na⁺], C₄₇H₆₄NaO₄S₂Si₂ requires 835).

**RCM Product (26c).** A solution of **24c** (9.5 mg, 11.6 µmol) in 20 mL anhydrous benzene under Ar was treated with Ru-catalyst **22** (1.0 mg, 1.2 µmol) and heated to 80 °C for 12 h. The reaction was concentrated and directly subjected to flash chromatography (SiO₂, 1x5 cm, 0-10% EtOAc/hexanes gradient) which provided pure macrocycle **26c** (1.8 mg, 20%) as a colorless oil: ¹H NMR (CDCl₃, 400 MHz) δ 7.67 (m, 4H), 7.45-7.30 (m, 7H), 7.00 (dd, *J*= 15.7, 9.6 Hz, 1H), 6.11 (d, *J* = 2.0 Hz, 1H), 5.89 (dd, *J* =11.7, 10.6 Hz, 1H), 5.62 (d, *J* = 15.7 Hz, 1H), 5.17 (m, 2H), 3.70 (d, *J* = 16.1 Hz, 1H), 3.19 (d, *J* = 16.1 Hz, 1H), 3.04 (m, 1H), 2.73 (m, 2H), 2.52 (m, 1H), 2.14 (m, 1H), 2.02 (m, 1H), 1.85 (m, 1H), 1.50 (m, 1H), 1.47 (d, *J*= 6.4 Hz, 3H), 1.07 (s, 9H), 0.83 (s, 9H), 0.80 (m, 2H obscured), 0.52 (ddd, *J* = 8.5, 4.9, 4.7 Hz, 1H), 0.44 (ddd, *J* = 8.9, 4.7, 4.0 Hz, 1H), -0.06 (s, 3H), -0.07 (s, 3H); ESIMS *m*/*z* 771 ([M + Na⁺], C₄₄H₅₈O₄Si₂S₂ requires 793).

**Cyclopropyl-Monocillin (2c).** A solution of **26c** (4.0 mg, 5.1 µmol) in 1 mL of anhydrous CH₂Cl₂ at -10 °C was treated with *m*CPBA (1.1 mg, 5.1 µmol, 80%) in one portion and stirred 5 min. The reaction was quenched with 10% Na₂S₂O₃ (1 mL) and 5% aqueous NaHCO₃ (1 mL), and extracted with CH₂Cl₂ (3 x 2 mL). The solvents were removed under reduced pressure and the crude monosulfoxide was dissolved in 2 mL of THF:H₂O:Ac₂O:Et₃N (10:1:3:4) and heated to 60 °C for 12 h. The reaction was concentrated under reduced pressure. The oil was dissovled in CH₂Cl₂ (10 mL), and washed with 5% aqueous NH₄Cl (10 mL), and the solvents were again removed. The crude was then dissolved in 3 mL 1:1 MeOH:5% aqueous NaHCO₃ and stirred an additional 12 h to remove the phenolic acetates. The resulting solution was extracted with CH₂Cl₂ (3 x 5 mL), washed with sat. aqueous NH₄Cl (10 mL), dried (Na₂SO₄) and the solvents were removed under reduced pressure. Flash chromatography (SiO₂, 2 × 5 cm, 30% EtOAc/hexanes) provided monocillin I (**2c**) (1.1 mg, 60%) as a pale white solid: ¹H NMR (CDCl₃, 400 MHz) δ 11.33 (s, 1H), 8.30 (dd, *J*= 15.8, 11.4 Hz, 1H), 7.00 (s, 1H), 6.40 (d, *J* = 2.3 Hz, 1H), 6.37 (d, *J* = 2.3 Hz, 1H), 6.20 (dd, *J*= 10.7, 10.6 Hz, 1H), 5.99 (d, *J* = 15.9 Hz, 1H), 5.69 (dd, *J* = 9.5, 6.0 Hz, 1H), 5.46 (m, 1H), 5.28 (d, *J*= 13.9 Hz, 1H), 3.62 (d, *J* = 13.9 Hz, 1H), 2.28 (m, 1H), 1.99 (m, 1H), 1.54 (d, *J*= 6.7 Hz, 3H), 0.90 (m, 2H), 0.72 (m, 1H), 0.62 (m, 1H); ESIMS *m*/*z* 351.0 ([M + Na⁺], C₁₉H₂₀NaO₅ requires 351.0).

**Cyclopropyl-Radicicol (40).** Cyclopropyl-monocillin I (**2c**) (2.0 mg, 6.0 µmol) was dissolved in anhydrous Et₂O (0.5 mL) at 0 °C and treated with 210 µL of SO₂Cl₂ solution (0.06 M in CH₂Cl₂). The reaction was monitored closely by TLC to prevent over chlorination. Upon completion, the reaction was diluted with EtOAc (5 mL), washed with 5% aqueous NH₄Cl (5 mL), saturated aqueous NaCl (5 mL) and dried (Na₂SO₄). The solvents were removed under reduced pressure and flash chromatography (SiO₂, 1 × 5 cm, 30% EtOAc/hexanes) provided cyclopropyl-radicicol (**40**) (1.6 mg, 80%) as a white solid:¹H NMR (CDCl₃, 500 MHz) δ 10.89 (s, 1H), 7.99 (dd, *J* = 15.6, 11.0, Hz, 1H), 6.62 (s, 1H), 6.12 (dd, *J* = 9.7, 9.6 Hz, 1H), 6.02 (d, *J* = 16.3 Hz, 1H), 5.60 (dd, *J*= 10.0, 5.8 Hz, 1H), 5.44 (m, 1H), 4.89 (m, 1H), 3.80 (m, 1H), 2.21 (dt, *J* = 15.8, 3.2 Hz, 1H), 1.70 (m, 1H), 1.47 (d, *J* = 6.7 Hz, 3H), 1.10 (m, 1H), 0.87 (m, 1H), 0.66 (ddd, *J*= 8.7, 5.1, 5.0 Hz, 1H), 0.55 (ddd, *J*= 8.5, 5.0, 4.9 Hz, 1H); ESIMS *m*/*z* 387.0 ([M + Na⁺], C₁₉H₁₉NaO₅Cl requires 387.0).

### Cell Culture Experimental:

The human cancer cell lines MCF7, BT474 and N417 were obtained from the American Type Culture Collection (Manassas, VA, USA) and maintained in a 1:1 mixture of DME:F12 supplemented with 2mM glutamine, 50 U/mL penicillin, 50 U/mL streptomycin and 5% heat inactivated fetal bovie serum (Gemini Bioproducts) and incubated at 37 °C in 5% CO₂.

### Protein Assays:

Cells were grown to 60-70% confluence and exposed to drugs or DMSO vehicle for the indicated time periods. Lysates were prepared using 50mM Tris pH 7.4, 2% SDS and 10% glycerol lysis buffer. Protein concentration was determined using the BCA kit (Pierce Chemical Co.) according to the manufacturer's instructions. Clarified protein lysates (20-50 µg) weere electrophoretically resolved on denaturing SDS-PAGE, transferred to ntirocellulose and probed with the following primary antibodies: anti-Her2 (C-18).

### Antiproliferative Index:

Growth assays were performed by seeding 10000 cells (MCF7, BT474 or N417) per well in 6-welldishes and incubating for 24 h before drug treatment. Drugs or vehicle were administered as outlined for each experiment, and cells were incubated for the time periods depcited and then the number quantified by a Coulter counter.

### Flow Cytometry:

Cell cycle distribution was assayed according to Nusse et al. With a Becton Dickinson fluorescence-activated cell sorter and analyzed by a Cell Cycle Multi-cycle system (Phoenix Flow System, San Diego, CA, USA).

### In vivo activity:

Although a variety of methods known in the art can be utilized, one exemplary method by which the *in vivo* activity of the inventive compounds is determined is by subcutaneously transplanting a desired tumor mass in mice. Drug treatment is then initiated when tumor mass reaches approximately 100 mm³ after transplantation of the tumor mass. A suitable composition, comprising any one inventive compounds described above, including classes thereof, subclasses thereof, or species thereof, optionally further comprising a pharmaceutically acceptable carrier and optionally further comprising an additional therapeutic agenthas, is then administered to the mice, preferably in saline and also administered once a day at doses in the range of 0.001 mg/kg, to about 50 mg/kg, although it will be appreciated that other doses can also be administered, as described herein (e.g., 0.01 mg/kg to about 25 mg/kg of body weight, or 0.1 mg/kg to about 10. mg/kg of body weight), or, in some embodiments, at dosages in the range of about 50 mg/kg to about 100 mg/kg, or dosages below 0.001 mg/kg. Body weight and tumor size are then measured daily and changes in percent ratio to initial values are plotted. In cases where the transplanted tumor ulcerates, the weight loss exceeds 25-30% of control weight loss, the tumor weight reaches 10% of the body weight of the cancer-bearing mouse, or the cancer-bearing mouse is dying, the animal is sacrificed in accordance with NIH guidelines for animal welfare. For additional guidance on mouse models see, http://mmhcc.nci.nih.gov/mmhcc/organ_models.

## Claims

1. A compound having the structure: 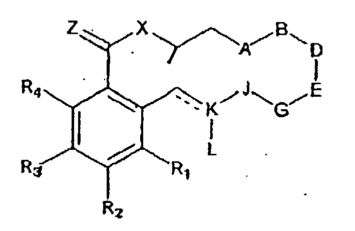 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylbeteroaryl moiety, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein Rp is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 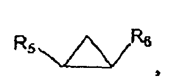 wherein R₅ and R₆ are each independently hydrogen, halogen, cyano, -OR_{J}, -N(R_{J})₂,-SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -O(O)NR_{K}, -S(O)₂R_{K}, -O(C=O)R_{K}, -N(R_{K})(C=O)(R_{K}), -C(O)R_{K}, , -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅-CHR₆-, R₅ and R₆ taken together represent a substituted or unsubstituted 3-7 membered aliphatic, heteroaliphatic, aryl or heteroaryl ring,
D and E together represent -CHR₈-CHR₉-, -CR₈=CR₉-, wherein R₈ and R₉ are each independently hydrogen or lower alkyl;
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geledanamycin, analogues of geldanamycin, and steroids; and
pharmaceutically acceptable derivatives thereof,

2. The compound of claim 1, wherein one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, wherein the linker is an aliphatic or heteroaliphatic moiety, whereby said aliphatic or heteroaliphatic moiety is substituted or unsubstituted, branched or unbranched, or cyclic or acyclic.

3. The compound of claim 1, wherein one or any two of R₁, R_{A}, R₂, R_{B}, R₃, R_{C}, R₄, R_{D}, R₅, R₆, R_{J}, or R_{L} are a linker covalently bonded to a compound selected from the group consisting of radicicol, monocillin, analogues of radicicol and monocillin, geldanamycin, analogues of geldanamycin, and steroids, wherein the linker is a moiety having one or the structures -(CH₂)ₙ₋CH=CH-(CH₂)ₘ-, -(CH₂)ₚ-C≡C-(CH₂)_{q}-, or -CH₂(CH₂)ₛCH₂-, wherein each occurrence of n, m, p, q and s is independently an integer from 0-10, and wherein one or more of the hydrogen atoms are optionally replaced with an alkyl, heteroalkyl, aryl, heteroaryl, alkylaryl or alkylheteroaryl moiety or a secondary or tertiary amine, hydroxyl, or thiol.

4. The compound of claim 1, wherein R₁ and R₃ are each independently halogen, hydrogen, or lower alkyl; R₂ is hydrogen or -OR_{B}, wherein each occurrence of R_{B} is independently hydrogen, a protecting group or an aliphatic, heteroaliphatic, aryl, heteroaryl, alkylaryl, or alkylheteroaryl moiety, and R₄ is hydrogen or -OR_{D}, wherein each occurrence of R_{D} is independently hydrogen, a protecting group or an aliphatic, heteroaliphatic, aryl, heteroaryl, alkylaryl, or alkylheteroaryl moiety.

5. The compound of claim 1, wherein the compound has the structure: 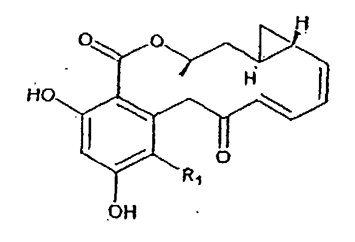 wherein R₁ is hydrogen or Cl.

6. A compound having the structure: 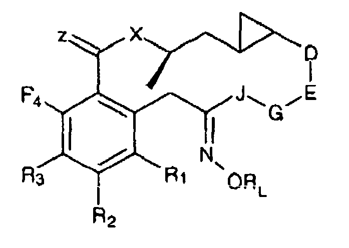 wherein
R¹ is halide, R² is OR_{B}, R³ is hydrogen and R⁴ is OR_{D} wherein R_{B} and R_{D} are hydrogen.
Z is O,
X is O
C and E are -CR₈-CR₉- wherein R₈ and R₉ are hydrogen.
G and J are -CR₁₀-CR₁₁- wherein R₁₀ and R₁₁ are hydrogen.
R_{L} is hydrogen, a protecting group, an aliphatic, heteroaliphatic; aryl, heteroaryl, arylalkyl or heteroarylalkyl moiety.

7. A pharmaceutical composition comprising a compound of claim 1 and a pharmaceutically acceptable carrier.

8. The pharmaceutical composition of claim 7, further comprising one or more additional therapeutic agents, wherein the one or more additional therapeutic agents preferably comprises an anticancer agent.

9. A compound of claim 1 for use in the treatment of cancer in a subject in need thereof.

10. A compound as claimed in claim 9 wherein the compound is provided in an amount in the range of 0.001 mg/kg to 100 mg/kg of body weight, preferably in the range of 0.01 mg/kg to about 25 mg/kg of body weight.

11. A compound as claimed in claim 10 for use in inhibiting the growth of or killing cancer cells.

12. A compound for treating a cancer in which the cancer cells comprise Rb negative cancer cells, which compound has the structure: 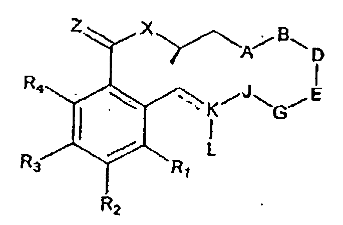 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein R_{F} is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 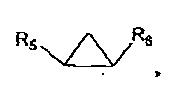 wherein R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(Rⱼ)₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -O(O)NR_{K}, -S(O)₂R_{K}, -O(C=O)R_{K}, -N(R_{K})(C=O)(R_{K}), -C(O)R_{K}, , -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅-CHR₆-, R₅ and R₆ taken together represent a substituted or unsubstituted 3-7 membered aliphatic, heteroaliphatic, aryl or heteroaryl ring,
D and E together represent -CHR₈-CHR₉-, -CR₈=CR₉-, wherein R₈ and R₉ are each independently hydrogen or lower alkyl;
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-CR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geledanamycin, analogues of geldanamycin, and steroids; and
pharmaceutically acceptable derivatives thereof.

13. A compound as claimed in claim 12 wherein the compound is provided in an amount in the range of 0.001 mg/kg to 50 mg/kg of body weight, preferably in the range of 0.01 mg/kg to about 25 mg/kg of body weight.

14. A compound as claimed in claim 12 wherein the cancer comprising Rb negative cells is small cell lung cancer, glioblastoma or retinoblastoma.

15. A compound for use in inhibiting the growth of or killing Rb negative cancer cells, which compound has the structure: 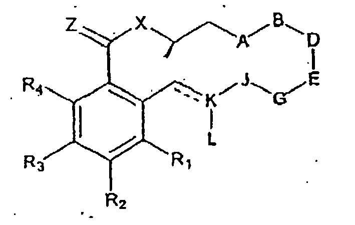 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(RD)(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein R_{F} is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 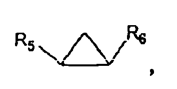 wherein R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCC₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -O(O)NR_{K}, -S(O)₂R_{K}, -O(C=O)R_{K}, -N(R_{K})(C=O)(R_{K}), -C(O)R_{K}, -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅-CHR₆-, R₅ and R₆ taken together represent a substituted or unsubstituted 3-7 membered aliphatic, heteroaliphatic, aryl or heteroaryl ring,
D and E together represent -CHR₈-CHR₉-, -CR₈=CR₉-, wherein R₈ and R₉ are each independently hydrogen or lower alkyl;
G and J together represent -CHR₁₀-CHR₁₁-, CR₁₀=CR₁₁-, wherein R₁₀ and R₁₁ are each independently hydrogen or lower alkyl;
K and L together represent C=O, C=S, CH-CH₃, CH-CH(RL)₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geledanamycin, analogues of geldanamycin, and steroids; and
pharmaceutically acceptable derivatives thereof.

16. A compound as claimed in claim 10 wherein the Rb negative cancer cells are small cell lung cancer, glioblastoma or retinoblastoma cells.

17. The use of a compound of formula I in the manufacture of a medicament for treating cancer.

18. A method for the synthesis of a compound having the structure (I): 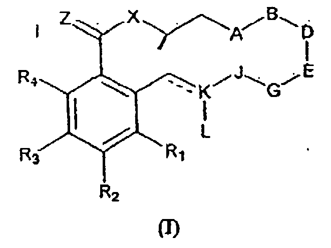 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{C})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein Rp is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 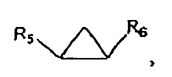 wherein R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -O(O)NR_{K}, -S(O)₂R_{K}, -O(C=O)R_{K}, -N(R_{K})(C=O)(R_{K}), -C(O)R_{K}, , -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅-CHR₆-, R₅ and R₆ taken together represent a substituted or unsubstituted 3-7 membered aliphatic, heteroaliphatic, aryl or heteroaryl ring,
D and E together represent -CHR_{B}-CHR₉-, -CR₈=CR₉-, wherein R₈ and R₉ are each independently hydrogen or lower alkyl;
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L}-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geledanamycin, analogues of geldanamycin, and steroids;
said method comprising:
(1) providing a benzoic acid component having the structure: 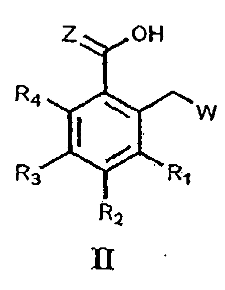 wherein R₁-R₄ and Z are as defined above, and wherein W is halogen; and
reacting the benzoic acid component with a chiral component (IV) having the structure: 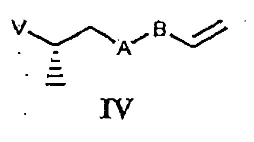 wherein A and B are as defined above, and wherein V is NHR_{G}, wherein R_{G} is hydrogen or lower alkyl; SH; or OH in the presence of an esterification reagent to generate an intermediate (V) having the structure: 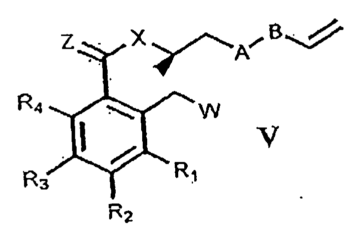
(2) reacting the intermediate (V) with a dithiane having the structure (III): 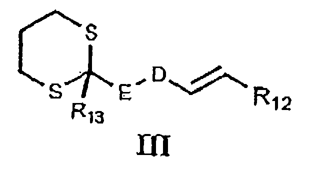 wherein R₁₃ is hydrogen or an alkali metal salt and wherein R₁₂ is hydrogen or lower alkyl under conditions to add the dithiane to generate an intermediate (VI) having the structure: 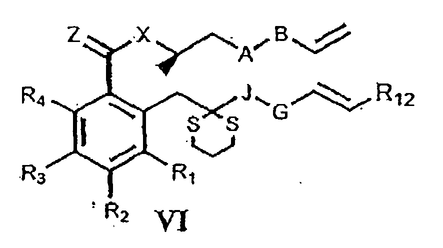
(3) if any one or more of R₁-R₄ is an unprotected thio, amino or hydroxyl group, optionally protecting said unprotected group;
(4) cyclizing the compound in the present of an olefin metathesis catalyst to generate the compound (VII): 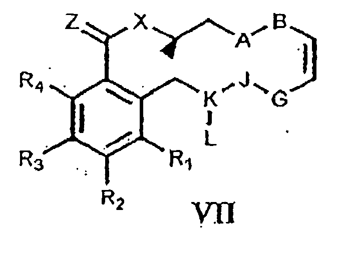
(5) optionally further reacting (VII) with one or more reagents to diversify and optionally deprotecting the macrolide to generate the compound (I).

19. The method of claim 18, wherein the step of esterification is performed using diethylazodicarboxylate (DIAD) in the presence of triphenylphosphine or trifurylphosphine.

20. The method of claim 18, wherein the step of olefin metathesis is performed using an olefin metathesis catalyst, or wherein the step of olefin metathesis is performed using a ruthenium-based olefin metathesis catalyst, or wherein the step of olefin metathesis is performed using Ru(1,3-dimesityl-4,5-dihydro-imidazol-2-ylidene)(=CHCH=C(CH₃)₂)PCp₃Cl₂.

21. A method for synthesis of a macrocycle having the structure (VII): 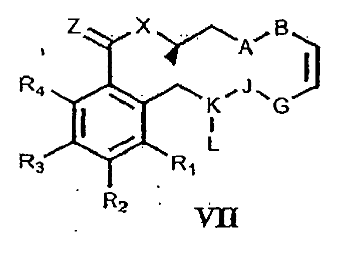 wherein the dotted line --- represents a bond, whereby a double bond is present, or the dotted line --- is absent, whereby a single bond is present;
R₁ is hydrogen, halogen, an aliphatic, heteroaliphatic, aryl, heteroaryl, alkylaryl, or alkylheteroaryl moiety, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or an aliphatic, heteroaliphatic, aryl, heteroaryl, alkylaryl, or alkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, an aliphatic, heteroaliphatic, aryl, heteroaryl, alkylaryl, or alkylheteroaryl moiety, or -N(R_{c})₂, wherein each occurrence of R_{C} is independently hydrogen, a protecting group, or an aliphatic, heteroaliphatic, aryl, heteroaryl, alkylaryl, or alkylheteroaryl moiety;
R₄ is hydrogen, halogen, cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or an aliphatic, heteroaliphatic, aryl, heteroaryl, alkylaryl, or alkylheteroaryl moiety, wherein each occurrence of R_{D} is independently hydrogen, a protecting group or an aliphatic, heteroaliphatic, aryl, heteroaryl, alkylaryl, or alkylheteroaryl moiety;
Z is O, S or NR_{E}, wherein R_{E} is hydrogen, a protecting group, an aliphatic, heteroaliphatic, aryl, heteroaryl, alkylaryl, or alkylheteroaryl moiety, or OR_{F}, wherein R_{F} is hydrogen, a protecting group, an aliphatic, heteroaliphatic, aryl, heteroaryl, alkylaryl, or alkylheteroaryl moiety;
X is O, S or NR_{G}, wherein R_{G} is hydrogen or lower alkyl;
A and B together represent 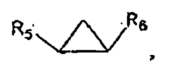 wherein R₅ and R₆ are each independently hydrogen, halogen, cyano, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} or an aliphatic, heteroaliphatic, aryl, heteroaryl, alkylaryl, or alkylheteroaryl moiety, wherein each occurrence of R_{J} is independently hydrogen, a protecting group, or an aliphatic, heteroaliphatic, aryl, heteroaryl, alkylaryl, or alkylheteroaryl moiety, and wherein R₇ is hydrogen, a protecting group, -OR_{K}, -SR_{K}, -C(O)OR_{K}, -O(O)NR_{K}, -S(O)₂R_{K}, -O(C=O)R_{K}, -N(R_{K})(C=O)(R_{K}), -C(O)R_{K}, , -C(O)OR_{K}, -CON(R_{K})₂, -OCO₂R_{K}, or an aliphatic, heteroaliphatic, aryl, heteroaryl, alkylaryl, or alkylheteroaryl moiety, wherein each occurrence of R_{K} is independently hydrogen, a protecting group or an aliphatic, heteroaliphatic, aryl, heteroaryl, alkylaryl, or alkylheteroaryl moiety, or when A and B together represent -CHR₅-CHR₆-, R₅ and R₆ taken together represent a substituted or unsubstituted 3-7 membered aliphatic, heteroaliphatic, aryl or heteroaryl ring,
G and J together represent -CHR₁₀-CHR₁₁-, -CR₁₀=CR₁₁-, wherein R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein said method comprises cyclizing the intermediate (VI): 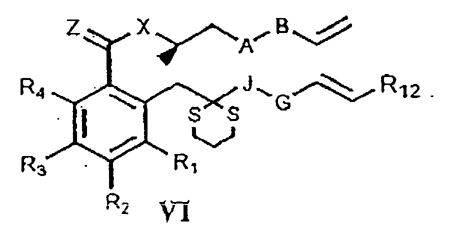 wherein R₁₂ is hydrogen or lower alkyl, in the presence of an olefin metathesis catalyst to generate the compound (VII).

22. The method of claim 21, wherein the method further comprises further diversifying the compound (VII) to generate a compound having the structure (I) as defined herein.

23. The method of claim 21, wherein the step of olefin metathesis is performed using an olefin metathesis catalyst, or wherein the step of olefin metathesis is performed using a ruthenium-based olefin metathesis catalyst, or wherein the step of olefin metathesis is performed using Ru(1,3-dimesityl-4,5-dihydro-imidazol-2-ylidene)(=CHCH=C(CH₃)₂)PCp₃Cl₂.

24. The compound of claim 1 having the structure:-

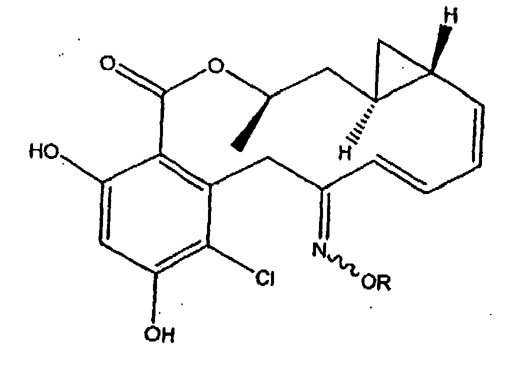

25. The compound of claim 1 having the structure:- 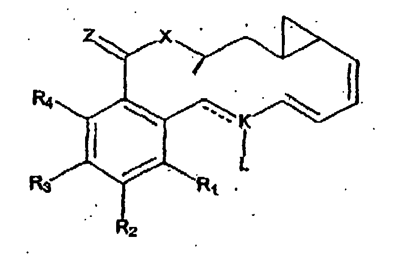 wherein Z is O and X is O or NH;
R₁ is hydrogen, halogen, lower alkyl, lower heteroalky, loweralkylaryl, loweralkylheteraaryl, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or lower alkyl, lower beteroalkyl, loweralkylaryl, loweralkylheteroaryl moiety; wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, loweralkyl, lowerheteroaryl, loweralkylaryl, loweralkylheteroaryl, or -N(R_{c})₂, wherein each occurrence of R_{c} is independently hydrogen, a protecting group, or an aliphatic, heteroaliphatic, aryl, heteroaryl, alkylaryl, or alkylheteroaryl moiety;
R₄ is hydrogen, halogen, -OR_{D}, -N(R_{D})₂. -SR_{D}, -O(C=O)R_{D}, -N(R_{D})(C=O)(R_{D}), -C(O)R_{D}, -C(O)OR_{D}, -CON(R_{D})₂, -OCO₂R_{D}, or loweralkyl, lowerheteroalkyl, loweralkylaryl, loweralkylheteroaryl, wherein each occurrence of R_{D} is independently hydrogen, a protecting group or an aliphatic, heteroaliphatic, aryl, heteroaryl, alkylaryl, or alkylheteroaryl moiety; and
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, R₅, R₆, R_{J}, or R_{L} are optionally a linker covalently bonded to a compound selected from the group consisting of radicicol, monocillin, analogues of radicicol and monocillin, geledanamycin, analogues of geldanamycin, and steroids.

26. The compound of claim 1 having the structure:- 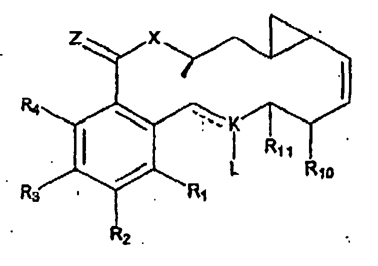 wherein Z is O and X is O or NH;
R₁ is hydrogen, halogen, lower alkyl, lower heteroalky, loweralkylaryl, loweralkylheteroaryl, or N(R_{A})₂, wherein each occurrence of R_{A} is independently hydrogen, a protecting group, or an aliphatic, heteroaliphatic, aryl, heteroaryl, alkylaryl, or alkylheteroaryl moiety;
R₂ is hydrogen, halogen. -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, or lower alkyl, lower heteroalkyl, loweralkylaryl, loweralkylheteroaryl moiety, wherein each occurrence of R_{B} is independently hydrogen, a protecting group or an aliphatic, heteroaliphatic, aryl, heteroaryl, alkylaryl, or alkylheteroaryl moiety;
R₃ is hydrogen, halogen, loweralkyl, lowerheteroaryl, loweralkylaryl, loweralkylheteroaryl, or -N(R_{c})₂, wherein each occurrence of R_{c} is independently hydrogen, a protecting group, or an aliphatic, heteroaliphatic, aryl, heteroaryl, alkylaryl, or alkylheteroaryl moiety;
R₄ is hydrogen, halogen, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, or loweralkyl, lowerheteroalkyl, loweralkylaryl, loweralkylheteroaryl, wherein each occurrence of R_{D} is independently hydrogen, a protecting group or an aliphatic, heteroaliphatic, aryl, heteroaryl, alkylaryl, or alkylheteroaryl moiety;
R₁₀ and R₁₁ are each independently hydrogen or lower alkyl;
K and L together represent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, or, if the dotted line --- represents a bond, whereby a double bond is present, then K and L together represent C-N(R_{L})₂, wherein each occurrence of R_{L} is independently hydrogen, a protecting group, an aliphatic, heteroaliphatic, aryl, heteroaryl, alkylaryl, or alkylheteroaryl moiety, or two occurrences of R_{L} taken together represent a 3 to 7-membered cyclic aliphatic, heteroaliphatic, aromatic or heteroaromatic moiety;
whereby each of the foregoing aliphatic and heteroaliphatic moieties may independently be substituted or unsubstituted, cyclic or acyclic, or branched or unbranched, and each aryl, heteroaryl, alkylaryl, and alkylheteroaryl moiety may be substituted or unsubstituted;
wherein one or any two of R₁, R_{A}, R₂, R_{B}, R₃, R_{C}, R₄, R_{D}, or R_{L} are optionally a linker covalently bonded to a compound selected from the group consisting of radicicol, monocillin, analogues of radicicol and monocillin, geledanamycin, analogues of geldanamycin, and steroids.

27. The compound of claim 1, having the structure:-

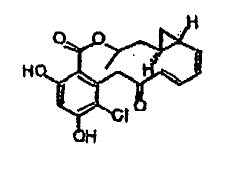

28. The compound of claim 1, having the structure:-

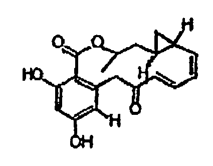

29. The compound of claim 1, having the structure :-

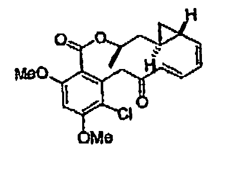

## Patentansprüche

1. Verbindung mit folgender Struktur: 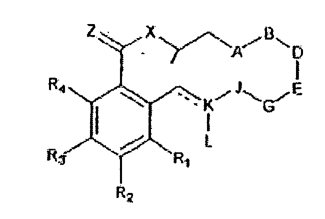 wobei die punktierte Linie --- eine Bindung darstellt, wenn eine Doppelbindung vorhanden ist, oder die punktierte Linie --- ist abwesend, wenn eine Einzelbindung vorhanden ist;
R₁ ist Wasserstoff, Halogen, ein aliphatischer, heteroaliphatischer, Aryl, Heteroaryl, Alkylaryl, oder Alkylheteroaryl-Anteil, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, Cyan, -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder ein aliphatischer, heteroaliphatischer, Aryl, Heteroaryl, Alkylaryl, oder Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, ein aliphatischer, heteroaliphatischer, Aryl, Heteroaryl, Alkylaryl, oder Alkylheteroaryl-Anteil, oder -N(R_{c})₂, wobei jedes Auftreten von R_{c} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, Cyan, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder ein aliphatischer, heteroaliphatischer, Aryl, Heteroaryl, Alkylaryl, oder Alkylheteroaryl-Anteil, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
Z ist O, S, oder NR_{E}, wobei R_{E} Wasserstoff, eine Schutzgruppe, ein aliphatischer, heteroaliphatischer, Aryl, Heteroaryl, Alkylaryl, oder Alkylheteroaryl-Anteil ist, oder OR_{F}, wobei R_{F} Wasserstoff, eine Schutzgruppe, ein aliphatischer, heteroaliphatischer; Aryl, Heteroaryl, Alkylaryl, oder Alkylheteroaryl-Anteil ist;
X ist O, S oder NP_{G}, wobei R_{G} Wasserstoff oder ein niederes Alkyl ist;
A und B stellen zusammen dar: 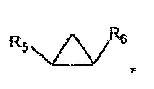 wobei R₅ und R₆ jeweils unabhängig Wasserstoff, Halogen, Cyan, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(Rⱼ)₂, -OCO₂R_{J}, -OS(=O)OR_{J} oder ein aliphatischer, heteroaliphatischer, Aryl, Heteroaryl, Alkylaryl, oder Alkylheteroaryl-Anteil ist, wobei jedes Auftreten von R_{J} unabhängig Wasserstoff, eine Schutzgruppe, oder ein aliphatischer, heteroaliphatischer, Aryl, Heteroaryl, Alkylaryl, oder Alkylheteroaryl-Anteil ist, und wobei R7 Wasserstoff, eine Schutzgruppe, -OR_{K}, -SR_{K}, -C(O)OR_{K}, -O(O)NR_{K}, -S(O)₂R_{K}, -O(C=O)R_{K}, -NR_{K}(C=O)(R_{K}), -C(O)R_{K}, -C(O)OR_{K}, -CON(R_{K})₂, -OCO₂R_{K}, oder ein aliphatischer, heteroaliphatischer, Aryl, Heteroaryl, Alkylaryl, oder Alkylheteroaryl-Anteil ist, wobei jedes Auftreten von R_{K} unabhängig Wasserstoff, eine Schutzgruppe, oder ein aliphatischer, heteroaliphatischer, Aryl, Heteroaryl, Alkylaryl, oder Alkylheteroaryl-Anteil ist, oder A und B zusammen -CHR₅-CHR₆ darstellen, R₅ und R₆ zusammengefasst stellen einen substituierten oder unsubstituierten 3-7 gliedrigen aliphatischen, heteroaliphatischen, Aryl oder Heteroaryl-Ring dar,
D und E stellen zusammen -CHR₈-CHR₉-, -CR₈=CR₉- dar, wobei R₈ und R₉ jeweils unabhängig Wasserstoff oder ein niederes Alkyl sind;
G und J stellen zusammen -CHR₁₀-CHR₁₁-, -CR₁₀=CR₁₁- dar, wobei R₁₀ und R₁₁ jeweils unabhängig Wasserstoff oder ein niederes Alkyl sind;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3 - 7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D}, R₅, R₆, R_{J} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist; und pharmazeutisch akzeptable Derivate davon;

2. Verbindung nach Anspruch 1, wobei ein oder zwei beliebige aus R₁, R_{A}, R₂, R_{B}, R₃, R_{C}, R₄, R_{D}, R₅, R₆, R_{J} oder R_{L} ein Linker ist/sind, der kovalent an eine Verbindung ausgewählt aus der Gruppe bestehend aus Radicicol, Monocillin, Analoga von Radicicol und Monocillin, Geldanamycin, Analoga von Geldanamycin, und Steroide gebunden ist, wobei der Linker ein aliphatischer oder heteroaliphatischer Anteil ist, wobei der aliphatische oder heteroaliphatische Anteil substituiert oder unsubstituiert, verzweigt oder unverzweigt, zyklisch oder azyklisch ist.

3. Verbindung nach Anspruch 1, wobei ein oder zwei beliebige aus R₁, R_{A}, R₂, R_{B}, R₃, R_{C}, R₄, R_{D}, R₅, R₆, R_{J} oder R_{L} ein Linker ist/sind, der kovalent an eine Verbindung ausgewählt aus der Gruppe bestehend aus Radicicol, Monocillin, Analoga von Radicicol und Monocillin, Geldanamycin, Analoga von Geldanamycin, und Steroide gebunden ist, wobei der Linker ein Anteil mit einer der folgenden Strukturen ist: -(CH₂)ₙ-, CH=CH-(CH₂)ₘ-, -(CH₂)ₚ-C≡C-(CH₂)_{q}-, oder -CH₂(CH₂)ₛCH₂-, wobei jedes Auftreten von n, m, p, q, und s unabhängig eine ganze Zahl von 0 bis 10 ist, und wobei eines oder mehrere der Wasserstoffatome gegebenenfalls durch Alkyl, Heteroalkyl, Aryl, Heteroaryl, Alkylaryl oder Alkylheteroaryl-Anteil oder einem sekundären oder tertiären Amin, Hydroxyl, oder Thiol ersetzt ist.

4. Verbindung nach Anspruch 1, wobei R₁ und R₃ jeweils unabhängig Halogen, Wasserstoff, oder ein niederes Alkyl sind; R₂ ist Wasserstoff oder -OR_{B}, wobei jedes Auftreten von R_{B} unabhängig Wasserstoff, eine Schutzgruppe, oder ein aliphatischer, heteroaliphatischer, Aryl, Heteroaryl, Alkylaryl, oder Alkylheteroaryl-Anteil ist, und R₄ ist Wasserstoff oder -OR_{D}, wobei jedes Auftreten von R_{D} unabhängig Wasserstoff, eine Schutzgruppe, oder ein aliphatischer, heteroaliphatischer, Aryl, Heteroaryl, Alkylaryl oder Alkylheteroaryl-Anteil ist.

5. Verbindung nach Anspruch 1, wobei die Verbindung folgende Struktur hat: 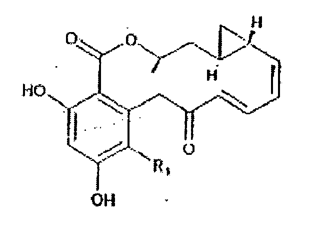 wobei R₁ Wasserstoff oder Cl ist.

6. Verbindung mit folgender Struktur 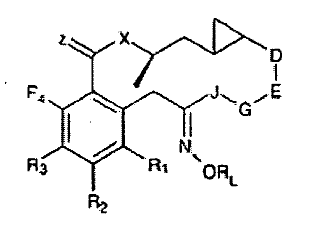 wobei
R₁ Halogenid ist, R₂ ist OR_{B}, R₃ ist Wasserstoff und R₄ ist OR_{D}, wobei R_{B} und R_{D} Wasserstoff sind.
Z ist O,
X ist O,
C und E sind -CR₈-CR₉-, wobei R₈ und R₉ Wasserstoff sind.
G und J sind -CR₁₀-CR₁₁-, wobei R₁₀ und R₁₁ Wasserstoff sind.
R_{L} ist Wasserstoff, eine Schutzgruppe, ein aliphatischer, heteroaliphatischer, Aryl, Heteroaryl, Arylalkyl oder Heteroarylalkyl-Anteil.

7. Pharmazeutische Zusammensetzung, die eine Verbindung nach Anspruch 1 und einen pharmazeutisch akzeptablen Träger aufweist.

8. Pharmazeutische Zusammensetzung nach Anspruch 7, welche weiters einen oder mehrere zusätzliche(n) therapeutische(n) Wirkstoff(e) aufweist, wobei der eine oder die mehreren zusätzliche(n) Wirkstoff(e) vorzugsweise ein Antikrebs-Wirkstoff aufweist/aufweisen.

9. Verbindung nach Anspruch 1 für die Verwendung in der Behandlung von Krebs in einem Lebewesen, das die Behandlung benötigt.

10. Verbindung nach Anspruch 9, wobei die Verbindung in einer Menge im Bereich von 0,001 mg/kg bis 100mg/kg Körpergewicht, vorzugsweise im Bereich von 0,01 mg/kg bis etwa 25mg/kg Körpergewicht bereitgestellt wird.

11. Verbindung nach Anspruch 10 für die Verwendung in der Inhibierung des Wachstums oder zum Abtöten von Krebszellen.

12. Verbindung für die Behandlung von Krebs, wobei die Krebszellen Rb negative Krebszellen aufweisen, wobei die Verbindung folgende Struktur aufweist: 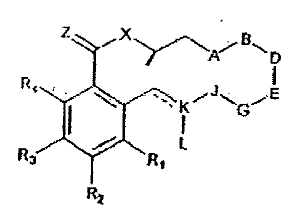 wobei die punktierte Linie --- eine Bindung darstellt, wenn eine Doppelbindung vorhanden ist, oder die punktierte Linie --- ist abwesend, wenn eine Einzelbindung vorhanden ist.
R₁ ist Wasserstoff, Halogen, ein aliphatischer, heteroaliphatischer, Aryl, Heteroaryl, Alkylaryl, oder Alkylheteroaryl-Anteil, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, Cyan, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B} oder ein aliphatischer, heteroaliphatischer, Aryl, Heteroaryl, Alkylaryl, oder Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, ein aliphatischer, heteroaliphatischer, Aryl, Heteroaryl, Alkylaryl, oder Alkylheteroaryl-Anteil, oder -N(R_{C})₂, wobei jedes Auftreten von R_{C} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, Cyan, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder ein aliphatischer, heteroaliphatischer, Aryl, Heteroaryl, Alkylaryl, oder Alkylheteroaryl-Anteil, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
Z ist O, S, oder NR_{E}, wobei R_{E} Wasserstoff, eine Schutzgruppe, ein aliphatischer, heteroaliphatischer, Aryl, Heteroaryl, Alkylaryl, oder Alkylheteroaryl-Anteil ist, oder OR_{F}, wobei R_{F} Wasserstoff, eine Schutzgruppe, ein aliphatischer, heteroaliphatischer, Aryl, Heteroaryl, Alkylaryl, oder Alkylheteroaryl-Anteil ist;
X ist O, S oder NR_{G}, wobei R_{G} Wasserstoff oder ein niederes Alkyl ist;
A und B stellen zusammen dar: 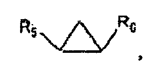 wobei R₅ und R₆ jeweils unabhängig Wasserstoff, Halogen, Cyan, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} oder ein aliphatischer, heteroaliphatischer, Aryl, Heteroaryl, Alkylaryl, oder Alkylheteroaryl-Anteil ist, wobei jedes Auftreten von R_{J} unabhängig Wasserstoff, eine Schutzgruppe, oder ein aliphatischer, heteroaliphatischer, Aryl, Heteroaryl, Alkylaryl, oder Alkylheteroaryl-Anteil ist, und wobei R₇ Wasserstoff, eine Schutzgruppe, -OR_{K}, -SR_{K}, -C(O)OR_{K}, -O(O)NR_{K}, -S(O)₂R_{K}, -O(C=O)R_{K}, -NR_{K}(C=O)(R_{K}), -C(O)R_{K}, -C(O)OR_{K}, -CON(R_{K})₂, -OCO₂R_{K}, oder ein aliphatischer, heteroaliphatischer, Aryl, Heteroaryl, Alkylaryl, oder Alkylheteroaryl-Anteil ist, wobei jedes Auftreten von R_{K} unabhängig Wasserstoff, eine Schutzgruppe, oder ein aliphatischer, heteroaliphatischer, Aryl, Heteroaryl, Alkylaryl, oder Alkylheteroaryl-Anteil ist, oder A und B zusammen -CHR₅-CHR₆ darstellen, R₅ und R₆ zusammengefasst stellen einen substituierten oder unsubstituierten 3-7 gliedrigen aliphatischen, heteroaliphatischen, Aryl oder Heteroaryl-Ring dar,
D und E stellen zusammen -CHR₈-CHR₉-, -CR₈=CR₉- dar, wobei R₈ und R₉ jeweils unabhängig Wasserstoff oder ein niederes Alkyl sind;
G und J stellen zusammen -CHR₁₀-CHR₁₁-, -CR₁₀=CR₁₁- dar, wobei R₁₀ und R₁₁ jeweils unabhängig Wasserstoff oder ein niederes Alkyl sind;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-CR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3 - 7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D}, R₅, R₆, R_{J} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist; und pharmazeutisch akzeptable Derivate davon.

13. Verbindung nach Anspruch 12, wobei die Verbindung in einer Menge im Bereich von 0,001 mg/kg bis 50mg/kg Körpergewicht, vorzugsweise im Bereich von 0,01 mg/kg bis etwa 25mg/kg Körpergewicht bereitgestellt wird.

14. Verbindung nach Anspruch 12, wobei der Krebs, welcher Rb negative Zellen aufweist, ein kleinzelliges Bronchialkarzinom, Glioblastom oder Retinoblastom ist.

15. Verbindung für die Verwendung in der Inhibierung des Wachstums oder zum Abtöten von Rb negativen Krebszellen, wobei die Verbindung folgende Struktur aufweist: 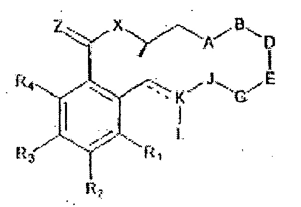 wobei die punktierte Linie --- eine Bindung darstellt, wenn eine Doppelbindung vorhanden ist, oder die punktierte Linie --- ist abwesend, wenn eine Einzelbindung vorhanden ist.
R₁ ist Wasserstoff, Halogen, ein aliphatischer, heteroaliphatischer, Aryl, Heteroaryl, Alkylaryl, oder Alkylheteroaryl-Anteil, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, Cyan, -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder ein aliphatischer, heteroaliphatischer, Aryl, Heteroaryl, Alkylaryl, oder Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, ein aliphatischer, heteroaliphatischer, Aryl, Heteroaryl, Alkylaryl, oder Alkylheteroaryl-Anteil, oder -N(R_{c})₂, wobei jedes Auftreten von R_{c} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, Cyan, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder ein aliphatischer, heteroaliphatischer, Aryl, Heteroaryl, Alkylaryl, oder Alkylheteroaryl-Anteil, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
Z ist O, S, oder NR_{E}, wobei R_{E} Wasserstoff, eine Schutzgruppe, ein aliphatischer, heteroaliphatischer, Aryl, Heteroaryl, Alkylaryl, oder Alkylheteroaryl-Anteil ist, oder OR_{F}, wobei R_{F} Wasserstoff, eine Schutzgruppe, ein aliphatischer, heteroaliphatischer, Aryl, Heteroaryl, Alkylaryl, oder Alkylheteroaryl-Anteil ist;
X ist O, S oder NR_{G}, wobei R_{G} Wasserstoff oder ein niederes Alkyl ist;
A und B stellen zusammen dar: 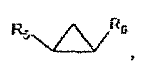 wobei R₅ und R₆ jeweils unabhängig Wasserstoff, Halogen, Cyan, -OR_{J}, -N(R_{J})2, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} oder ein aliphatischer, heteroaliphatischer, Aryl, Heteroaryl, Alkylaryl, oder Alkylheteroaryl-Anteil ist, wobei jedes Auftreten von R_{J} unabhängig Wasserstoff, eine Schutzgruppe, oder ein aliphatischer, heteroaliphatischer, Aryl, Heteroaryl, Alkylaryl, oder Alkylheteroaryl-Anteil ist, und wobei R₇ Wasserstoff, eine Schutzgruppe, -OR_{K}, -SR_{K}, -C(O)OR_{K}, -O(O)NR_{K}, -S(O)₂R_{K}, -O(C=O)R_{K}, -NR_{K}(C=O)(R_{K}), -C(O)R_{K}, -C(O)OR_{K}, -CON(R_{K})₂, -OCO₂R_{K}, oder ein aliphatischer, heteroaliphatischer, Aryl, Heteroaryl, Alkylaryl, oder Alkylheteroaryl-Anteil ist, wobei jedes Auftreten von R_{K} unabhängig Wasserstoff, eine Schutzgruppe, oder ein aliphatischer, heteroaliphatischer, Aryl, Heteroaryl, Alkylaryl, oder Alkylheteroaryl-Anteil ist, oder A und B zusammen -CHR₅-CHR₆ darstellen, R₅ und R₆ zusammengefasst stellen einen substituierten oder unsubstituierten 3-7 gliedrigen aliphatischen, heteroaliphatischen, Aryl oder Heteroaryl-Ring dar,
D und E stellen zusammen -CHR₈-CHR₉-, -CR₈=CR₉- dar, wobei R₈ und R₉ jeweils unabhängig Wasserstoff oder ein niederes Alkyl sind;
G und J stellen zusammen -CHR₁₀-CHR₁₁-, -CR₁₀=CR₁₁- dar, wobei R₁₀ und R₁₁ jeweils unabhängig Wasserstoff oder ein niederes Alkyl sind;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3-7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D}, R₅, R₆, R_{J} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist; und pharmazeutisch akzeptable Derivate davon;

16. Verbindung nach Anspruch 10, wobei die Rb negativen Krebszellen ein kleinzelliges Bronchialkarzinom, Glioblastom oder Retinoblastom sind.

17. Verwendung der Verbindung der Formel I in der Herstellung eines Medikamentes zum Behandeln von Krebs.

18. Verfahren für die Synthese einer Verbindung mit der Struktur (I): 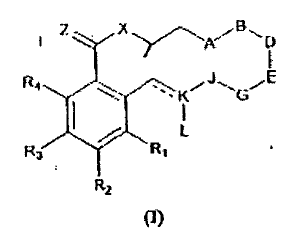 wobei die punktierte Linie --- eine Bindung darstellt, wenn eine Doppelbindung vorhanden ist, oder die punktierte Linie --- ist abwesend, wenn eine Einzelbindung vorhanden ist.
R₁ ist Wasserstoff, Halogen, ein aliphatischer, heteroaliphatischer, Aryl, Heteroaryl, Alkylaryl, oder Alkylheteroaryl-Anteil, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R2 ist Wasserstoff, Halogen, Cyan, -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder ein aliphatischer, heteroaliphatischer, Aryl, Heteroaryl, Alkylaryl, oder Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, ein aliphatischer, heteroaliphatischer, Aryl, Heteroaryl, Alkylaryl, oder Alkylheteroaryl-Anteil, oder -N(R_{C})₂, wobei jedes Auftreten von R_{C} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, Cyan, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder ein aliphatischer, heteroaliphatischer, Aryl, Heteroaryl, Alkylaryl, oder Alkylheteroaryl-Anteil, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
Z ist O, S, oder NR_{E}, wobei R_{E} Wasserstoff, eine Schutzgruppe, ein aliphatischer, heteroaliphatischer, Aryl, Heteroaryl, Alkylaryl, oder Alkylheteroaryl-Anteil ist, oder OR_{F}, wobei R_{F} Wasserstoff, eine Schutzgruppe, ein aliphatischer, heteroaliphatischer, Aryl, Heteroaryl, Alkylaryl, oder Alkylheteroaryl-Anteil ist;
X ist O, S oder NR_{G}, wobei R_{G} Wasserstoff oder ein niederes Alkyl ist;
A und B stellen zusammen dar: 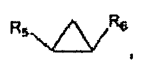 wobei R₅ und R₆ jeweils unabhängig Wasserstoff, Halogen, Cyan, -OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} oder ein aliphatischer, heteroaliphatischer, Aryl, Heteroaryl, Alkylaryl, oder Alkylheteroaryl-Anteil, wobei jedes Auftreten von R_{J} unabhängig Wasserstoff, eine Schutzgruppe, oder ein aliphatischer, heteroaliphatischer, Aryl, Heteroaryl, Alkylaryl, oder Alkylheteroaryl-Anteil ist, und wobei R₇ Wasserstoff, eine Schutzgruppe, -OR_{K}, -SR_{K}, -C(O)OR_{K}, -O(O)NR_{K}, -S(O)₂R_{K}, -O(C=O)R_{K}, -NR_{K}(C=O)(R_{K}), -C(O)R_{K}, -C(O)OR_{K}, -CON(R_{K})₂, -OCO₂R_{K}, oder ein aliphatischer, heteroaliphatischer, Aryl, Heteroaryl, Alkylaryl, oder Alkylheteroaryl-Anteil ist, wobei jedes Auftreten von R_{K} unabhängig Wasserstoff, eine Schutzgruppe, oder ein aliphatischer, heteroaliphatischer, Aryl, Heteroaryl, Alkylaryl, oder Alkylheteroaryl-Anteil ist, oder A und B zusammen -CHR₅-CHR₆ darstellen, R₅ und R₆ zusammengefasst stellen einen substituierten oder unsubstituierten 3-7 gliedrigen aliphatischen, heteroaliphatischen, Aryl oder Heteroaryl-Ring dar,
D und E stellen zusammen -CHR₈-CHR₉-, -CR₈=CR₉- dar, wobei R₈ und R₉ jeweils unabhängig Wasserstoff oder ein niederes Alkyl sind;
G und J stellen zusammen -CHR₁₀-CHR₁₁-, -CR₁₀=CR₁₁- dar, wobei R₁₀ und R₁₁ jeweils unabhängig Wasserstoff oder ein niederes Alkyl sind;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3-7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D}, R₅, R₆, R_{J} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist;
wobei das Verfahren aufweist:
1. Bereitstellen einer Benzoesäure-Komponente mit folgender Struktur: 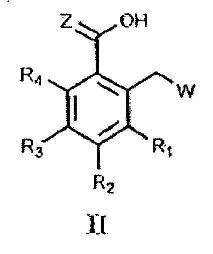 wobei R₁-R₄ und Z wie oben definiert sind, und wobei W Halogen ist:
und
reagieren der Benzoesäure-Komponente mit einer chiralen Komponente (IV) mit folgender Struktur: 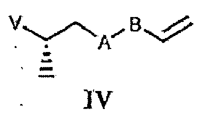 wobei A und B wie oben definiert sind, und wobei V NHR_{G} ist, wobei R_{G} ist Wasserstoff oder niederes Alkyl; SH; oder OH in der Gegenwart von einem Veresterungsreagens um ein Zwischenprodukt (V) mit folgender Struktur zu erzeugen: 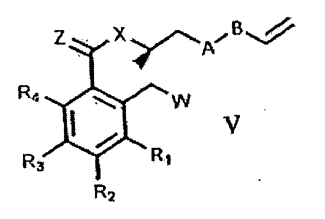
2. Reagieren des Zwischenprodukts (V) mit einem Dithian mit folgender Struktur (III): 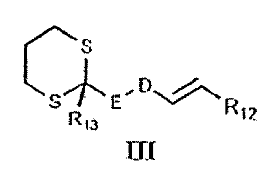 wobei R₁₃ Wasserstoff oder ein Alkalimetallsalz ist und wobei R₁₂ Wasserstoff oder ein niederes Alkyl ist unter Bedingungen um das Dithian hinzuzufügen um ein Zwischenprodukt (VI) mit folgender Struktur zu erzeugen: 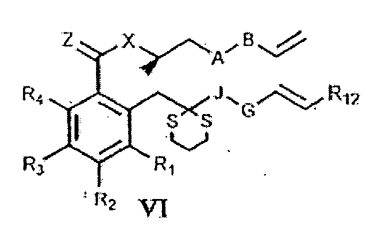
3. Wenn einer oder mehrere von R₁-R₄ eine ungeschützte Thio, Amino oder Hydroxylgruppe ist, dann gegebenenfalls schützen dieser ungeschützten Gruppe;
4. Zyklisierung der Verbindung in der Gegenwart eines Olefinmetathese-Katalysators um die Verbindung (VII) zu erzeugen: 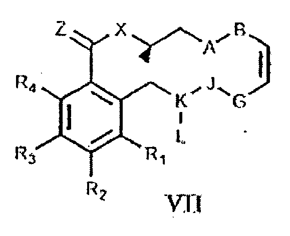
5. Gegebenenfalls weiter reagieren (VII) mit einem oder mehreren Reagenzien zum Diversifizieren und gegebenenfalls Entschützen des Makroliden um die Verbindung (I) zu erzeugen.

19. Verfahren nach Anspruch 18, wobei der Veresterungsschritt mittels Diethylazodicarboxylat (DIAD) in der Gegenwart von Triphenylphosphin oder Trifurylphosphin durchgeführt wird.

20. Verfahren nach Anspruch 18, wobei der Olefinmetatheseschritt mittels eines Olefinmetathese-Katalysators durchgeführt wird, oder wobei der Olefinmetatheseschritt mittels eines Olefinmetathese-Katalysators auf Rutheniumbasis durchgeführt wird, oder wobei der Olefinmetatheseschritt mittels Ru(1,3-dimesityl-4,5-dihydro-imidazol-2-ylidene)(=CHCH=C(CH₃)₂)PCp₃Cl₂ durchgeführt wird.

21. Verfahren zur Synthese einer makrozyklischen Verbindung mit folgender Struktur (VII): 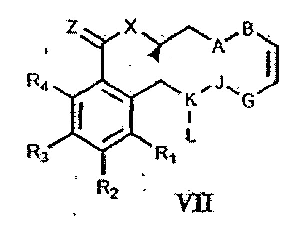 wobei die punktierte Linie --- eine Bindung darstellt, wenn eine Doppelbindung vorhanden ist, oder die punktierte Linie --- ist abwesend, wenn eine Einzelbindung vorhanden ist.
R₁ ist Wasserstoff, Halogen, ein aliphatischer, heteroaliphatischer, Aryl, Heteroaryl, Alkylaryl, oder Alkylheteroaryl-Anteil, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, Cyan, -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder ein aliphatischer, heteroaliphatischer, Aryl, Heteroaryl, Alkylaryl, oder Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, ein aliphatischer, heteroaliphatischer, Aryl, Heteroaryl, Alkylaryl, oder Alkylheteroaryl-Anteil, oder -N(R_{C})₂, wobei jedes Auftreten von R_{C} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, Cyan, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder ein aliphatischer, heteroaliphatischer, Aryl, Heteroaryl, Alkylaryl, oder Alkylheteroaryl-Anteil, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
Z ist O, S, oder NR_{E}, wobei R_{E} Wasserstoff, eine Schutzgruppe, ein aliphatischer, heteroaliphatischer, Aryl, Heteroaryl, Alkylaryl, oder Alkyheteroaryl-Anteil, oder OR_{F}, wobei R_{F} Wasserstoff, eine Schutzgruppe, ein aliphatischer, heteroaliphatischer, Aryl, Heteroaryl, Alkylaryl, oder Alkylheteroaryl-Anteil ist;
X ist O, S oder NR_{G}, wobei R_{G} Wasserstoff oder ein niederes Alkyl ist;
A und B stellen zusammen dar: 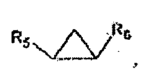 wobei R₅ und R₆ jeweils unabhängig Wasserstoff, Halogen, Cyan, -OR_{J}, -N(R_{J})2, -SR_{J}, -O(C=O)R_{J}, -O(S=O)R_{J}, -N(R_{J})(C=O)(R_{J}), -C(=O)R_{J}. -C(=O)OR_{J}, -CON(R_{J})2, -OCO₂R_{J}, -OS(=O)OR_{J} oder ein aliphatischer, heteroaliphatischer, Aryl, Heteroaryl, Alkylaryl, oder Alkylheteroaryl-Anteil ist, wobei jedes Auftreten von R_{J} unabhängig Wasserstoff, eine Schutzgruppe, oder ein aliphatischer, heteroaliphatischer, Aryl, Heteroaryl, Alkylaryl, oder Alkylheteroaryl-Anteil ist, und wobei R₇ Wasserstoff, eine Schutzgruppe, -OR_{K}, -SR_{K}, -C(O)OR_{K}, -O(O)NR_{K}, -S(O)₂R_{K}, -O(C=O)R_{K}, -NR_{K}(C=O)(R_{K}), -C(O)R_{K}, -C(O)OR_{K}, -CON(R_{K})₂, -OCO₂R_{K}, oder ein aliphatischer, heteroaliphatischer, Aryl, Heteroaryl, Alkylaryl, oder Alkylheteroaryl-Anteil ist, wobei jedes Auftreten von R_{K} unabhängig Wasserstoff, eine Schutzgruppe, oder ein aliphatischer, heteroaliphatischer, Aryl, Heteroaryl, Alkylaryl, oder Alkylheteroaryl-Anteil ist, oder A und B zusammen -CHR₅-CHR₆ darstellen, R₅ und R₆ zusammengefasst stellen einen substituierten oder unsubstituierten 3-7 gliedrigen aliphatischen, heteroaliphatischen, Aryl oder Heteroaryl-Ring dar,
G und J stellen zusammen -CHR₁₀-CHR₁₁-, -CR₁₀=CR₁₁- dar, wobei R₁₀ und R₁₁ jeweils unabhängig Wasserstoff oder ein niederes Alkyl sind;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3 - 7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei das Verfahren die Zyklisierung des Zwischenprodukts (VI) aufweist: 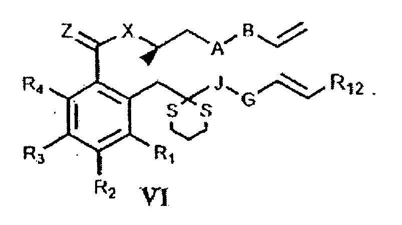 wobei R₁₂ Wasserstoff oder ein niederes Alkyl ist, in der Gegenwart eines Olefinmetathese-Katalysators um die Verbindung (VII) zu erzeugen.

22. Verfahren nach Anspruch 21, wobei das Verfahren weiters die Diversifizierung der Verbindung (VII) aufweist, um die Verbindung mit der Struktur (I), wie hierin definiert ist, herzustellen.

23. Verfahren nach Anspruch 21, wobei der Olefinmetatheseschritt mittels eines Olefinmetathese-Katalysators durchgeführt wird, oder wobei der Olefinmetatheseschritt mittels eines Olefinmetathese-Katalysators auf Rutheniumbasis durchgeführt wird, oder wobei der Olefinmetatheseschritt mittels Ru(1,3-dimesityl-4,5-dihydro-imidazol-2-ylidene)(=CHCH=C(CH₃)₂)PCₚ₃Cl₂ durchgeführt wird.

24. Verbindung nach Anspruch 1 mit folgender Struktur:

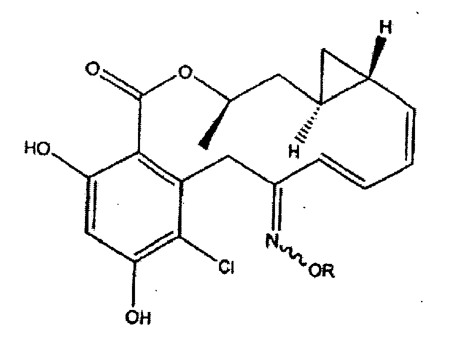

25. Verbindung nach Anspruch 1 mit folgender Struktur: 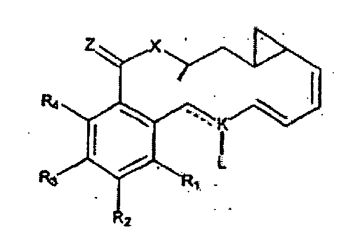 wobei Z ist O und X ist O oder NH;
R₁ ist Wasserstoff, Halogen, niederes Alkyl, niederes Heteroalkyl, niederes Alkylaryl, niederes Alkylheteroaryl, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, , -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder niederer Alkyl, niederer Heteroalkyl, niederer Alkylaryl, niederer Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, niederes Alkyl, niederes Heteroalkyl, niederes Alkylaryl, niederes Alkylheteroaryl, oder N(R_{C})₂, wobei jedes Auftreten von R_{C} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D} oder niederes Alkyl, niederes Heteroalkyl, niederes Alkylaryl, niederes Alkylheteroaryl, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist; und
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3 - 7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D}, R₅, R₆, R_{J} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist.

26. Verbindung nach Anspruch 1 mit folgender Struktur: 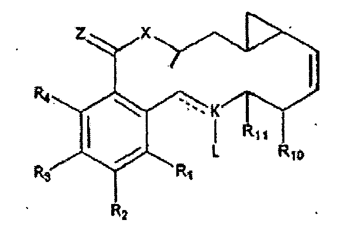 wobei Z ist O und X ist O oder NH;
R₁ ist Wasserstoff, Halogen, niederes Alkyl, niederes Heteroalkyl, niederes Alkylaryl, niederes Alkylheteroaryl, oder N(R_{A})₂, wobei jedes Auftreten von R_{A} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₂ ist Wasserstoff, Halogen, , -OR_{B}, -N(R_{B})₂, SR_{B}, -O(C=O)R_{B}, -N(R_{B})(C=O)(R_{B}), -C(O)R_{B}, -C(O)OR_{B}, -CON(R_{B})₂, -OCO₂R_{B} oder niederer Alkyl, niederer Heteroalkyl, niederer Alkylaryl, niederer Alkylheteroaryl-Anteil, wobei jedes Auftreten von R_{B} unabhängig Wasserstoff, eine Schutzgruppe oder ein aliphatischer, heteroaliphatischer, Aryl, Heteroaryl, Alkylaryl, oder Alkylheteroaryl-Anteil ist;
R₃ ist Wasserstoff, Halogen, niederes Alkyl, niederes Heteroaryl, niederes Alkylaryl, niederes Alkylheteroaryl, oder N(R_{c})₂, wobei jedes Auftreten von R_{C} unabhängig Wasserstoff, eine Schutzgruppe, oder ein aliphatischer, heteroaliphatischer, Aryl, Heteroaryl, Alkylaryl, oder Alkylheteroaryl-Anteil ist;
R₄ ist Wasserstoff, Halogen, -OR_{D}, -N(R_{D})₂, SR_{D}, -O(C=O)R_{D}, -N(R_{D})(C=O)(R_{D}), -C(O)R_{D}, -C(O)OR_{D}, -CON(R_{D})₂, -OCO₂R_{D} oder niederes Alkyl, niederes Heteroalkyl, niederes Alkylaryl, niederes Alkylheteroaryl, wobei jedes Auftreten von R_{D} unabhängig Wasserstoff, eine Schutzgruppe oder ein aliphatischer, heteroaliphatischer, Aryl, Heteroaryl, Alkylaryl, oder Alkylheteroaryl-Anteil ist;
R₁₀ und R₁₁ sind unabhängig Wasserstoff oder niederes Alkyl;
K und L stellen zusammen C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ dar, oder, wenn die punktierte Linie --- eine Bindung darstellt, wodurch eine Doppelbindung vorhanden ist, dann stellen K und L zusammen C-N(R_{L})₂ dar, wobei jedes Auftreten von R_{L} unabhängig Wasserstoff, eine Schutzgruppe, ein aliphatischer, heteroaliphatischer, Aryl, Heteroaryl, Alkylaryl, oder Alkylheteroaryl-Anteil ist, oder zwei Auftreten von R_{L} zusammengefasst stellen einen 3 - 7 gliedrigen zyklisch-aliphatischen, heteroaliphatischen, aromatischen oder heteroaromatischen Anteil dar;
wobei jeder der vorangehenden aliphatischen oder heteroaliphatischen Anteile unabhängig substituiert oder unsubstituiert, zyklisch oder azyklisch, oder verzweigt oder unverzweigt sein kann, und jeder Aryl, Heteroaryl, Alkylaryl, und Alkylheteroaryl-Anteil kann substituiert oder unsubstituiert sein;
wobei ein oder zwei beliebige aus R₁, R_{A}, R₂, R_{B}, R₃, R_{C}, R₄, R_{D} oder R_{L} gegebenenfalls ein Linker ist/sind, der kovalent an eine Verbindung ausgewählt aus der Gruppe bestehend aus Radicicol, Monocillin, Analoga von Radicicol und Monocillin, Geledanamycin, Analoga von Geldanamycin, und Steroide gebunden ist.

27. Verbindung nach Anspruch 1 mit folgender Struktur:

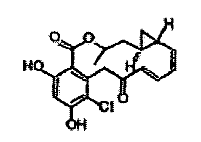

28. Verbindung nach Anspruch 1 mit folgender Struktur:

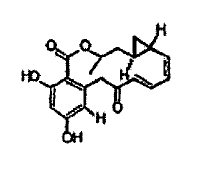

29. Verbindung nach Anspruch 1 mit folgender Struktur:

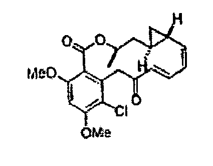

## Revendications

1. Composé ayant la structure : 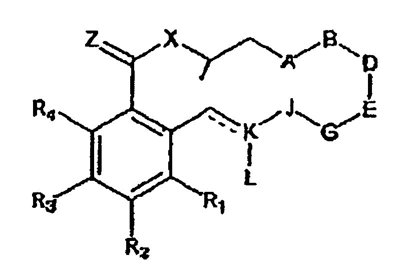 dans laquelle la ligne en traits interrompus --- représente une liaison, auquel cas une double liaison est présente, ou la ligne en traits interrompus --- est absente, auquel cas une simple liaison est présente;
R₁ est un hydrogène, un halogène, un groupement aliphatique, hétéroaliphatique, aryle, hétéroaryle, alkylaryle ou alkylhétéroaryle, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, un cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, ou un groupement aliphatique, hétéroaliphatique, aryle, hétéroaryle, alkylaryle ou alkylhétéroaryle, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un groupement aliphatique, hétéroaliphatique, aryle, hétéroaryle, alkylaryle ou alkylhétéroaryle, ou N(R_{c})₂, dans lequel chaque occurrence de R_{c} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, un cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un groupement aliphatique, hétéroaliphatique, aryle, hétéroaryle, alkylaryle ou alkylhétéroaryle, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
Z est O, S ou NR_{E}, dans lequel R_{E} est un hydrogène, un groupe protecteur, un groupement aliphatique, hétéroaliphatique, aryle, hétéroaryle, alkylaryle ou alkylhétéroaryle, ou OR_{F}, dans lequel R_{F} est un hydrogène, un groupe protecteur, un groupement aliphatique, hétéroaliphatique, aryle, hétéroaryle, alkylaryle ou alkylhétéroaryle;
X est O, S ou NR_{G}, dans lequel R_{G} est un hydrogène ou un alkyle inférieur;
A et B ensemble représentent 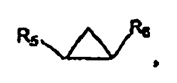 dans lequel R₅ et R₆ sont chacun indépendamment un hydrogène, un halogène, un cyano, -OR_{J}, -N(R_{J})₂, -SR_{J}, -O(C=O)R_{J}, -O(S=O)R_{J}, -N(R_{J}) (C=O) (R_{J}), -C(=O)R_{J}, -C(=O)OR_{J}, -CON(R_{J})₂, -OCO₂R_{J}, -OS(=O)OR_{J} ou un groupement aliphatique, hétéroaliphatique, aryle, hétéroaryle, alkylaryle ou alkylhétéroaryle, dans lequel chaque occurrence de R_{J} est indépendamment un hydrogène, un groupe protecteur ou un groupement aliphatique, hétéroaliphatique, aryle, hétéroaryle, alkylaryle ou alkylhétéroaryle, et dans lequel R₇ est un hydrogène, un groupe protecteur, -OR_{K}, -SR_{K}, C(O)OR_{K}, -O(O)NR_{K}, -S(O)₂R_{K}, -O(C=O)R_{K}, -N(R_{K})(C=O)(R_{K}), -C(O)R_{K}, C(O)OR_{K}, -CON(R_{K})₂, -OCO₂R_{K}, ou un groupement aliphatique, hétéroaliphatique, aryle, hétéroaryle, alkylaryle ou alkylhétéroaryle, dans lequel chaque occurrence de R_{K} est indépendamment un hydrogène, un groupe protecteur ou un groupement aliphatique, hétéroaliphatique, aryle, hétéroaryle, alkylaryle ou alkylhétéroaryle, ou quand A et B ensemble représentent -CHR₅-CHR₆-, R₅ et R₆ pris ensemble représentent un anneau aliphatique, hétéroaliphatique, aryle ou hétéroaryle à 3 à 7 chaînons, substitué ou non substitué;
D et E ensemble représentent -CHR₈-CHR₉-, -CR₈=CR₉-, dans lequel R₈ et R₉ sont chacun indépendamment un hydrogène ou un alkyle inférieur;
G et J ensemble représentent -CHR₁₀-CHR₁₁-, -CR₁₀=CR₁₁-, dans lequel R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D}, R₅, R₆, R_{J} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes; et
les dérivés pharmaceutiquement acceptables de ces composés.

2. Composé selon la revendication 1, dans lequel un ou deux quelconques de R₁, R_{A}, R₂, R_{B}, R₃, R_{C}, R₄, R_{D}, R₅, R₆, R_{J} ou R_{L} sont un lieur lié par covalence à un composé sélectionné dans le groupe composé de radicicol, monocilline, analogues du radicicol et de la monocilline, gélédanamycine, analogues de la gélédanamycine, et stéroïdes, dans lequel le lieur est un groupement aliphatique ou hétéroaliphatique, auquel cas ledit groupement aliphatique ou hétéroaliphatique est substitué ou non substitué, ramifié ou non ramifié, ou cyclique ou acyclique.

3. Composé selon la revendication 1, dans lequel un ou deux quelconques de R₁, R_{A}, R₂, R_{B}, R₃, R_{C}, R₄, R_{D}, R₅, R₆, R_{J} ou R_{L} sont un lieur lié par covalence à un composé sélectionné dans le groupe composé de radicicol, monocilline, analogues du radicicol et de la monocilline, gélédanamycine, analogues de la gélédanamycine, et stéroïdes, dans lequel le lieur est un groupement ayant l'une des structures -(CH₂)ₙ-CH=CH-(CH₂)ₘ-, -(CH₂)ₚ-C≡C-(CH₂)_{q}-, ou -CH₂(CH₂)_{S}-CH₂-, dans lequel chaque occurrence de n, m, p, q et s est indépendamment un entier de 0 à 10 et dans lequel un ou plusieurs des atomes d'hydrogène sont facultativement remplacés par un groupement alkyle, hétéroalkyle, aryle, hétéroaryle, alkylaryle ou alkylhétéroaryle ou une amine secondaire ou tertiaire, un hydroxyle ou un thiol.

4. Composé selon la revendication 1, dans lequel R₁ et R₃ sont chacun indépendamment un halogène, un hydrogène ou un alkyle inférieur; R₂ est un hydrogène ou -OR_{B}, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle, et R₄ est un hydrogène ou -OR_{D}, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle.

5. Composé selon la revendication 1, dans lequel le composé a la structure: 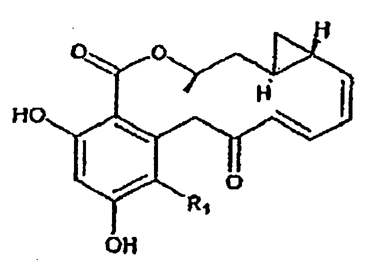 dans laquelle R₁ est un hydrogène ou C1.

6. Composé ayant la structure : 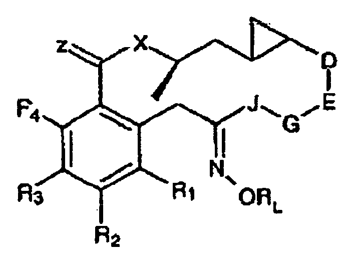 dans laquelle
R₁ est un halogénure, R₂ est OR_{B}, R₃ est un hydrogène et R₄ est OR_{D}, dans lequel R_{B} et R_{D} sont un hydrogène.
Z est O,
X est O,
C et E sont -CR₈-CR₉-, dans lequel R₈ et R₉ sont un hydrogène,
G et J sont -CR₁₀-CR₁₁-, dans lequel R₁₀ et R₁₁ sont un hydrogène,
R_{L} est un hydrogène, un groupe protecteur, un groupement aliphatique, hétéroaliphatique, aryle, hétéroaryle, arylalkyle ou hétéroarylalkyle.

7. Composition pharmaceutique comprenant un composé selon la revendication 1 et un excipient pharmaceutiquement acceptable.

8. Composition pharmaceutique selon la revendication 7, comprenant en outre un ou plusieurs agents thérapeutiques additionnels, dans laquelle le ou les agents thérapeutiques additionnels comprend de préférence un agent anticancéreux.

9. Composé selon la revendication 1, pour usage dans le traitement du cancer chez un sujet qui le nécessite.

10. Composé selon la revendication 9, dans lequel le composé est présent en une quantité allant dans la plage de 0,001 mg/kg à 100 mg/kg de poids corporel, de préférence allant dans la plage de 0,01 mg/kg à environ 25 mg/kg de poids corporel.

11. Composé selon la revendication 10, pour usage afin d'inhiber la croissance des cellules cancéreuses ou de les détruire.

12. Composé pour traiter un cancer, dans lequel les cellules cancéreuses comprennent des cellules cancéreuses Rb-négatives, lequel composé a la structure: 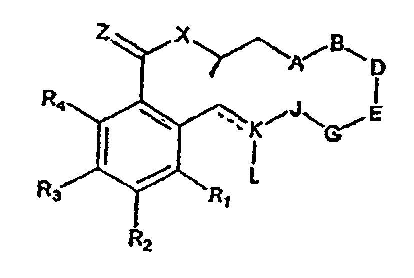 dans laquelle la ligne en traits interrompus --- représente une liaison, auquel cas une double liaison est présente, ou la ligne en traits interrompus est --- absente, auquel cas une simple liaison est présente;
R₁ est un hydrogène, un halogène, un groupement aliphatique, hétéroaliphatique, aryle, hétéroaryle, alkylaryle ou alkylhétéroaryle, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, un cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, ou un groupement aliphatique, hétéroaliphatique, aryle, hétéroaryle, alkylaryle ou alkylhétéroaryle, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un groupement aliphatique, hétéroaliphatique, aryle, hétéroaryle, alkylaryle ou alkylhétéroaryle, ou N(R_{C})₂, dans lequel chaque occurrence de R_{C} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, un cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un groupement aliphatique, hétéroaliphatique, aryle, hétéroaryle, alkylaryle ou alkylhétéroaryle, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
Z est O, S ou NR_{E}, dans lequel R_{E} est un hydrogène, un groupe protecteur, un groupement aliphatique, hétéroaliphatique, aryle, hétéroaryle, alkylaryle ou alkylhétéroaryle, ou OR_{F}, dans lequel R_{F} est un hydrogène, un groupe protecteur, un groupement aliphatique, hétéroaliphatique, aryle, hétéroaryle, alkylaryle ou alkylhétéroaryle;
X est O, S ou N%, dans lequel R_{G} est un hydrogène ou un alkyle inférieur;
A et B ensemble représentent 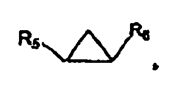 dans lequel R₅ et R₆ sont chacun indépendamment un hydrogène, un halogène, un cyano, OR_{J}, -N(R_{J})₂, -SR_{J}, -O(C=O)R_{J}, -O(S=O)R_{J}, -N(R_{J}) (C=O) (R_{J}), -C(=O)R_{J}, -C(=O)OR_{J}, -CON(R_{J})₂, -OCO₂R_{J}, -OS(=O)OR_{J} ou un groupement aliphatique, hétéroaliphatique, aryle, hétéroaryle, alkylaryle ou alkylhétéroaryle, dans lequel chaque occurrence de R_{J} est indépendamment un hydrogène, un groupe protecteur ou un groupement aliphatique, hétéroaliphatique, aryle, hétéroaryle, alkylaryle ou alkylhétéroaryle, et dans lequel R₇ est un hydrogène, un groupe protecteur, -OR_{K}, -SR_{K}, C(O)OR_{K}, -O(O)NR_{K}, -S(O)₂R_{K}, -O(C=O)R_{K}, -N(R_{K})(C=O)(R_{K}), -C(O)R_{K}, C(O)OR_{K}, -CON(R_{K})₂, -OCO₂R_{K}, ou un groupement aliphatique, hétéroaliphatique, aryle, hétéroaryle, alkylaryle ou alkylhétéroaryle, dans lequel chaque occurrence de R_{K} est indépendamment un hydrogène, un groupe protecteur ou un groupement aliphatique, hétéroaliphatique, aryle, hétéroaryle, alkylaryle ou alkylhétéroaryle, ou quand A et B ensemble représentent -CHR₅-CHR₆-, R₅ et R₆ pris ensemble représentent un anneau aliphatique, hétéroaliphatique, aryle ou hétéroaryle à 3 à 7 chaînons, substitué ou non substitué;
D et E ensemble représentent -CHR₈-CHR₉-, -CR₈=CR₉-, dans lequel R₈ et R₉ sont chacun indépendamment un hydrogène ou un alkyle inférieur;
G et J ensemble représentent -CHR₁₀-CHR₁₁-, -CR₁₀=CR₁₁-, dans lequel R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})2 ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D}, R₅, R₆, R_{J} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes; et
les dérivés pharmaceutiquement acceptables de ces composés.

13. Composé selon la revendication 12, dans lequel le composé est présent en une quantité allant dans la plage de 0,001 mg/kg à 50 mg/kg de poids corporel, de préférence allant dans la plage de 0,01 mg/kg à environ 25 mg/kg de poids corporel.

14. Composé selon la revendication 12, dans lequel le cancer comprenant des cellules Rb-négatives est un cancer du poumon à petites cellules, un glioblastome ou un rétinoblastome.

15. Composé pour usage dans l'inhibition de la croissance ou dans la destruction de cellules cancéreuses Rb-négatives, lequel composé a la structure: 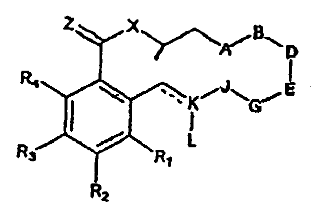 dans laquelle la ligne en traits interrompus --- représente une liaison, auquel cas une double liaison est présente, ou la ligne en traits interrompus est absente, auquel cas une simple liaison est présente;
R₁ est un hydrogène, un halogène, un groupement aliphatique, hétéroaliphatique, aryle, hétéroaryle, alkylaryle ou alkylhétéroaryle, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, un cyano, -OR_{B}, -N(R_{B})2, -SR_{B}, -O(C=O)R_{B}, -N(R_{B})(C=O)(R_{B}), -C(O)R_{B}, C(O)OR_{B}, -CON(R_{B})₂, -OCO2R_{B}, ou un groupement aliphatique, hétéroaliphatique, aryle, hétéroaryle, alkylaryle ou alkylhétéroaryle, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un groupement aliphatique, hétéroaliphatique, aryle, hétéroaryle, alkylaryle ou alkylhétéroaryle, ou N(R_{C})₂, dans lequel chaque occurrence de R_{C} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, un cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un groupement aliphatique, hétéroaliphatique, aryle, hétéroaryle, alkylaryle ou alkylhétéroaryle, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
Z est O, S ou NR_{E}, dans lequel R_{E} est un hydrogène, un groupe protecteur, un groupement aliphatique, hétéroaliphatique, aryle, hétéroaryle, alkylaryle ou alkylhétéroaryle, ou OR_{F}, dans lequel R_{F} est un hydrogène, un groupe protecteur, un groupement aliphatique, hétéroaliphatique, aryle, hétéroaryle, alkylaryle ou alkylhétéroaryle;
X est O, S ou NR_{G}, dans lequel R_{G} est un hydrogène ou un alkyle inférieur;
A et B ensemble représentent 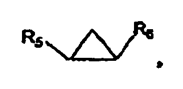 dans lequel R₅ et R₆ sont chacun indépendamment un hydrogène, un halogène, un cyano, OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} ou un groupement aliphatique, hétéroaliphatique, aryle, hétéroaryle, alkylaryle ou alkylhétéroaryle, dans lequel chaque occurrence de R_{J} est indépendamment un hydrogène, un groupe protecteur ou un groupement aliphatique, hétéroaliphatique, aryle, hétéroaryle, alkylaryle ou alkylhétéroaryle, et dans lequel R₇ est un hydrogène, un groupe protecteur, -OR_{K}, -SR_{K}, C(O)OR_{K}, -O(O)NR_{K}, -S(O)₂R_{K}, -O(C=O)R_{K}, -N(R_{K})(C=O)(R_{K}), -C(O)R_{K}, C(O)OR_{K}, -CON(R_{K})₂, -OCO₂R_{K}, ou un groupement aliphatique, hétéroaliphatique, aryle, hétéroaryle, alkylaryle ou alkylhétéroaryle, dans lequel chaque occurrence de R_{K} est indépendamment un hydrogène, un groupe protecteur ou un groupement aliphatique, hétéroaliphatique, aryle, hétéroaryle, alkylaryle ou alkylhétéroaryle, ou quand A et B ensemble représentent -CHR₅-CHR₆-, R₅ et R₆ pris ensemble représentent un anneau aliphatique, hétéroaliphatique, aryle ou hétéroaryle à 3 à 7 chaînons, substitué ou non substitué;
D et E ensemble représentent -CHR₈-CHR₉-, -CR₈=CR₉-, dans lequel R₈ et R₉ sont chacun indépendamment un hydrogène ou un alkyle inférieur;
G et J ensemble représentent -CHR₁₀-CHR₁₁-, -CR₁₀=CR₁₁-, dans lequel R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D}, R₅, R₆, R_{J} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes; et
les dérivés pharmaceutiquement acceptables de ces composés.

16. Composé selon la revendication 10, dans lequel les cellules cancéreuses Rb-négatives sont des cellules de cancer du poumon à petites cellules, de glioblastome ou de rétinoblastome.

17. Utilisation d'un composé de la formule I dans la fabrication d'un médicament pour traiter un cancer.

18. Procédé pour la synthèse d'un composé ayant la structure (I): 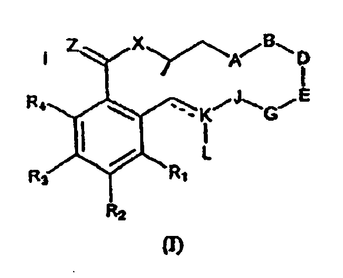 dans laquelle la ligne en traits interrompus -- représente une liaison, auquel cas une double liaison est présente, ou la ligne en traits interrompus -- est absente, auquel cas une simple liaison est présente;
R₁ est un hydrogène, un halogène, un groupement aliphatique, hétéroaliphatique, aryle, hétéroaryle, alkylaryle ou alkylhétéroaryle, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, un cyano, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, ou un groupement aliphatique, hétéroaliphatique, aryle, hétéroaryle, alkylaryle ou alkylhétéroaryle, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un groupement aliphatique, hétéroaliphatique, aryle, hétéroaryle, alkylaryle ou alkylhétéroaryle, ou N(R_{c})₂, dans lequel chaque occurrence de R_{c} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, un cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un groupement aliphatique, hétéroaliphatique, aryle, hétéroaryle, alkylaryle ou alkylhétéroaryle, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
Z est O, S ou NR_{E}, dans lequel R_{E} est un hydrogène, un groupe protecteur, un groupement aliphatique, hétéroaliphatique, aryle, hétéroaryle, alkylaryle ou alkylhétéroaryle, ou OR_{F}, dans lequel R_{F} est un hydrogène, un groupe protecteur, un groupement aliphatique, hétéroaliphatique, aryle, hétéroaryle, alkylaryle ou alkylhétéroaryle;
X est O, S ou NR_{G}, dans lequel % est un hydrogène ou un alkyle inférieur;
A et B ensemble représentent 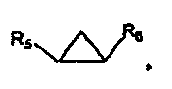 dans lequel R₅ et R₆ sont chacun indépendamment un hydrogène, un halogène, un cyano, OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} ou un groupement aliphatique, hétéroaliphatique, aryle, hétéroaryle, alkylaryle ou alkylhétéroaryle, dans lequel chaque occurrence de R_{J} est indépendamment un hydrogène, un groupe protecteur ou un groupement aliphatique, hétéroaliphatique, aryle, hétéroaryle, alkylaryle ou alkylhétéroaryle, et dans lequel R₇ est un hydrogène, un groupe protecteur, -OR_{K}, -SR_{K}, C(O)OR_{K}, -O(O)NR_{K}, -S(O)₂R_{K}, -O(C=O)R_{K}, -N(R_{K})(C=O)(R_{K}), -C(O)R_{K}, C(O)OR_{K}, -CON(R_{K})₂, -OCO₂R_{K}, ou un groupement aliphatique, hétéroaliphatique, aryle, hétéroaryle, alkylaryle ou alkylhétéroaryle, dans lequel chaque occurrence de R_{K} est indépendamment un hydrogène, un groupe protecteur ou un groupement aliphatique, hétéroaliphatique, aryle, hétéroaryle, alkylaryle ou alkylhétéroaryle, ou quand A et B ensemble représentent -CHR₅-CHR₆-, R₅ et R₆ pris ensemble représentent un anneau aliphatique, hétéroaliphatique, aryle ou hétéroaryle à 3 à 7 chaînons, substitué ou non substitué;
D et E ensemble représentent -CHR₈-CHR₉-, -CR₈=CR₉-, dans lequel R₈ et R₉ sont chacun indépendamment un hydrogène ou un alkyle inférieur;
G et J ensemble représentent -CHR₁₀-CHR₁₁-, -CR₁₀=CR₁₁-, dans lequel R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D}, R₅, R₆, R_{J} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes;
ledit procédé comprenant :
(1) l'apport d'un composant acide benzoïque ayant la structure: 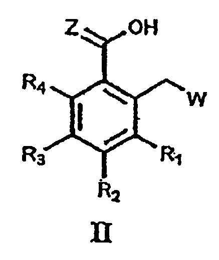 dans laquelle R₁-R₄ et Z sont tels que définis ci-dessus et dans laquelle W est un halogène; et
la réaction du composant acide benzoïque avec un composant chiral (IV) ayant la structure: 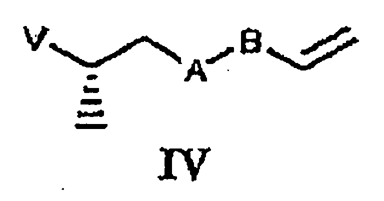 dans laquelle A et B sont tels que définis ci-dessus et dans laquelle V est NHR_{G}, dans lequel R_{G} est un hydrogène ou un alkyle inférieur; SH; ou OH en présence d'un réactif d'estérification pour générer un intermédiaire (V) ayant la structure: 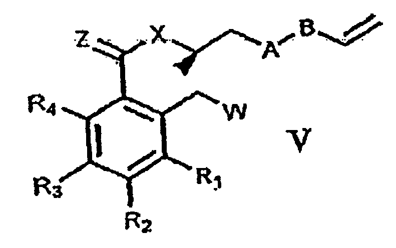
(2) la réaction de l'intermédiaire (V) avec un dithiane ayant la structure (III): 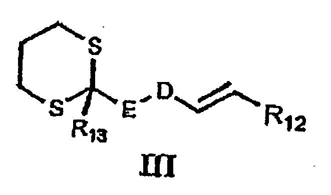 dans laquelle R₁₃ est un hydrogène ou un sel de métal alcalin et dans laquelle R₁₂ est un hydrogène ou un alkyle inférieur, dans des conditions menant à l'addition du dithiane, pour générer un intermédiaire (VI) ayant la structure: 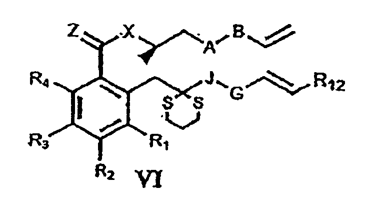
(3) si au moins un de R₁-R₄ est un groupe thio, amino ou hydroxyle non protégé, protégeant facultativement ledit groupe non protégé;
(4) la cyclisation du composé en présence d'un catalyseur de métathèse d'oléfines, pour générer le composé (VII): 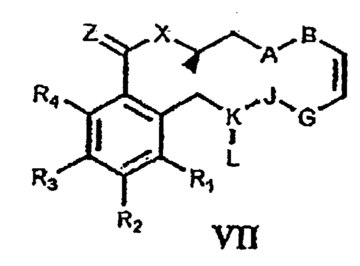
(5) facultativement la réaction en outre de (VII) avec un ou plusieurs réactifs, pour diversifier et facultativement déprotéger le macrolide pour générer le composé (I).

19. Procédé selon la revendication 18, dans lequel l'étape d'estérification est réalisée en utilisant le diéthylazodicarboxylate (DIAD) en présence de triphénylphosphine ou de trifurylphosphine.

20. Procédé selon la revendication 18, dans lequel l'étape de métathèse d'oléfine est réalisée avec un catalyseur de métathèse d'oléfine ou dans lequel l'étape de métathèse d'oléfine est réalisée avec un catalyseur de métathèse d'oléfine à base de ruthénium ou dans lequel l'étape de métathèse d'oléfine est réalisée avec le Ru(1,3-dimésityl-4,5-dihydro-imidazol-2-ylidène) (=CHCH=C(CH₃)₂)PCp₃Cl₂.

21. Procédé pour la synthèse d'un macrocycle ayant la structure (VII): 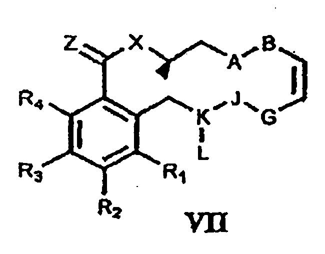 dans laquelle la ligne en traits interrompus --- représente une liaison, auquel cas une double liaison est présente, ou la ligne en traits interrompus est absente, auquel cas une simple liaison est présente;
R₁ est un hydrogène, un halogène, un groupement aliphatique, hétéroaliphatique, aryle, hétéroaryle, alkylaryle ou alkylhétéroaryle, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, un cyano, -OR_{B}, -N(R_{B})₂, -SR_{B}, -O(C=O)R_{B}, -N(R_{H})(C=O)(R_{B}), -C(O)R_{B}, -C(O)OR_{B}, -CON(R_{B})₂, -OCO₂R_{B}, ou un groupement aliphatique, hétéroaliphatique, aryle, hétéroaryle, alkylaryle ou alkylhétéroaryle, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un groupement aliphatique, hétéroaliphatique, aryle, hétéroaryle, alkylaryle ou alkylhétéroaryle, ou N(R_{c})₂, dans lequel chaque occurrence de R_{c} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, un cyano, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un groupement aliphatique, hétéroaliphatique, aryle, hétéroaryle, alkylaryle ou alkylhétéroaryle, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
Z est O, S ou NR_{E}, dans lequel R_{E} est un hydrogène, un groupe protecteur, un groupement aliphatique, hétéroaliphatique, aryle, hétéroaryle, alkylaryle ou alkylhétéroaryle, ou OR_{F}, dans lequel R_{F} est un hydrogène, un groupe protecteur, un groupement aliphatique, hétéroaliphatique, aryle, hétéroaryle, alkylaryle ou alkylhétéroaryle;
X est O, S ou NR_{G}, dans lequel R_{G} est un hydrogène ou un alkyle inférieur;
A et B ensemble représentent 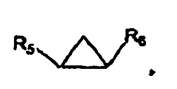 dans lequel R₅ et R₆ sont chacun indépendamment un hydrogène, un halogène, un cyano, OR_{J}, -N(R_{J})₂, -SR_{J}, -O(C=O)R_{J}, -O(S=O)R_{J}, -N(R_{J})(C=O)(R_{J}), -C(=O)R_{J}, -C(=O)OR_{J}, -CON(R_{J})₂, -OCO₂R_{J}, -OS(=O)OR_{J} ou un groupement aliphatique, hétéroaliphatique, aryle, hétéroaryle, alkylaryle ou alkylhétéroaryle, dans lequel chaque occurrence de R_{J} est indépendamment un hydrogène, un groupe protecteur ou un groupement aliphatique, hétéroaliphatique, aryle, hétéroaryle, alkylaryle ou alkylhétéroaryle, et dans lequel R₇ est un hydrogène, un groupe protecteur, -OR_{K}, -SR_{K}, C(O)OR_{K}, -O(O)NR_{K}, -S(O)₂R_{K}, -O(C=O)R_{K}, -N(R_{K})(C=O)(R_{K}), -C(O)R_{K}, C(O)OR_{K}, -CON(R_{K})₂, -OCO₂R_{K}, ou un groupement aliphatique, hétéroaliphatique, aryle, hétéroaryle, alkylaryle ou alkylhétéroaryle, dans lequel chaque occurrence de R_{K} est indépendamment un hydrogène, un groupe protecteur ou un groupement aliphatique, hétéroaliphatique, aryle, hétéroaryle, alkylaryle ou alkylhétéroaryle, ou quand A et B ensemble représentent -CHR₅-CHR₆-, R₅ et R₆ pris ensemble représentent un anneau aliphatique, hétéroaliphatique, aryle ou hétéroaryle à 3 à 7 chaînons, substitué ou non substitué;
G et J ensemble représentent -CHR₁₀-CHR₁₁-, -CR₁₀=CR₁₁-, dans lequel R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel ledit procédé comprend la cyclisation de l'intermédiaire (VI): 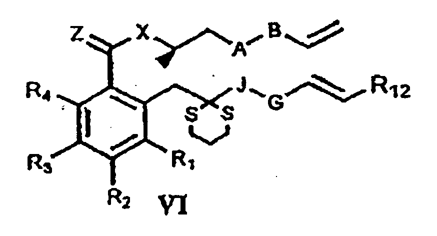 dans lequel R₁₂ est un hydrogène ou un alkyle inférieur, en présence d'un catalyseur de métathèse d'oléfine, pour générer le composé (VII).

22. Procédé selon la revendication 21, dans lequel le procédé comprend en outre la diversification du composé (VII) pour générer un composé ayant la structure (I) comme défini ici.

23. Procédé selon la revendication 21, dans lequel l'étape de métathèse d'oléfine est réalisée avec un catalyseur de métathèse d'oléfine ou dans lequel l'étape de métathèse d'oléfine est réalisée avec un catalyseur de métathèse d'oléfine à base de ruthénium ou dans lequel l'étape de métathèse d'oléfine est réalisée avec le Ru(1,3-dimésityl-4,5-dihydro-imidazol-2-ylidène) (=CHCH=C(CH₃)₂)PCp₃Cl₂.

24. Composé selon la revendication 1, ayant la structure:

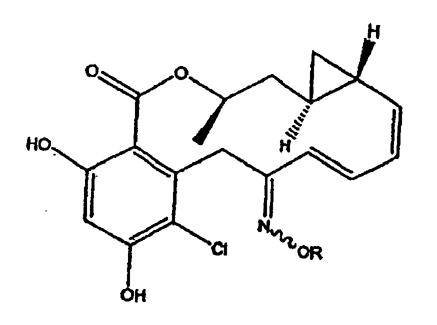

25. Composé selon la revendication 1, ayant la structure: 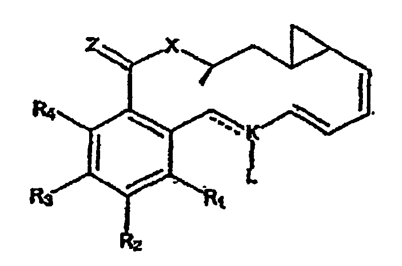 dans laquelle Z est O et X est O ou NH;
R₁ est un hydrogène, un halogène, un alkyle inférieur, un hétéroalkyle inférieur, un alkylaryle inférieur, un alkylhétéroaryle inférieur, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, ou un groupement alkyle inférieur, hétéroalkyle inférieur, alkylaryle inférieur, alkylhétéroaryle inférieur, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un alkyle inférieur, hétéroalkyle inférieur, alkylaryle inférieur, alkylhétéroaryle inférieur, ou N(R_{c})₂, dans lequel chaque occurrence de R_{c} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un alkyle inférieur, hétéroalkyle inférieur, alkylaryle inférieur, alkylhétéroaryle inférieur, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
K et L ensemble représentent C=O, C=S, CH-CH₃, CH-CH(R_{L})₂, C=C(R_{L})₂, -CH₂-, -C(-S(CH₂)₃S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D}, R₅, R₆, R_{J} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes.

26. Composé selon la revendication 1, ayant la structure: 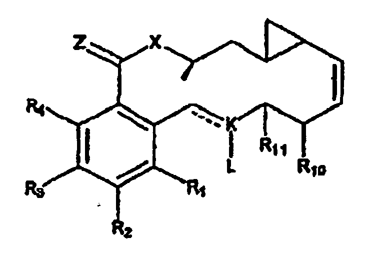 dans laquelle Z est O et X est O ou NH;
R₁ est un hydrogène, un halogène, un alkyle inférieur, un hétéroalkyle inférieur, un alkylaryle inférieur, un alkylhétéroaryle inférieur, ou N(R_{A})₂, dans lequel chaque occurrence de R_{A} est indépendamment un hydrogène, un groupe protecteur, ou un groupement aliphatique, hétéroaliphatique, aryle, hétéroaryle, alkylaryle ou alkylhétéroaryle;
R₂ est un hydrogène, un halogène, -OR_{B}, -N(R_{B})₂, -SR_{B}, -O(C=O)R_{B}, -N(R_{B})(C=O)(R_{B}), -C(O)R_{B}, -C(O)OR_{B}, -CON(R_{B})₂, -OCO₂R_{B}, ou un groupement alkyle inférieur, hétéroalkyle inférieur, alkylaryle inférieur, alkylhétéroaryle inférieur, dans lequel chaque occurrence de R_{B} est indépendamment un hydrogène, un groupe protecteur ou un groupement aliphatique, hétéroaliphatique, aryle, hétéroaryle, alkylaryle ou alkylhétéroaryle;
R₃ est un hydrogène, un halogène, un alkyle inférieur, hétéroalkyle inférieur, alkylaryle inférieur, alkylhétéroaryle inférieur, ou N(R_{C})₂, dans lequel chaque occurrence de R_{c} est indépendamment un hydrogène, un groupe protecteur, ou un groupement aliphatique, hétéroaliphatique, aryle, hétéroaryle, alkylaryle ou alkylhétéroaryle;
R₄ est un hydrogène, un halogène, -OR_{D}, -N(R_{D})₂, -SR_{D}, -O(C=O)R_{D}, -N(R_{D})(C=O)(R_{D}), -C(O)R_{D}, -C(O)OR_{D}, -CON(R_{D})₂, -OCO₂R_{D}, ou un alkyle inférieur, hétéroalkyle inférieur, alkylaryle inférieur, alkylhétéroaryle inférieur, dans lequel chaque occurrence de R_{D} est indépendamment un hydrogène, un groupe protecteur ou un groupement aliphatique, hétéroaliphatique, aryle, hétéroaryle, alkylaryle ou alkylhétéroaryle;
R₁₀ et R₁₁ sont chacun indépendamment un hydrogène ou un alkyle inférieur;
K et L ensemble représentent C=O, C=S, CH-CH₃, CH-CH(R_{L})₂, C=C(R_{L})₂, -CH₂-, -C(-S(CH₃)₂S-), CH-OR_{L}, CH-SR_{L}, CH-N(R_{L})₂, CH-N(R_{L})(C=O)(R_{L}), C=N-O-R_{L}, CH-N=O, C=C(R_{L})-N(R_{L})₂, C=N-R_{L}, C=N-N(R_{L})₂ ou, si la ligne en traits interrompus représente une liaison, auquel cas une double liaison est présente, alors K et L ensemble représentent C-N(R_{L})₂, dans lequel chaque occurrence de R_{L} est indépendamment un hydrogène, un groupe protecteur, un groupement aliphatique, hétéroaliphatique, aryle, hétéroaryle, alkylaryle ou alkylhétéroaryle, ou deux occurrences de R_{L} prises ensemble représentent un groupement cyclique aliphatique, hétéroaliphatique, aromatique ou hétéroaromatique à 3 à 7 chaînons;
auquel cas chacun des groupements aliphatiques et hétéroaliphatiques précédents peut indépendamment être substitué ou non substitué, cyclique ou acyclique, ou ramifié ou non ramifié, et chaque groupement aryle, hétéroaryle, alkylaryle et alkylhétéroaryle peut être substitué ou non substitué;
dans lequel un ou deux quelconques de R₁, R_{A}, R₂, R_{B}, R₃, R_{C}, R₄, R_{D} ou R_{L} sont facultativement un lieur lié par covalence à un composé sélectionné dans le groupe composé de radicicol, monocilline, analogues du radicicol et de la monocilline, gélédanamycine, analogues de la gélédanamycine, et stéroïdes.

27. Composé selon la revendication 1, ayant la structure:

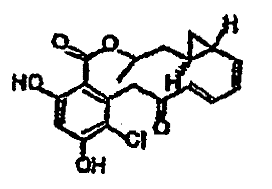

28. Composé selon la revendication 1, ayant la structure:

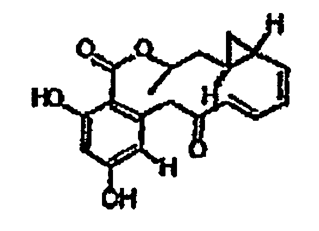

29. Composé selon la revendication 1, ayant la structure:
